# EUROPEAN PATENT SPECIFICATION

(11) **EP 3 954 764 B1**
(45) Date of publication and mention of the grant of the patent: **19.02.2025**
(21) Application number: 19924173.8
(22) Date of filing: 04.11.2019
(51) Int. Cl.: C12N 5/09, C12Q 1/02

(54) **METHOD FOR CULTURING PRIMARY CELLS OF GASTRIC CANCER AND GALLBLADDER AND BILE DUCT CANCER, AND SUPPORTING REAGENTS**
VERFAHREN ZUR ZÜCHTUNG VON PRIMÄRZELLEN VON MAGENKREBS UND GALLENBLASEN- UND GALLENGANGSKREBS UND UNTERSTÜTZENDE REAGENZIEN
PROCÉDÉ DE CULTURE DE CELLULES PRIMAIRES DE CANCER GASTRIQUE ET DE CANCER DE LA VÉSICULE BILIAIRE ET DE CANAL CHOLÉDOQUE, ET RÉACTIFS DE SUPPORT

(30) Priority: 11.04.2019 CN 201910289073; 11.04.2019 CN 201910289074
(43) Date of publication of application: 16.02.2022
(73) Proprietor: Genex Health Co.,Ltd, Beijing 100195 (CN)
(72) Inventor: YIN, Shenyi, Beijing 101105 (CN); ZHANG, Hanshuo, Beijing 101105 (CN)
(74) Representative: Isarpatent
(86) International application number: PCT/CN2019/115306
(87) International publication number: WO 2020/206999

(56) References cited:
- WO-A1-2018/096881
- CN-A- 103 194 429
- CN-A- 103 966 168
- CN-A- 104 403 996
- CN-A- 104 560 878
- CN-A- 105 229 150
- CN-A- 108 624 561
- US-A1- 2012 164 732
- US-A1- 2014 243 227
- GEORGIOS VLACHOGIANNIS ET AL: "Patient-derived organoids model treatment response of metastatic gastrointestinal cancers", SCIENCE, vol. 359, no. 6378, 23 February 2018 (2018-02-23), US, pages 920 - 926, XP055596283, ISSN: 0036-8075, DOI: 10.1126/science.aao2774

## Description

### TECHNICAL FIELD

The present invention relates to the field of biotechnology, specifically to a primary cell culture medium for gastric cancer and/or gallbladder cancer and cholangiocarcinoma, an *in vitro* method for culturing primary cells of gastric cancer and gallbladder cancer and cholangiocarcinoma and auxiliary reagents.

### BACKGROUND

Gastric cancer is one of the most common malignant tumors that seriously threaten human health. China is a country where gastric cancer frequently occurs and the incidence and death of gastric cancer account for 42.6% and 45% of the global incidence and death of gastric cancer respectively. The incidence of gastric cancer in China is 11.8%, which is the fourth place among all malignant tumors. The death of gastric cancer is 22.0%, which is the fifth place among all malignant tumors. The incidence of gastric cancer will keep increasing with the development of economy, growth in the living standard and lifestyle change. Furthermore, there is a high the risk in the recurrence and metastasis of gastric cancer. Different degrees of recurrence and metastasis will occur to more than 50% of gastric cancer patients within months to years after radical treatment.

Gallbladder cancer and cholangiocarcinoma are common malignant tumors of a digestive system occurring at gallbladder, bile duct and intrahepatic bile duct sites, including gallbladder cancer, cholangiocarcinoma and the like. The overall incidence of gallbladder and bile duct related malignant tumors in China is about 3%, in which cholangiocarcinoma accounts for 2%, ranking the fifth among malignant tumors of a digestive tract in China. Although the incidence is not high, all gallbladder and bile duct related cancers are extremely malignant, and cholangiocarcinoma is even called the "king of liver cancer" and the "king of cancer". For unresectable gallbladder cancer, the median survival time is only 8 months.

Although scientific research and medical institutions all over the world have invested heavily in the research on the etiology, occurrence and development processes of gastric cancer, gallbladder cancer and cholangiocarcinoma, little is known about this diseases. Gastric cancer, gallbladder cancer and cholangiocarcinoma are complex diseases, and their occurrence and development are a dynamic process which involves the interaction of numerous signaling molecules and forms a complex molecular regulating network; moreover, this process is also affected by external environmental factors. We cannot generalize the etiology, occurrence and development process of gastric cancer, gallbladder cancer and cholangiocarcinoma, which are highly different among individuals. Therefore, it is a trend to use primary cell cultures of gastric cancer and gallbladder cancer and cholangiocarcinoma as models for individualized accurate research in the research field of gastric cancer and gallbladder cancer and cholangiocarcinoma, and even in the field of diagnosis and treatment of gastric cancer.

The existing primary tumor cell culturing technologies mainly include 2D culture, 3D culture, reprogramming culture and so on. These methods are facing the problems of extremely long culture cycle, low culture success rate, difficult removal of impurity cells and the like.US2014243227A1 describes a culture medium and methods for expanding populations of stem cells grown from carcinomas or healthy tissues.GEORGIOS VLACHOGIANNIS ET AL( "Patient-derived organoids model treatment response of metastatic gastrointestinal cancers", SCIENCE, vol. 359, no. 6378, 23 February 2018 (2018-02-23), pages 920-926, XP055596283) describe a method for isolating and culturing of primary cells from patients with gastrointestinal cancers, and also describes a method of establishing a cell culture system from the metastatic gastrointestinal cancers.

CN105229150A describes a primary culture of human cancer cells and tumor mass pieces can be digested with collagenase I, collagenase IV, Dispase, Unidasas and DNA enzyme.

US2012164732 describes a use of specific collagenases for cell dissociation.

### SUMMARY

To effectively solve the above-mentioned technical problems, the present invention provides a new *in vitro* method for culturing primary cells of gastric cancer and gallbladder cancer and cholangiocarcinoma and auxiliary reagents, according to claim 3. The core of the technology is that: (1) the solid tumor tissues of gastric cancer and gallbladder cancer and cholangiocarcinoma are treated with a mild cell dissociation reagent to ensure the vitality of cancer cells in tissues to the greatest extent; the primary tumor cells of gallbladder cancer and cholangiocarcinoma in a bile sample are isolated by a mild method to ensure the vitality of cancer cells to the greatest extent; (2) a special serum-free medium is prepared, and tumor cells of gastric cancer and gallbladder cancer and cholangiocarcinoma are cultured in vitro by a suspension culture system to eliminate the interference of normal cells to the greatest extent while ensuring normal amplification of cancer cells.

In the first aspect, the present invention claims a primary cell culture medium for gastric cancer and/or gallbladder cancer and cholangiocarcinoma, according to claim 1.

According to claim 1, the primary cell culture medium for gastric cancer and/or gallbladder cancer and cholangiocarcinoma claimed by the present invention is composed of antibiotic-antimycotic (penicillin-streptomycin-amphotericin B), HEPES, GlutaMax, non-essential amino acids, human recombinant protein EGF, human recombinant protein bFGF, human recombinant protein HGF, human recombinant protein FGF-10, human recombinant protein Wnt-3a, human recombinant protein Noggin, SB202190 (4-(4-fluorophenyl)-2-(4-hydroxyphenyl)-5-(4-pyridyl)-1H-Imidazole), A83-01 (3-(6-Methyl -2-pyridinyl)-N-phenyl-4-(4-quinolinyl)-1H-pyrazole-1-carbothioami de), Primocin^{™}, N-acetyl-L-cysteine, Nicotinamide, N-2 Supplement, cortisol, B27, ITS-X (Insulin, Transferrin, Selenium, Ethanolamine Solution), Gastrin 1, Y-27632 and Advanced DMEM/F12 culture medium. Wherein, the final concentration of penicillin in the antibiotic-antimycotic is 100-200U/mL (such as 100 U/mL); the final concentration of streptomycin in the antibiotic-antimycotic is 100-200µg/mL (such as 100µg /mL); the final concentration of amphotericin B in the antibiotic-antimycotic is 250-250ng/mL (such as 250ng/mL); the final concentration of the HEPES is 8-12mM (such as 10 mM); the final concentration of the GlutaMax is 0.8-1.2% (such as 1%, % means the volume percentage); the concentration of glycine in the non-essential amino acids is 80-120µM; the concentration of L-alanine in the non-essential amino acids is 80-120µM (such as 100µM); the concentration of L-asparagine in the non-essential amino acids is 80-120µM (such as 100µM); the concentration of L-aspartic acid in the non-essential amino acids is 80-120µM (such as 100µM); the concentration of L-glutamic acid in the non-essential amino acids is 80-120µM (such as 100µM); the concentration of L-proline in the non-essential amino acids is 80-120µM (such as 100µM); the concentration of L-serine in the non-essential amino acids is 80-120µM (such as 100µM); the final concentration of the human recombinant protein EGF is 10-100 ng/mL; the final concentration of the human recombinant protein bFGF is 10-50 ng/mL; the final concentration of the human recombinant protein HGF is 5-25 ng/mL; the final concentration of the human recombinant protein FGF-10 is 5-25 ng/mL; the final concentration of the human recombinant protein Wnt-3a is 200-300 ng/mL; the final concentration of the human recombinant protein Noggin is 100-200 ng/mL; the final concentration of the SB202190 is 5-10µM; the final concentration of the A83-01 is 0.25-1.25µM; the final concentration of Primocin is 1% (volume percentage); the final concentration of the N-acetyl-L-cysteine is 0.5-2mM; the final concentration of the Nicotinamide is 5-10mM; the final concentration of the N-2 Supplement is 1% (volume percentage); the final concentration of the cortisol is 20-50 ng/mL; the final concentration of the B27 is 1.5-2.5% (such as 2%, % represents the volume percentage); the final concentration of the ITS-X is 0.8-1.2% (such as 1%, % represents the volume percentage); the final concentration of the Gastrin 1 is 8-12nM (such as 10nM); the final concentration of the Y-27632 is 5-20 µM; and the rest is the Advanced DMEM/F12 medium, wherein the gastric cancer is primary gastric cancer; and the gallbladder cancer and cholangiocarcinoma is primary gallbladder cancer and cholangiocarcinoma; or, the gastric cancer is a metastatic lesion of gastric cancer; and the gallbladder cancer and cholangiocarcinoma is a metastatic lesion of gallbladder cancer and cholangiocarcinoma.

Further, the composition of the antibiotic-antimycotic (penicillin-streptomycin-amphotericin B) is as follows: each milliliter is composed of 10,000 units of penicillin (base), 10,000 µg of streptomycin (base) and 25 µg of amphotericin B. The antibiotic-antimycotic (penicillin-streptomycin-amphotericin B) is "Antibiotic-Antimycotic, 100X" (such as Gibco#15240062, or other products with the same composition). Each milliliter of "Antibiotic-Antimycotic, 100X" is composed of 10000 units of penicillin (base), 10,000 µg of streptomycin (base) and 25 µg of amphotericin B, using penicillin G (sodium salt), streptomycin sulfate and amphotericin B in a form of 0.85% salt solution as a Fungizone^{®} antimycotic agent. The GlutaMAX is "GlutaMAX^{™} Supplement" (such as Gibco#35050061, or other products with the same composition). The component of the "GlutaMAX^{™} Supplement" is L-alanyl-L-glutamine, an alternative to L-glutamine, with a concentration of 200nM, and a solvent of 0.85% NaCl solution. The composition of the non-essential amino acids is as follows: each milliliter is composed of 750µg of glycine, 890µg of L-alanine, 1,320µg of L-asparagine, 1,330µg of L-aspartate, 1,470µg of L-glutamic acid, 1,150µg of L-proline and 1,050µg of L-serine, and the solvent is water (the concentration of various amino acids involved above is 10mM per milliliter of non-essential amino acids). The Primocin is an antibacterial agent for primary cells (such as Invivogene#ant-pm-1, or other products with the same composition), which is an antibiotic for protecting primary cells against microbial contamination and can kill Gram-positive bacteria, Gram-negative bacteria, mycoplasma and fungi. The N-2 Supplement is "N-2 Supplement (100X)" (such as Gibco# 17502001, or other products with the same composition). The "N-2 Supplement (100X)" is composed of Human Transferrin (Holo) with a final concentration of 1mM, 500mg/L Insulin Recombinant Full Chain, 0.63mg/L Progesterone, 10mM Putrescine and 0.52mg/L Selenite. The B27 is "B-27^{™} Supplement (50X), minus vitamin A" (such as Gibco#12587010, or other products with the same composition). The "B-27^{™} Supplement (50X), minus vitamin A" is composed of Biotin, DL Alpha Tocopherol Acetate, DL Alpha-Tocopherol, BSA (fatty acid free Fraction V), Catalase, Human Recombinant Insulin, Human Transferrin, Superoxide Dismutase, Corticosterone, D-Galactose, Ethanolamine HCl, Glutathione (reduced), L-Carnitine HCl, Linoleic Acid, Linolenic Acid, Progesterone, Putrescine 2HCl, Sodium Selenite and T3 (triodo-I-thyronine). The solvent of the ITS-X is an EBSS solution (Earle's balanced salt solution), and the solute and concentration are as follows: insulin 1g/L; transferrin 0.55 g/L; sodium selenite 0.00067 g/L; ethanolamine 0.2 g/L. The GlutaMAX is an advanced cell culture additive that can directly replace L-glutamine in the cell culture medium. The GlutaMAX is "GlutaMAX^{™} Supplement" (such as Gibco#35050061, or other products with the same composition). The Y-27632 is "Y-27632 dihydrochloride (an ATP-competitive ROCK-I and ROCK-II inhibitor, with Ki of 220 nM and 300 nM respectively)" (such as MCE#129830-38-2, or other products with the same composition).

In the embodiments of the present invention, the brand article No. of the antibiotic-antimycotic (penicillin-streptomycin-amphotericin B) is Gibco#15240062; the brand article No. of the HEPES is Gibco#15630080; the brand article No. of the GlutaMAX is Gibco#35050061; the brand article No. of the non-essential amino acids is Gibco#11140050; the brand article No. of the human recombinant protein EGF is Peprotech AF-100-15-100; the brand article No. of the human recombinant protein bFGF is Peprotech AF-100-18B-50; the brand article No. of the human recombinant protein HGF is Peprotech AF-100-39-100; the brand article No. of the human recombinant protein FGF-10 is Peprotech AF-100-26-100; the brand article No. of the human recombinant protein Wnt-3a is R&D 5036-WN-500; the brand article No. of the human recombinant protein Noggin is Shanghai nearshore #C018; the brand article No. of the SB202190 is Sigma#S7067; the brand article No. of the A83-01 is Tocris#2939; the brand article No. of the Primocin^{™} is Invivogene#ant-pm-1; the brand article No. of the N-acetyl-L-cysteine is Sigma#A9165; the brand article No. of the Nicotinamide is Sigma#N0636; the brand article No. of the N-2 Supplement is ibco#17502001; the brand article No. of the cortisol is Sigma#H0888; the brand article No. of the B27 is Gibco#12587010; the brand article No. of the ITS-X is Gibco#51500056; the brand article No. of the gastrin is NJPeptide#Pep12307; the brand article No. of the Y-27632 is MCE#129830-38-2; the brand article No. of the Advanced DMEM/F12 medium is Gibco#12634010.

Further, the primary cell culture medium for gastric cancer and/or gallbladder cancer and cholangiocarcinoma can exist in two forms:
Firstly, the primary cell culture medium for gastric cancer and/or gallbladder cancer and cholangiocarcinoma is a solution being composed of the antibiotic-antimycotic, the HEPES, the GlutaMax, the non-essential amino acids, the human recombinant protein EGF, the human recombinant protein bFGF, the human recombinant protein HGF, the human recombinant protein FGF-10, the human recombinant protein Wnt-3a, the human recombinant protein Noggin, the SB202190, the A83-01, the Primocin^{™}, the N-acetyl-L-cysteine, the nicotine, the N-2 Supplement, the cortisol, the B27, the ITS-X, the gastrin, the Y-27632, and the Advanced DMEM/F12 medium.

The medium is prepared, filtered and sterilized with a 0.22 µM syringe-driven filter (Millipore SLGP033RS). The medium can be preserved at 4°C for two weeks.

Secondly, the components of the primary cell culture medium for gastric cancer and/or gallbladder cancer and cholangiocarcinoma can exist separately, and the medium will be prepared according to the formula on demand.

Further, the human recombinant protein EGF, human recombinant protein bFGF, human recombinant protein HGF, human recombinant protein FGF-10, human recombinant protein Wnt-3a and human recombinant protein Noggin that can exist in a form of stock solution (mother solution) (long-term storage at -80°C), specifically 1,000× stock solution (mother solution). SB202190, N-acetyl-L-cysteine, Nicotinamide, cortisol, gastrin and Y-27632 can exist in a form of stock solution (mother solution) (long-term storage at -20°C), specifically 1,000× stock solution (mother solution). A83-01 can exist in a form of stock solution (mother solution) (long-term storage at -20°C), specifically 100,000× stock solution (mother solution).

A 1,000× human recombinant protein EGF stock solution is composed of human recombinant protein EGF, BSA and PBS, wherein the final concentration of the human recombinant protein EGF is 20 µg/mL, the final concentration of the BSA is 0.01g/mL, and the rest is PBS.

A 1,000× human recombinant protein bFGF stock solution is composed of human recombinant protein bFGF, BSA and PBS, wherein the final concentration of the human recombinant protein bFGF is 20 µg/mL, the final concentration of the BSA is 0.01g/mL, and the rest is PBS.

A 1,000× human recombinant protein HGF stock solution is composed of human recombinant protein HGF, BSA and PBS, wherein the final concentration of the human recombinant protein HGF is 20 µg/mL, the final concentration of the BSA is 0.01g/mL, and the rest is PBS.

A 1,000× human recombinant protein FGF-10 stock solution is composed of human recombinant protein FGF-10, BSA and PBS, wherein the final concentration of the human recombinant protein FGF-10 is 20 µg/mL, the final concentration of the BSA is 0.01g/mL, and the rest is PBS.

A 1,000× human recombinant protein Wnt-3a stock solution is composed of human recombinant protein Wnt-3a, BSA and PBS, wherein the final concentration of the human recombinant protein Wnt-3a is 200 µg/mL, the final concentration of the BSA is 0.01g/mL, and the rest is PBS.

A 1,000× human recombinant protein Noggin stock solution is composed of human recombinant protein Noggin, BSA and PBS, wherein the final concentration of the human recombinant protein Noggin is 100 µg/mL, the final concentration of the BSA is 0.01g/mL, and the rest is PBS.

Among the above six 1,000× stock solutions, the BSA can exist in a form of 100× stock solution (mother solution) (prepared just before use) and specifically is composed of BSA and PBS, wherein the final concentration of BSA (Sigma#A1933) is 0.1g/mL, and the rest is PBS.

In addition, 1,000×SB202190 stock solution is composed of SB202190 and DMSO, wherein the final concentration of the SB202190 is 10 mM, and the rest is DMSO.

A 100,000×A83-01 stock solution is composed of A83-01 and DMSO, wherein the concentration of the A83-01 is 25mM, and the rest is DMSO.

A 1,000×N-acetyl-L-cysteine stock solution is composed of N-acetyl-L-cysteine and ultrapure water, wherein the concentration of the N-acetyl-L-cysteine is 0.5M, and the rest is ultrapure water.

A 1,000×Nicotinamide stock solution is composed of Nicotinamide and ultrapure water, wherein the concentration of the Nicotinamide is 5 M, and the rest is ultrapure water.

A 1,000×cortisol stock solution is composed of cortisol, absolute ethyl alcohol and ultrapure water, wherein the final concentration of the cortisol is 25 µg/mL, the final concentration of the absolute ethyl alcohol is 5% (volume percentage), and the rest is ultrapure water.

A 1,000×gastrin stock solution is composed of gastrin and ultrapure water, wherein the concentration of the gastrin is 10 µM, and the rest is ultrapure water.

A 1,000×Y-27632 stock solution is composed of Y-27632 and ultrapure water, wherein the final concentration of the Y-27632 is 10 mM, and the rest is ultrapure water.

In the second aspect, the present invention claims a kit of reagents for culturing primary cells of gastric cancer and/or gallbladder cancer and cholangiocarcinoma, according to claim 2.

According to claim 2, the kit of reagents claimed by the present invention is any of the following:
(A1) Consisting of the culture medium described in claim 1 and all or part of the following: sample dissociation solution, sample preservation solution and sample washing solution;
The sample dissociation solution is composed of collagenase I, collagenase II, collagenase IV and PBS; wherein the final concentration of the collagenase I is 150-250 U/mL; the final concentration of the collagenase II is 150-250 U/mL; the final concentration of the collagenase IV is 50-150 U/mL; and the rest is PBS;
The sample preservation solution is composed of fetal bovine serum, antibiotic-antimycotic, HEPES and HBSS; wherein the final concentration of the fetal bovine serum is 1-5% (volume percentage); the final concentration of penicillin in the antibiotic-antimycotic is 100-200U/mL; the final concentration of streptomycin in the antibiotic-antimycotic is 100-200µg/mL; the final concentration of amphotericin B in the antibiotic-antimycotic is 250-500 ng/mL; the final concentration of the HEPES is 8-12mM; and the rest is HBSS.
The sample washing solution is composed of antibiotic-antimycotic and PBS; wherein the final concentration of penicillin in the antibiotic-antimycotic
is 100-200 U/mL; the final concentration of streptomycin in the antibiotic-antimycotic is 100-200 µg /mL; the final concentration of amphotericin B in the antibiotic-antimycotic is 250-500 ng /mL; and the rest is PBS.
(A2) Consisting of the culture medium described in claim 1 and the cell isolation buffer;
The cell isolation buffer is composed of P/S, heparin sodium and PBS; wherein the final concentration of penicillin in the P/S is 100-200 U/mL; the final concentration of streptomycin in the P/S is 100-200 µg /mL; the final concentration of the heparin sodium is 10IU/mL; and the rest is PBS;
(A3) Consisting of (A1) and all or part of the following reagents: cell digestion solution, digestion termination solution and cell cryopreserving solution;
(A4) Consisting of (A2) and all or part of the following reagents: cell digestion solution, digestion termination solution and cell cryopreserving solution;
The composition of the cell digestion solution is as follows: each 10mL of the cell digestion solution contains 4-6mL of Accutase, EDTA with a final concentration of 5mM and 1.5-2.5mL of TrypLE Express, and the rest is PBS;
The digestion termination solution is composed of fetal bovine serum, antibiotic-antimycotic and DMEM culture medium; wherein the final concentration of the fetal bovine serum is 8-12% (volume percentage); the final concentration of penicillin in the antibiotic-antimycotic is 100-200 U/mL; the final concentration of streptomycin in the antibiotic-antimycotic is 100-200 µg /mL; the final concentration of amphotericin B in the antibiotic-antimycotic is 250-500 ng /mL; and the rest is the DMEM culture medium;
The cell cryopreserving solution is composed of Advanced DMEM/F12 medium, DMSO and 1% methylcellulose solution; wherein the volume ratio of the Advanced DMEM/F12 medium, the DMSO and the 1% methylcellulose solution is 20:2: (0.8-1.2); the 1% methylcellulose solution is an aqueous solution of methylcellulose with a concentration of 1g/100ml.
The sample dissociation solution is composed of collagenase I, collagenase II, collagenase IV and PBS; wherein the final concentration of the collagenase I is 150-250 U/mL (such as 200 U/mL); the final concentration of the collagenase II is 150-250 U/mL (such as 200 U/mL); the final concentration of the collagenase IV is 50-150 U/mL (such as 100 U/mL); and the rest is PBS.

Wherein the unit U of the collagenase (the collagenase I, the collagenase II or the collagenase IV) is defined by an enzyme activity of protease: 1 µmol of L-leucine can be released by treating the collagenase (the collagenase I, the collagenase II or the collagenase IV) with 1U of protease for 5 hours at 37°C and pH 7.5.

In the embodiments of the present invention, the brand article No. of the collagenase I is Gibco#17100-017. The brand article No. of the collagenase II is Gibco#17101-015; the brand article No. of the collagenase IV is Gibco#17104-019; and the brand article No. of the PBS is Gibco#21-040-CVR.

The sample preservation solution is composed of fetal bovine serum, antibiotic-antimycotic (penicillin-streptomycin-amphotericin B), HEPES and HBSS (Hank's balanced salt solution); wherein the final concentration of the fetal bovine serum is 1-5% (such as 2%,% represents the volume percentage); the final concentration of penicillin in the antibiotic-antimycotic (penicillin-streptomycin-amphotericin B) is 100-200U/mL (such as 100 U/mL); the final concentration of streptomycin in the antibiotic-antimycotic (penicillin-streptomycin-amphotericin B) is 100-200µg /mL (such as 100µg /mL); the final concentration of amphotericin B in the antibiotic-antimycotic (penicillin-streptomycin-amphotericin B) is 250-500 ng /mL (such as 250 ng /mL); the final concentration of the HEPES is 8-12mM (such as 10 mM); and the rest is HBSS.

Further, the composition of the antibiotic-antimycotic (penicillin-streptomycin-amphotericin B) is as follows: each milliliter is composed of 10,000 units of penicillin (base), 10,000 µg of streptomycin (base) and 25 µg of amphotericin B. The antibiotic-antimycotic (penicillin-streptomycin-amphotericin B) is "Antibiotic-Antimycotic, 100X" (such as Gibco#15240062, or other products with the same composition). Each milliliter of "Antibiotic-Antimycotic, 100X" is composed of 10000 units of penicillin (base), 10000 µg of streptomycin (base) and 25 µg of amphotericin B, using penicillin G (sodium salt), streptomycin sulfate and amphotericin B in a form of 0.85% salt solution as a Fungizone^{®} antimycotic agent.

In the embodiments of the present invention, the brand article No. of the fetal bovine serum is Gibco#16000-044; the brand article No. of the antibiotic-antimycotic (penicillin-streptomycin-amphotericin B) is Gibco#15240062; the brand article No. of the HEPES is Gibco# 15630080; the brand article No. of the HBSS is Gibco# 14170161.

The sample washing solution is composed of antibiotic-antimycotic (penicillin-streptomycin-amphotericin B) and PBS; wherein the final concentration of penicillin in the antibiotic-antimycotic (penicillin-streptomycin-amphotericin B) is 100-200 U/mL (such as 100 U/mL); the final concentration of streptomycin in the antibiotic-antimycotic (penicillin-streptomycin-amphotericin B) is 100-200 µg /mL (such as 100 µg /mL); the final concentration of amphotericin B in the antibiotic-antimycotic (penicillin-streptomycin-amphotericin B) is 250-500 ng /mL (such as 250 ng /mL); and the rest is PBS.

Further, the composition of the antibiotic-antimycotic (penicillin-streptomycin-amphotericin B) is as follows: each milliliter is composed of 10,000 units of penicillin (base), 10,000 µg of streptomycin (base) and 25 µg of amphotericin B. The antibiotic-antimycotic (penicillin-streptomycin-amphotericin B) is "Antibiotic-Antimycotic, 100X" (such as Gibco#15240062, or other products with the same composition). Each milliliter of "Antibiotic-Antimycotic, 100X" contains 10,000 units of penicillin (base), 10,000 µg of streptomycin (base) and 25 µg of amphotericin B, using penicillin G (sodium salt), streptomycin sulfate and amphotericin B in a form of 0.85% salt solution as a Fungizone^{®} antimycotic agent.

In the embodiments of the present invention, the brand article No. of the antibiotic-antimycotic (penicillin-streptomycin-amphotericin B) is Gibco#15240062; and the brand article No. of the PBS is Gibco#21-040-CVR.

The cell isolation buffer is composed of P/S (penicillin-streptomycin), heparin sodium and PBS; wherein the final concentration of penicillin in the P/S (penicillin-streptomycin) is 100-200 U/mL (such as 100 U/mL); the final concentration of streptomycin in the P/S (penicillin-streptomycin) is 100-200 µg /mL (such as 100 µg /mL); the final concentration of the heparin sodium is 10IU/mL; and the rest is PBS.

In the embodiments of the present invention, the brand article No. of the P/S (penicillin-streptomycin) is Gibco#15140122; the brand article No. of the heparin sodium is Solarbio#H8270; and the brand article No. of the PBS is Gibco#21-040-CVR.

The composition of the cell digestion solution is as follows: each 10mL of the cell digestion solution contains 4-6mL (such as 5mL) of Accutase, EDTA with a final concentration of 5mM (i.e. 10µL 0.5M EDTA) and 1.5-2.5mL (such as 2mL) of TrypLE Express, and the rest is PBS.

Further, the Accutase is "StemPro^{™} Accutase^{™} Cell Dissociation Reagent" (such as Gibco#A11105-01, or other products with the same composition). The Accutase is a single-component enzyme dissolved in a D-PBS, 0.5 mM EDTA solution. The TrypLE Express is "TrypLE^{™} Express Enzyme (1X), no phenol red" (such as Gibco#12604013, or other products with the same composition). The "TrypLE^{™} Express Enzyme (1X), no phenol red" is composed of 200mg/L KCl, 200 mg/L KH₂PO₄, 8000 mg/L NaCl, 2160 mg/L Na₂HPO₄·7H₂O and 457.6mg/L EDTA; and the recombinant protease.

In the embodiments of the present invention, the brand article No. of the Accutase is Gibco#A11105-01; the brand article No. of the 0.5M EDTA is Invitrogen#AM9261; the brand article No. of the TrypLE Express is Gibco#12604013; and the brand article No. of the PBS is Gibco#21-040-CVR.

The digestion termination solution is composed of fetal bovine serum, antibiotic-antimycotic (penicillin-streptomycin-amphotericin B) and DMEM culture medium; wherein the final concentration of the fetal bovine serum is 8-12% (such as 10%, % means the volume percentage); the final concentration of penicillin in the antibiotic-antimycotic (penicillin-streptomycin-amphotericin B) is 100-200 U/mL (such as 100 U/mL); the final concentration of streptomycin in the antibiotic-antimycotic (penicillin-streptomycin-amphotericin B) is 100-200 µg /mL (such as 100 µg /mL); the final concentration of amphotericin B in the antibiotic-antimycotic (penicillin-streptomycin-amphotericin B) is 250-500 ng /mL (such as 250 ng /mL); and the rest is the DMEM culture medium.

Further, the composition of the antibiotic-antimycotic (penicillin-streptomycin-amphotericin B) is as follows: each milliliter is composed of 10,000 units of penicillin (base), 10,000 µg of streptomycin (base) and 25 µg of amphotericin B. The antibiotic-antimycotic (penicillin-streptomycin-amphotericin B) is "Antibiotic-Antimycotic, 100X" (such as Gibco#15240062, or other products with the same composition). Each milliliter of "Antibiotic-Antimycotic, 100X" contains 10,000 units of penicillin (base), 10,000 µg of streptomycin (base) and 25 µg of amphotericin B, with penicillin G (sodium salt), streptomycin sulfate and amphotericin B in a form of 0.85% salt solution as a Fungizone^{®} antimycotic agent.

In the embodiments of the present invention, the brand article No. of the fetal bovine serum is Gibco#16000-044; the brand article No. of the antibiotic-antimycotic (penicillin-streptomycin-amphotericin B) is Gibco#15240062; and the brand article No. of the DMEM culture medium is Gibco#11965-092.

The cell cryopreserving solution is composed of Advanced DMEM/F12 medium, DMSO and 1% methylcellulose solution; wherein the volume ratio of the Advanced DMEM/F12 medium, the DMSO and the 1% methylcellulose solution is 20:2: (0.8-1.2), such as 20:2:1; the 1% methylcellulose solution is an aqueous solution of methylcellulose with a concentration of 1g/100ml.

In the embodiments of the present invention, the brand article No. of the Advanced DMEM/F12 medium is Gibco#12634010; the brand article No. of the DMSO is Sigma#D2438; and the brand article No. of the methylcellulose is Sigma#M7027.

The sample preservation solution can be used for temporarily preserving the detached sample, so that the activity of cells in the sample can be maintained in a short time after the sample is detached. The prepared sample preservation solution can be preserved at 4°C for 1 month.

The sample washing solution can be used for washing and disinfection of samples. The sample washing solution is to be prepared just before use.

The sample dissociation solution can be used for sample dissociation, so that primary cells in solid tumor tissues of gastric cancer and/or gallbladder cancer and cholangiocarcinoma in the sample can be dissociated from tissues. The sample dissociation solution is to be prepared just before use, wherein collagenase I, collagenase II and collagenase IV can be preserved in a form of stock solution (mother solution) for a long time at -20°C, specifically 10 or 20× stock solution (mother solution). A 10× collagenase I stock solution is composed of the collagenase I and PBS; wherein the final concentration of the collagenase I is 2,000 U/mL. A 10× collagenase II stock solution is composed of the collagenase II and PBS; wherein the final concentration of the collagenase II is 2,000 U/mL; and the rest is PBS. A 20× collagenase IV stock solution is composed of the collagenase IV and PBS; wherein the final concentration of the collagenase IV is 2,000 U/mL; the rest is PBS. The enzyme activity of the collagenase I, collagenase II and collagenase IV are defined above.

The cell isolation buffer is used to suspend cells in a bile sample. The prepared cell isolation buffer can be preserved at 4°C for 1 month.

The cell digestion solution can be used for the digestion and passage of cell masses, so that the tumor masses of gastric cancer and/or gallbladder cancer and cholangiocarcinoma can be digested into individual cells. The cell digestion solution is to be prepared just before use.

The digestion termination solution can be used to terminate the process of sample dissociation or cell digestion. The prepared digestion termination solution can be preserved at 4°C for 1 month.

The primary cell culture medium for gastric cancer and/or gallbladder cancer and cholangiocarcinoma can be used to culture primary cells of gastric cancer and/or gallbladder cancer and cholangiocarcinoma.

The cell cryopreserving solution is to be prepared just before use. Wherein the 1% methylcellulose solution can be preserved at 4°C for a long time.

Described and not claimed *per se* is any of the following applications:
(B1) application of the culture medium previously described in the first aspect in culturing primary cells of gastric cancer and/or gallbladder cancer and cholangiocarcinoma;
(B2) application of the kit of reagents previously described in (A1) or (A3) in the second aspect in culturing primary cells in solid tumor tissues of gastric cancer and/or gallbladder cancer and cholangiocarcinoma;
(B3) application of the kit of reagents previously described in (A2) or (A4) in the second aspect in culturing primary tumor cells in a bile sample of gallbladder cancer and cholangiocarcinoma.

In the fourth aspect, the present invention claims an *in vitro* method for culturing primary cells of gastric cancer and/or gallbladder cancer and cholangiocarcinoma, according to claim 3.

According to claim 3, the *in vitro* method for culturing primary cells of gastric cancer and/or gallbladder cancer and cholangiocarcinoma claimed by the present invention is either *in vitro* method A or *in vitro* method B:
Method A: An *in vitro* method for culturing primary cells in obtained solid tumor tissues of gastric cancer and/or gallbladder cancer and cholangiocarcinoma, comprising the following steps:
   (a1) dissociating obtained solid tumor tissues of gastric cancer and/or gallbladder cancer and cholangiocarcinoma with the sample dissociation solution previously described in claim 2, to obtain primary cells in solid tumor tissues of gastric cancer and/or gallbladder cancer and cholangiocarcinoma;
   (a2) suspension-culturing the dissociated primary cells in solid tumor tissues of gastric cancer and/or gallbladder cancer and cholangiocarcinoma in step (a1) with the medium previously described in claim 1.
Method B: An *in vitro* method for culturing primary tumor cells in a bile sample of gallbladder cancer and cholangiocarcinoma, comprising the following steps:
   (b1) separating primary tumor cells in the bile sample of gallbladder cancer and cholangiocarcinoma, to obtain primary tumor cells in the bile sample of gallbladder cancer and cholangiocarcinoma;
   (b2) suspension-culturing the primary tumor cells in the bile sample of gallbladder cancer and cholangiocarcinoma separated in step (b1) with the medium previously described in claim 1.

Further, in step (a1), the obtained solid tumor tissues of gastric cancer and/or gallbladder cancer and cholangiocarcinoma can be dissociated with the sample dissociation solution according to the method comprising the following steps: according to the dosage of 0.1-0.3 mL (such as 0.1 mL) of sample dissociation solution per mg of tissue, treating the solid tumor tissues of gastric cancer and/or gallbladder cancer and cholangiocarcinoma, which were cut up (e.g. cut into 0.8-1.2 mm³ small pieces), with the sample dissociation solution preheated to 37°C in advance, dissociating the sample at 37°C for 15 minutes to 3 hours, and observing the dissociation of the sample under a microscope every 15 minutes until a large number of individual cells are observed.

Further, in step (b1), the primary tumor cells in the bile sample of gallbladder cancer and cholangiocarcinoma can be separated from the bile sample of gallbladder cancer and cholangiocarcinoma according to a method comprising the following steps: suspending the cells in the bile sample of gallbladder cancer and cholangiocarcinoma with the cell isolation buffer previously described in claim 2, and then obtaining the primary tumor cells in the bile sample of gallbladder cancer and cholangiocarcinoma by density gradient centrifugation (using a Ficoll lymphocyte separation medium).

Further, in step (a2), the primary cells in solid tumor tissues of gastric cancer and/or gallbladder cancer and cholangiocarcinoma can be suspension-cultured with said medium according to a method comprising the following steps: suspension-culturing the primary cells in solid tumor tissues of gastric cancer and/or gallbladder cancer and cholangiocarcinoma with the medium using a cell culture vessel M under the condition of 37°C, 5% CO₂, and replacing the medium every 2-4 days (such as 3 days) until the cells form masses with a diameter of 50-80µm (such as 80 µm).

Further, in step (b2), the primary tumor cells in the bile sample of gallbladder cancer and cholangiocarcinoma can be suspension-cultured with said medium according to a method comprising the following steps: suspension-culturing the primary tumor cells in the bile sample of gallbladder cancer and cholangiocarcinoma with the medium using a cell culture vessel M under the condition of 37°C, 5% CO₂, and replacing the medium every 2-4 days (such as 3 days) until the cells form masses with a diameter of 50-80µm (such as 80 µm).

Wherein the initial inoculation density can be 10⁵ cells/cm² vessel bottom area; a 6-well plate is taken as an example, and cells are planked at a density of 10⁶ cells per well.

Wherein the cell culture vessel M can be any of the following: (I) a cell culture vessel made of polystyrene, a cell culture vessel made of polycarbonate, a cell culture vessel made of polymethylmethacrylate, a cell culture vessel made of COC resin, a cell culture vessel made of cycloolefin polymer or a cell culture vessel with a low attachment surface; (II) a cell culture vessel after CYTOP modification of the cell culture vessel in (I).

Further, the cell culture vessel is a cell culture dish, cell culture well plate or microplate chip for cell culture.

In (II), the cell culture vessel in (I) can be CYTOP-modified according to a method comprising the following steps: performing pure oxygen etching on the cell culture vessel in (I) at an etching power of 20W for 3 minutes; then covering the surface of the cell culture vessel with 1% CYTOP solution, and drying the 1% CYTOP solution in the air to complete CYTOP modification.

Wherein the composition of the 1% CYTOP solution is as follows: each 100mL of the 1% CYTOP solution contains 1mL of CYTOP, and the rest is fluorocarbon oil.

Further, before step (a1), the following step for predissociation treatment of the obtained solid tumor tissues of gastric cancer and/or gallbladder cancer and cholangiocarcinoma can also be included: washing the surface of a solid tumor tissue sample of gastric cancer and/or gallbladder cancer and cholangiocarcinoma with 70-75% ethanol (by volume); washing the solid tumor tissue sample of gastric cancer and/or gallbladder cancer and cholangiocarcinoma for 10-20 times (such as 10 times) with the sample washing solution described in claim 2, and washing the solid tumor tissue sample of gastric cancer and/or gallbladder cancer and cholangiocarcinoma for 5-10 times (such as 5 times) with sterile PBS solution; then removing impurities, connective tissues, fatty tissues, necrotic tissues and other components affecting primary cell culture from the solid tumor tissue sample of gastric cancer and/or gallbladder cancer and cholangiocarcinoma.

The step of predissociation treatment of the solid tumor tissue sample of gastric cancer and/or gallbladder cancer and cholangiocarcinoma needs to be operated on ice, and the whole operation step needs to be completed within 10 minutes.

Further, the detachment time of the solid tumor tissue sample of gastric cancer and/or gallbladder cancer and cholangiocarcinoma before the predissociation treatment is within 2 hours, and the solid tumor tissue sample of gastric cancer and/or gallbladder cancer and cholangiocarcinoma is preserved in the sample preservation solution previously described in claim 2 before the predissociation treatment.

Further, in step (a1), the following steps are also included after dissociation treatment of the obtained solid tumor tissue of gastric cancer and/or gallbladder cancer and cholangiocarcinoma with the sample dissociation solution: terminating a dissociation reaction with 8-15 times (such as 10 times) the volume of the digestion termination solution described in claim 2, and collecting a cell suspension; filtering the cell suspension with a 100 µm or 40µm sterile cell strainer to remove tissue fragments and adherent cells; centrifuging at 800-1,000g (such as 800g) at a room temperature for 10-15 minutes (such as 10 minutes), and discarding a supernatant; resuspending cells with 3-5 mL (such as 5mL) sterile PBS; centrifuging at 800-1,000g (such as 800g) at a room temperature for 10-15 minutes (such as 10 minutes), and discarding a supernatant; then, resuspending the cell precipitation with the medium described in claim 1, observing the cell state under a microscope, and counting the cells.

Further, before step (b1), the step of pre-separation treatment of the bile sample of gallbladder cancer and cholangiocarcinoma can also be included: removing impurities, sludged blood and other components that affect the cell density gradient separation from the bile sample of gallbladder cancer and cholangiocarcinoma.

Further, before step (a2), the following step can also be included: passaging the primary cells in solid tumor tissues of gastric cancer and/or gallbladder cancer and cholangiocarcinoma when masses with a diameter of 50-80 µm (such as 80 µm) are formed by the primary cells in solid tumor tissues of gastric cancer and/or gallbladder cancer and cholangiocarcinoma.

Further, in step (b2), the following step can also be included: passaging the primary tumor cells in the bile sample of gallbladder cancer and cholangiocarcinoma when masses with a diameter of 50-80 µm (such as 80 µm) are formed by the primary tumor cells in the bile sample of gallbladder cancer and cholangiocarcinoma.

Wherein, the cell digestion solution for the passage is the cell digestion solution described in claim 2.

Wherein, the digestion termination solution for the passage is the digestion termination solution described in claim 2.

Further, the digestion temperature for the passage is 37°C.

More specifically, the steps of performing the passage are as follows: collecting the cell masses to be passaged, washing the cell mass with sterile PBS solution after centrifugation, resuspending the cell masses with the cell digestion solution after centrifugation, digesting the cell masses at 37°C until all are digested into individual cells, terminating the digestion reaction with the digestion termination solution (the dosage can be 5-10 times the volume, such as 10 times the volume), and collecting the cell suspension; after centrifugation, resuspending the cell precipitation with the medium previously described in the first aspect, counting, and then suspension-culturing the cells in the cell culture vessel M previously described (the initial inoculation density can be 10⁵ cells/cm² vessel bottom area; a 6-well plate is taken as an example, and cells are planked at a density of 10⁶ cells per well) under the condition of 37°C, 5% CO₂. All the centrifugations in the above passage steps can be ones at 800-1,000g (such as 800g) at a room temperature for 10-20 minutes (such as 10 minutes).

Further, the method can also comprise a step of cryopreserving and/or resuscitating the primary cells in solid tumor tissues of gastric cancer and/or gallbladder cancer and cholangiocarcinoma or the primary tumor cells in the bile sample of gallbladder cancer and cholangiocarcinoma after 2-3 passages and amplifications.

The cell cryopreserving solution for the cryopreserving is the cell cryopreserving solution described in claim 2.

Further, the specific steps of performing the cryopreserving are as follows: collecting the cell masses to be cryopreserved, washing the cell mass with sterile PBS solution after centrifugation, resuspending the cell masses with the cell digestion solution after centrifugation, digesting the cell masses at 37°C until all are digested into individual cells, terminating the digestion reaction with the digestion termination solution (the dosage can be 5-10 times the volume, such as 10 times the volume), and collecting the cell suspension; after centrifugation, resuspending the cell precipitation with the cell cryopreserving solution at a density of 0.5-2×10⁶/mL (such as 10⁶/mL), cryopreserving in a gradient cooling box overnight and transferring into liquid nitrogen for long-term preservation. All the centrifugations in the above cryopreserving steps can be ones at 800-1,000g (such as 800g) at a room temperature for 10-20 minutes (such as 10 minutes).

Further, the specific steps of performing the resuscitating are as follows: removing a cryopreserving tube containing the cells to be resuscitated from liquid nitrogen, and quickly melting the cells in sterile water at 37-39°C (such as 37°C); after centrifugation (such as at 800-1,000g, e.g. centrifugation at 800g at a room temperature for 5-10 minutes, such as 10 minutes), resuspending the cell precipitation with the medium previously described in the first aspect, then suspension-culturing the cells in the cell culture vessel M previously described (the initial inoculation density can be 10⁵ cells/cm² vessel bottom area), and resuscitating the cells (10⁶ cells) in each tube to a 3.5 cm culture dish under the condition of 37°C, 5% CO₂.

Described and not claimed *per se* is any of the following reagents:
(C1) The sample dissociation solution for solid tumor tissues of gastric cancer and/or gallbladder cancer and cholangiocarcinoma is the sample dissociation solution previously described in the second aspect;
(C2) The sample preservation solution for solid tumor tissues of gastric cancer and/or gallbladder cancer and cholangiocarcinoma is the sample preservation solution previously described in the second aspect;
(C3) The isolation buffer of the bile sample of gallbladder cancer and cholangiocarcinoma is the cell isolation buffer previously described in the second aspect.

Described and not claimed *per se* is any of the following applications:
(D1) application of the sample dissociation solution previously described in (C1) in the fifth aspect in dissociating the primary cells in solid tumor tissues of gastric cancer and/or gallbladder cancer and cholangiocarcinoma from the solid tumor tissues of gastric cancer and/or gallbladder cancer and cholangiocarcinoma.
(D2) application of the sample preservation solution previously described in (C2) in the fifth aspect in preserving the solid tumor tissues of gastric cancer and/or gallbladder cancer and cholangiocarcinoma.
(D3) application of the cell isolation buffer previously described in (C3) in the fifth aspect in isolating the primary tumor cells in the bile sample of gallbladder cancer and cholangiocarcinoma from the bile sample of gallbladder cancer and cholangiocarcinoma.

Described and not claimed *per se* is any of the following methods:
(E1) A method for dissociating primary cells in solid tumor of gastric cancer and/or gallbladder cancer and cholangiocarcinoma from the solid tumor tissues of gastric cancer and/or gallbladder cancer and cholangiocarcinoma, comprising step (a1) in the method previously described in the fourth aspect.
(E2) A method for preserving solid tumor tissues of gastric cancer and/or gallbladder cancer and cholangiocarcinoma, comprising the following steps: preserving solid tumor tissues of gastric cancer and/or gallbladder cancer and cholangiocarcinoma just detached in the sample preservation solution previously described in the second aspect for no more than 2 hours.
(E3) A method for isolating primary tumor cells in the bile sample of gallbladder cancer and cholangiocarcinoma from the bile sample of gallbladder cancer and cholangiocarcinoma, comprising step (b1) in the method previously described in the fourth aspect.

In the above aspects, the gastric cancer can be primary gastric cancer; and the gallbladder cancer and cholangiocarcinoma can be primary gallbladder cancer and cholangiocarcinoma.

In the above aspects, the gastric cancer can be a metastatic lesion of gastric cancer; and the gallbladder cancer and cholangiocarcinoma can be a metastatic lesion of gallbladder cancer and cholangiocarcinoma.

In the above aspects, the primary cells of gastric cancer can be primary cells in solid tumor tissues of gastric cancer; and the primary cells of gallbladder cancer and cholangiocarcinom can be primary cells in solid tumor tissues of gallbladder cancer and cholangiocarcinoma or primary tumor cells in the bile sample of gallbladder cancer and cholangiocarcinoma.

In the above aspects, the primary cells of gastric cancer can be isolated from surgical samples of a patient with gastric cancer; and the primary cells of gallbladder cancer and cholangiocarcinoma can be isolated from a surgical sample (solid tumor tissue sample), a puncture sample (solid tumor tissue sample) or a bile sample.

In the above aspects, the clinical staging of the gastric cancer refers to stage II, III or IV (by TNM), wherein the surgical specimen is a sample weighing more than 20 mg.

In the above aspects, the clinical staging of the gallbladder cancer and cholangiocarcinoma refers to stage II, III or IV (by TNM), wherein the solid tumor tissue specimen of gallbladder cancer and cholangiocarcinoma obtained from the surgical sample preferably weighs more than 20 mg. The bile sample is preferably not less than 10mL. The number of puncture samples is not less than 4.

In the present invention, all the above described PBSs can be 1×PBS, pH7.3-7.5. The specific composition is as follows: the solvent is water, and the solute and concentration are as follows: KH₂PO₄ 144mg/L, NaCl 9,000 mg/L, Na₂HPO₄·7H₂O 795 mg/L.

### BRIEF DESCRIPTION OF THE DRAWINGS

FIG. 1 illustrates the single cells obtained from a gastric cancer tissue after treatment. The scale is 100µm, subject to 100× magnification.
FIG. 2 illustrates the cell masses obtained from a gastric cancer tissue after primary culture. The scale is 100µm, subject to 100× magnification.
FIG. 3 illustrates a HE staining image of a gastric cancer cell mass section obtained from a gastric cancer tissue after primary culture. The scale is 100µm, subject to 200× magnification.
FIG. 4 illustrates an immunofluorescence staining image of cancer cell masses obtained from a gastric cancer tissue after primary culture. The scale is 50 µm, subject to 200× magnification.
FIG. 5 illustrates a copy number variation (CNV) analysis based on the sequencing results, showing that the copy number variation of all generations of primary cell cultures of gastric cancer (P1, P2, P3, P4 and P5) is highly consistent with that of the primary tumor tissue (Tumor) of gastric cancer.
FIG. 6 illustrates the results of an in vitro drug sensitivity test on the primary cells of gastric cancer cultured by using the present invention.
FIG. 7 illustrates the single cells obtained from a cholangiocarcinoma tissue after treatment. The scale is 100µm, subject to 100× magnification.
FIG. 8 illustrates the cell masses obtained from a cholangiocarcinoma tissue after primary culture. The scale is 100µm, subject to 100× magnification.
FIG. 9 illustrates a HE staining image of a cholangiocarcinoma cell mass section obtained from a cholangiocarcinoma tissue after primary culture. The scale is 100µm, subject to 200× magnification.
FIG. 10 illustrates an immunohistochemical staining image of a cancer cell mass paraffin section obtained from a cholangiocarcinoma tissue after primary culture. The scale is 100µm, subject to 200× magnification.
FIG. 11 illustrates the single cells obtained from a cholangiocarcinoma bile sample after treatment. The scale is 100µm, subject to 100× magnification.
FIG. 12 illustrates the cell masses obtained from a cholangiocarcinoma bile sample after primary culture. The scale is 100µm, subject to 100× magnification.
FIG. 13 illustrates a HE staining image of a cholangiocarcinoma cell mass section obtained from a cholangiocarcinoma bile sample after primary culture. The scale is 100 µm, subject to 100× magnification.
FIG. 14 illustrates an immunohistochemical staining image of a cancer cell mass paraffin section obtained from a cholangiocarcinoma bile sample after primary culture. The scale is 50 µm, subject to 100× magnification.
FIG. 15 illustrates a design view of a microplate chip of the present invention.

### DETAILED DESCRIPTION OF THE EMBODIMENTS

The following embodiments are intended for a better understanding of the present invention, but not limiting the present invention. Unless otherwise specified, all the experimental methods in the following embodiments are conventional methods. Unless otherwise specified, all the test materials used in the following embodiments are available from the conventional biochemical stores. All the quantitative tests in the following embodiments are provided with three repeated experiments, and the average value of the results is taken.

### Embodiment 1. Preparing a reagent for culturing primary cells of gastric cancer

### 1. Sample preservation solution (100 mL)

The specific formula of sample preservation solution (100 mL) is as shown in Table 1.

**Table 1 Sample preservation solution (100 mL)**

| Reagent | Brand Article No. | Dosage | Final Concentration |
|---|---|---|---|
| Fetal bovine serum | Gibco#16000-044 | 2 mL | 2% |
| Antibiotic-antimycotic | Gibco#15240062 | 1 mL | 1% |
| HEPES | Gibco# 15630080 | 1 mL | 10 mM |
| HBSS | Gibco# 14170161 | Replenish to 100 mL | |

After being prepared, the sample preservation solution was divided in 15 mL centrifuge tubes, and each tube was filled with 5 mL. After aliquoting, the sample preservation solution can be preserved at 4°C for 1 month .

### 2. Sample washing solution (100 mL)

The specific formula of sample washing solution (100 mL) is as shown in Table 2.

**Table 2 Sample washing solution (100 mL)**

| Reagent | Brand Article No. | Dosage | Final Concentration |
|---|---|---|---|
| Antibiotic-antimycotic | Gibco#15240062 | 1 mL | 1% |
| PBS | Gibco#21-040-CVR | Replenish to 100 mL | |

The sample washing solution is to be prepared just before use.

### 3. Sample dissociation solution (10 mL)

The specific formula of sample dissociation solution (10 mL) is as shown in Table 3.

**Table 3 Sample dissociation solution (10 mL)**

| Reagent | Brand Article No. | Dosage | Final Concentration |
|---|---|---|---|
| 10× collagenase I | 10× stock solution | 1 mL | 200 U/mL |
| 10× collagenase II | 10× stock solution | 1 mL | 200 U/mL |
| 20× collagenase IV | 20× stock solution | 0.5 mL | 100 U/mL |
| PBS | Gibco#21-040-CVR | Replenish to 10 mL | |

Note: The sample dissociation solution is to be prepared just before use.

In Table 3, the preparation of collagenase stock solution is as shown in Tables 4-6.

**Table 4 10×collagenase I stock solution (100 mL)**

| Reagent | Brand Article No. | Dosage | Final Concentration |
|---|---|---|---|
| Collagenase I | Gibco #17100-017 | 1 g | 2000U/mL |
| PBS | Gibco#21-040-CVR | Replenish to 100 mL | |

After being prepared, the 10×collagenase I stock solution was divided in 1.5 mL sterile centrifuge tubes, and each tube was filled with 1 mL. The stock solution can be kept at -20 °C for a long term.

**Table 5 10×collagenase II stock solution (100 mL)**

| Reagent | Brand Article No. | Dosage | Final Concentration |
|---|---|---|---|
| Collagenase II | Gibco # 17101-015 | 1 g | 2000U/mL |
| PBS | | Gibco#21-040-CVR | Replenish to 100 mL |

After being prepared, the 10×collagenase II stock solution was divided in 1.5 mL sterile centrifuge tubes, and each tube was filled with 1 mL. The stock solution can be kept at -20°C for a long term.

**Table 6 20×collagenase IV stock solution (100 mL)**

| Reagent | Brand Article No. | Dosage | Final Concentration |
|---|---|---|---|
| Collagenase IV | Gibco #17104-019 | 1 g | 2000 U/mL |
| PBS | Gibco#21-040-CVR | Replenish to 100 mL | |

After being prepared, the 20×collagenase IV stock solution was divided in 1.5 mL sterile centrifuge tubes, and each tube was filled with 1 mL. The stock solution can be preserved at -20°C for a long term.

In Tables 4, 5 and 6, the unit U of the collagenase (the collagenase I or the collagenase IV) is defined by the enzyme activity of protease: 1 µmol of L-leucine can be released by treating the collagenase (the collagenase I or the collagenase IV) with 1U of protease for 5 hours at 37°C and pH 7.5.

### 4. Cell digestion solution (10 mL)

The specific formula of cell digestion solution (10 mL) is as shown in Table 7.

**Table 7 Cell digestion solution (10 mL)**

| Reagent | Brand Article No. | Dosage | Final Concentration |
|---|---|---|---|
| Accutase | Gibco#A11105-01 | 5 mL | |
| 0.5M EDTA | Invitrogen#AM9261 | 10 µL | 5 mM |
| TrypLE Express | Gibco#12604013 | 2 mL | |
| PBS | Gibco#21-040-CVR | Replenish to 10 mL | |

The cell digestion solution is to be prepared just before use.

### 5. Digestion termination solution (100 mL)

The specific formula of digestion termination solution (100 mL) is as shown in Table 8.

**Table 8 Digestion termination solution (100 mL)**

| Reagent | Brand Article No. | Dosage | Final Concentration |
|---|---|---|---|
| Fetal bovine serum | Gibco#16000-044 | 10 mL | 10% |
| Antibiotic-antimycotic | Gibco#15240062 | 1 mL | 1% |
| DMEM culture medium | Gibco#11965-092 | Replenish to 100 mL | |

The prepared digestion termination solution can be preserved at 4°C for 1 month.

### 6. Primary cell culture medium for solid tumor tissues of gastric cancer (100 mL)

The specific formula of primary cell culture medium for solid tumor tissues of gastric cancer (100 mL) is as shown in Table 9.

**Table 9 Primary cell culture medium for solid tumor tissues of gastric cancer (100 mL)**

| Reagent | Brand Article No. | Dosage | Final Concentration |
|---|---|---|---|
| Antibiotic-antimycotic | Gibco#15240062 | 1 mL | 1% |
| HEPES | Gibco#15630080 | 1 mL | 10 mM |
| GlutaMax | Gibco#35050061 | 1 mL | 1% |
| Non-essential amino acids | Gibco#11140050 | 1mL | 1% |
| 1000× human recombinant protein EGF | 1000× stock solution | 50-500 µL | 10-100 ng/mL |
| 1000× human recombinant protein bFGF | 1000× stock solution | 50-250 µL | 10-50 ng/mL |
| 1000× human recombinant protein HGF | 1000× stock solution | 25-125 µL | 5-25 ng/mL |
| 1000× human recombinant protein FGF-10 | 1000× stock solution | 25-125 µL | 5-25 ng/mL |
| 1000× human recombinant | 1000× stock solution | 100-150 µL | 200-300 ng/mL |
| protein Wnt-3a | | | |
| 1000× human recombinant protein Noggin | 1000× stock solution | 100-200 µL | 100-200 ng/mL |
| SB202190 | 1000× stock solution | 50-100 µL | 5-10 µM |
| A83-01 | 100000× stock solution | 1-5 µL | 0.25-1.25 µM |
| Primocin^{™} | Invivogene#ant-pm-1 | 1 mL | 1% |
| N-acetyl-L-cysteine | 1000× stock solution | 100-400 µL | 0.5-2 mM |
| Nicotinamide | 1000× stock solution | 100-200 µL | 5-10 mM |
| N-2 Supplement | Gibco#17502001 | 1 mL | 1% |
| Cortisol | 1000× stock solution | 80-200 µL | 20-50 ng/mL |
| B27 | Gibco#12587010 | 2 mL | 2% |
| ITS-X | Gibco#51500056 | 1 mL | 1% |
| Gastrin | 1000× stock solution | 100µL | 10nM |
| Y-27632 | 1000× stock solution | 50-200 µL | 5-20 µM |
| Advanced DMEM/F12 culture medium | Gibco#12634010 | Replenish to 100 mL | |

After being prepared, the primary cell culture medium for solid tumor tissues of gastric cancer was filtered and sterilized with a 0.22 µM syringe-driven filter (Millipore SLGP033RS). The culture medium can be preserved at 4°C for two weeks.

In Table 9, the preparation of human recombinant protein stock solution is as shown in Table 11-Table 16, the preparation of SB202190 stock solution is as shown in Table 17, the preparation of A83-01 stock solution is as shown in Table 18, the preparation of N-acetyl-L-cysteine stock solution is as shown in Table 19, the preparation of Nicotinamide stock solution is as shown in Table 20, the preparation of cortisol stock solution is as shown in Table 21, the preparation of gastrin stock solution is as shown in Table 22, and the preparation of Y-27632 stock solution is as shown in Table 23. The preparation of 100× BSA solution required in preparation of these stock solutions is as shown in Table 10.

**Table 10 100× BSA solution (1 mL)**

| Reagent | Brand Article No. | Dosage | Final Concentration |
|---|---|---|---|
| BSA | Sigma#A1933 | 0.1 g | 0.1 g/mL |
| PBS | Gibco#21-040-CVR | Replenish to 1 mL | |
| 100× BSA solution is to be prepared just before use. | | | |

| Table 11 1,000× human recombinant protein EGF stock solution (5 mL) | | | |
|---|---|---|---|
| Reagent | Brand Article No. | Dosage | Final Concentration |
| Human recombinant protein EGF | Peprotech AF-100-15-100 | 100 µg | mL |
| 100× BSA solution | | 500 µL | O.Olg/mL |
| PBS | Gibco#21-040-CVR | Replenish to 5 mL | |
| After being prepared, the 1,000x human recombinant EGF stock sloution was divided in 1.5 ,L sterile centrifuge tubes.The stock solution can be preserved at -80^{○} C for a long term. | | | |

| Table 12 1,000× human recombinant protein bFGF stock solution (2.5 mL) | | | |
|---|---|---|---|
| Reagent | Brand Article No. | Dosage | Final Concentration |
| Human recombinant protein bFGF | Peprotech AF-100-18B-50 | 50 µg | 20 µg/mL |
| 100× BSA solution | | 250 µL | O.Olg/mL |
| PBS | Gibco#21-040-CVR | Replenish to 2.5 mL | |

After being prepared, the 1,000× human recombinant protein bFGF stock solution was divided in 1.5 mL sterile centrifuge tubes. The stock solution can be preserved at -80°C for a long term.

**Table 13 1,000× human recombinant protein HGF stock solution (5 mL)**

| Reagent | Brand Article No. | Dosage | Final Concentration |
|---|---|---|---|
| Human recombinant | Peprotech AF-100-39-100 | 100 µg | 20 µg/mL |
| protein HGF | | | |
| 100× BSA solution | | 500 µL | 0.01g/mL |
| PBS | Gibco#21-040-CVR | Replenish to 5 mL | |

After being prepared, the 1,000× human recombinant protein HGF stock solution was divided in 1.5 mL sterile centrifuge tubes. The stock solution can be preserved at -80°C for a long term.

**Table 14 1,000× human recombinant protein FGF-10 stock solution (5 mL)**

| Reagent | Brand Article No. | Dosage | Final Concentration |
|---|---|---|---|
| Human recombinant protein FGF-10 | Peprotech AF-100-26-100 | 100 µg | 20 µg/mL |
| 100× BSA solution | | 500 µL | 0.01g/mL |
| PBS | Gibco#21-040-CVR | Replenish to 5 mL | |

After being prepared, the 1,000× human recombinant protein FGF-10 stock solution was divided in 1.5 mL sterile centrifuge tubes. The stock solution can be preserved at -80°C for a long term.

**Table 15 1,000× human recombinant protein Wnt-3a stock solution (2.5 mL)**

| Reagent | Brand Article No. | Dosage | Final Concentration |
|---|---|---|---|
| Human recombinant protein Wnt-3a | R&D 5036-WN-500 | 500 µg | 200 µg/mL |
| 100× BSA solution | | 250 µL | 0.01g/mL |
| PBS | Gibco#21-040-CVR | Replenish to 2.5 mL | |

After being prepared, the 1,000× human recombinant protein Wnt-3a stock solution was divided in 1.5 mL sterile centrifuge tubes. The stock solution can be preserved at -80°C for a long term.

**Table 16 1,000× human recombinant protein Noggin stock solution (5 mL)**

| Reagent | Brand Article No. | Dosage | Final Concentration |
|---|---|---|---|
| Human recombinant Noggin | Shanghai nearshore protein #C018 | 500 µg | 100 µg/mL |
| 100× solution | BSA | 500 µL | 0.01g/mL |
| PBS | Gibco#21-040-CVR | Replenish to 5 mL | |

After being prepared, the 1,000× human recombinant protein Noggin stock solution was divided in 1.5 mL sterile centrifuge tubes. The stock solution can be preserved at -80°C for a long term.

**Table 17 1,000×SB202190 stock solution (1.51mL)**

| Reagent | Brand Article No. | Dosage | Final Concentration |
|---|---|---|---|
| SB202190 | Sigma#S7067 | 5 mg | 10 mM |
| DMSO | | Replenish to 1.51 mL | |
| After being prepared, the 1,000×SB202190 stock solution was divided in 0.5 mL sterile centrifuge tubes. The stock solution can be preserved at -20°C for a long term. | | | |

| Table 18 100,000×A83-01 stock solution (1.05mL) | | | |
|---|---|---|---|
| Reagent | Brand Article No. | Dosage | Final Concentration |
| A83-01 | Tocris#2939 | 10 mg | 25 mM |
| DMSO | | Replenish to 1.05 mL | |

After being prepared, the 1,000×A83-01 stock solution was divided in 0.5 mL sterile centrifuge tubes. The stock solution can be preserved at -20°C for a long term.

**Table 19 1,000×N-acetyl-L-cysteine stock solution (5mL)**

| Reagent | Brand Article No. | Dosage | Final Concentration |
|---|---|---|---|
| N-acetyl-L-cysteine | Sigma#A9165 | 0.41 g | 0.5 M |
| Ultrapure water | | Replenish to 5 mL | |

After being prepared, the 1,000×N-acetyl-L-cysteine stock solution was divided in 0.5 mL sterile centrifuge tubes. The stock solution can be preserved at -20°C for a long term.

**Table 20 1,000×Nicotinamide stock solution (4mL)**

| Reagent | Brand Article No. | Dosage | Final Concentration |
|---|---|---|---|
| Nicotinamide | Sigma#N0636 | 2.44 g | 5 M |
| Ultrapure water | | Replenish to 4 mL | |

After being prepared, the 1,000×Nicotinamide stock solution was divided in 0.5 mL sterile centrifuge tubes. The stock solution can be preserved at -20°C for a long term.

**Table 21 1,000× cortisol stock solution (100 mL)**

| Reagent | Brand Article No. | Dosage | Final Concentration |
|---|---|---|---|
| Cortisol | Sigma#H0888 | 2.5 mg | 25 µg/mL |
| Absolute ethyl alcohol | Sigma#E7023 | 5 mL | 5% |
| Ultrapure water | | Replenish to 100 mL | |

After being prepared, the 1,000× cortisol stock solution was divided in 1.5 mL sterile centrifuge tubes. The stock solution can be preserved at -20°C for a long term.

**Table 22 1,000× gastrin stock solution (48mL)**

| Reagent | Brand Article No. | Dosage | Final Concentration |
|---|---|---|---|
| Gastrin | NJPeptide#Pep12307 | 1 mg | 10µM |
| Ultrapure | | Replenish to 48 mL | |
| water | | | |

After being prepared, the 1,000× gastrin stock solution was divided in 0.5 mL sterile centrifuge tubes. The stock solution can be preserved at -20°C for a long term.

**Table 23 1,000× Y-27632 stock solution (3.125mL)**

| Reagent | Brand Article No. | Dosage | Final Concentration |
|---|---|---|---|
| Y-27632 | MCE#129830-38-2 | 10 mg | 10 mM |
| Ultrapure water | | Replenish to 3.125 mL | |

After being prepared, the 1000×Y-27632 stock solution was divided in 0.5 mL sterile centrifuge tubes. The stock solution can be preserved at -20°C for a long term.

### 7. Cell cryopreserving solution

The specific formula of cell cryopreserving solution is as shown in Table 24.

**Table 24 Cell cryopreserving solution**

| Reagent | Brand Article No. | Dosage |
|---|---|---|
| Advanced DMEM/F12 culture medium | Gibco#12634010 | 20 mL |
| DMSO | Sigma#D2438 | 2 mL |
| 1% methylcellulose solution | | 1 mL |

The cell cryopreserving solution is to be prepared just before use.

In Table 24, the preparation of 1% methylcellulose solution is as shown in Table 25.

**Table 25 1% methylcellulose solution (10 mL)**

| Reagent | Brand Article No. | Dosage | Final Concentration |
|---|---|---|---|
| Methylcellulose | Sigma#M7027 | 0.1 g | 10g/L |
| Ultrapure water | | Replenish to 10 mL | |

The prepared 1% methylcellulose solution can be preserved at 4°C for a long time.

### 8. 1% CYTOP solution

**Table 26 1% CYTOP solution (100 mL)**

| Reagent | Brand Article No. | Dosage | Final Concentration |
|---|---|---|---|
| CYTOP | Asashi glass#CTL-809M | 1 mL | 1% |
| Fluorocarbon oil | 3M# FC40 | Replenish to 100 mL | |

The prepared 1% CYTOP solution can be preserved at a normal temperature for a long time.

### Embodiment 2. Obtaining postoperative specimen of gastric cancer

1. The inventor cooperated with national triple A, first-class hospitals in China, and the cooperation passed formal medical ethical review.
2. The attending physician selected the enrolled patients according to the clinical indications specified in the medical guidelines, and selected suitable samples for in vitro culture according to the intraoperative clinical indications. The selection criteria of samples were as follows: primary gastric cancer, with pathological staging of stage II, III or IV, gastric cancer or metastatic lesions of various pathological types, and samples with the surgical specimen of gastric cancer weighing more than 20 mg.
3. The attending physician provided the patient's basic clinical information, such as gender, age, medical history, family history, smoking history, pathological stages and types, and clinical diagnosis. The information related to patient privacy, such as patient's name and ID number, was concealed and replaced by a unified experimental number, and the naming principle of the experimental number was the 8-digit date of sample collection + last four digits of the patient's admission number. For example, for a sample provided on January 1, 2018, when the patient's admission number was T001512765, then the sample experiment number was 201801012765.
4. During the operation, a surgeon collected a fresh specimen in a sterile environment of an operating room and placed it in a sample preservation solution prepared in advance (see Embodiment 1). After being detached, the sample was temporarily stored on ice and transported to a laboratory within two hours for the next operation.

### Embodiment 3. Performing predissociation treatment for tissue sample of gastric cancer

The following steps were operated on ice, and the whole operation process was completed within 10 minutes.

All the surgical apparatuses used in the following actions were used only after high-temperature and high-pressure sterilization and oven drying in advance.
1. The sample was weighed.
2. The sample surface was washed with 75% (volume percentage) ethanol for 10 to 30 seconds.
3. The sample was washed with sample washing solution for 10 times and with sterile PBS solution for 5 times.
4. Fatty tissues, connective tissues and necrotic tissues in the sample were carefully peeled off with ophthalmic scissors, ophthalmic forceps, scalpel and other apparatuses.

### Embodiment 4. Dissociating tissue sample of gastric cancer

All the surgical apparatuses used in the following actions were used only after high-temperature and high-pressure sterilization and oven drying in advance.
1. The tissues were cut into small pieces of about 1mm³ with ophthalmic scissors.
2. The tissue sample cut into pieces was treated with sample dissociation solution preheated to 37°C in advance according to the dosage of 0.1 mL of sample dissociation solution (see Embodiment 1) per mg of tissue, and the sample was dissociated at 37°C for 15 minutes to 3 hours. The dissociation of the sample was observed under a microscope every 15 minutes until a large number of individual cells were observed.
3. The dissociation reaction was terminated with a 10× volume of digestion termination solution (see Embodiment 1), and the cell suspension was collected.
4. The cell suspension was filtered with a 40 µm sterile cell strainer to remove tissue fragments and adherent cells.
5. The cell suspension was centrifugated at 800g at a room temperature for 10 minutes, and a supernatant was discarded.
6. The cells were resuspended with 5mL of sterile PBS and centrifugated at 800g at a room temperature for 10 minutes, and a supernatant was discarded.
7. The cell precipitation was resuspended with the primary cell culture medium for solid tumor tissues of gastric cancer (see Embodiment 1), the cell state was observed under a microscope, and the cells were counted.

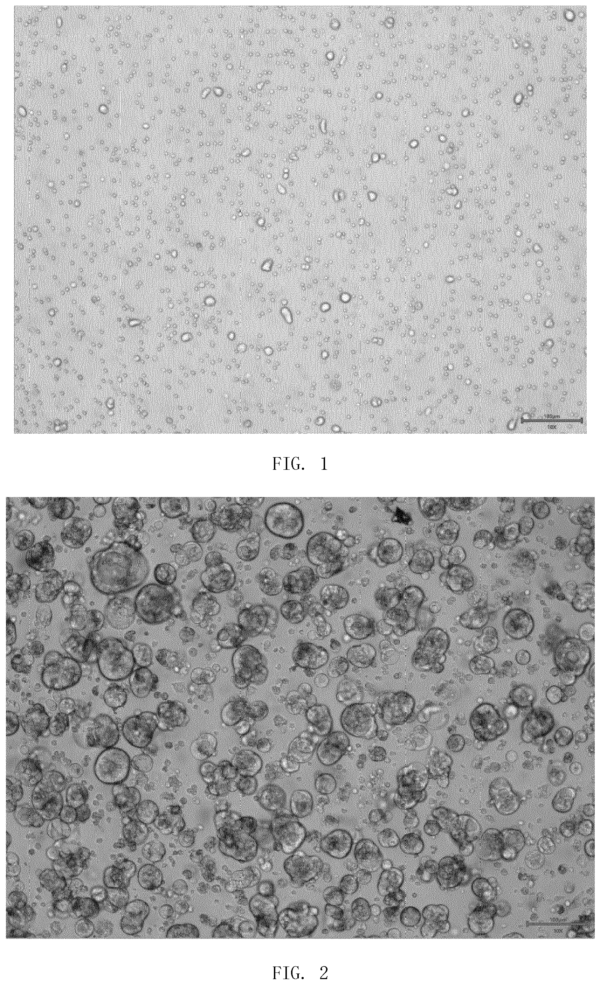

In addition to tumor cells, there were also a large number of various types of other cells, such as red blood cells, lymphocytes and fiber cells, mixed in the single cell suspension obtained by dissociation, as shown in FIG. 1. One of the advantages of the present method is that only the cancer cells could be amplified in the subsequent culture process, the proportion of other cells gradually decreases or even disappears, and primary tumor cells of gastric cancer with a high purity are finally obtained.

### Embodiment 5. Culturing primary cells of gastric cancer

1. A low-attachment-surface was used for suspension-culturing primary cells of gastric cancer, and the culture medium in use was the primary cell culture medium for solid tumor tissues of gastric cancer in Embodiment 1 (wherein the final concentration of human recombinant protein EGF was 50 ng/mL; the final concentration of human recombinant protein bFGF was 20 ng/mL; the final concentration of human recombinant protein HGF was 20 ng/mL; the final concentration of human recombinant protein FGF-10 was 20 ng/mL; the final concentration of human recombinant protein Wnt-3a was 250 ng/mL; the final concentration of human recombinant protein noggin was 100 ng/mL; the final concentration of SB202190 was 10 µM; the final concentration of A83-01 was 0.5 µM; the final concentration of N-acetyl-L-cysteine was 1 mM; the final concentration of Nicotinamide was 10 mM; the final concentration of cortisol was 25 ng/mL; the final concentration of Y-27632 was 10 µM); a 6-well plate was taken as an example, and cells were planked at the density of 10⁶ cells per well in a cell incubator under the condition of 37 °C, 5% CO₂.
2. The cell state was observed every day, and the culture medium was replaced every 3 days until the cells formed masses with a diameter of about 80µm.

As shown in FIG. 2, after 3-10 days of culture, cancer cells were amplified, forming cell masses with a diameter of 80µm, the total number of tumor cells exceeded 10⁷, and the number of other types of cells was significantly reduced or even disappeared. After a large number of sample tests, the success rate of the present method for culturing in vitro primary tumor cells of gastric cancer could reach 80%.

### Embodiment 6. Passaging primary cells of gastric cancer

1. The cell masses in a culture dish were collected and centrifugated at 800g at a room temperature for 10 minutes, and a supernatant was discarded.
2. The cell masses were washed with sterile PBS solution and centrifugated at 800g at a room temperature for 10 minutes, and a supernatant was discarded.
3. The cell masses were resuspended with cell digestion solution (see Embodiment 1) and digested at 37°C. The digestion of cell masses was observed under the microscope every 5 minutes until all the cell masses were digested into individual cells.
4. The dissociation reaction was terminated with a 10× volume of digestion termination solution (see Embodiment 1), and the cell suspension was collected.
5. The cell suspension was centrifugated at 800g at a room temperature for 10 minutes, and a supernatant was discarded.
6. The cell precipitation was resuspended with the primary cell culture medium for solid tumor tissues of gastric cancer, and the cells were counted.
7. A low-attachment-surface was used for culturing primary cells of gastric cancer, and the culture medium in use was the primary cell culture medium for solid tumor tissues of gastric cancer in Embodiment 1; a 6-well plate was taken as an example, and cells were planked at the density of 10⁶ cells per well in a cell incubator under the condition of 37°C, 5% CO₂.

### Embodiment 7. Cryopreserving primary cells of gastric cancer

The primary cells of gastric cancer suspension-cultured could be frozen after 2-3 passages and amplifications:
1. The cell masses in a culture dish were collected and centrifugated at 800g at a room temperature for 10 minutes, and a supernatant was discarded.
2. The cell masses were washed with sterile PBS solution and centrifugated at 800g at a room temperature for 10 minutes, and a supernatant was discarded.
3. The cell masses were resuspended with cell digestion solution (see Embodiment 1) and digested at 37°C. The digestion of cell masses was observed under the microscope every 15 minutes until all the cell masses were digested into individual cells.
4. The dissociation reaction was terminated with a 10× volume of digestion termination solution (see Embodiment 1), the cell suspension was collected, and the cells were counted.
5. The cell suspension was centrifugated at 800g at a room temperature for 10 minutes, and a supernatant was discarded.
6. The cell precipitation was resuspended at a density of 10⁶/mL with cell cryopreserving solution (see Embodiment 1), each 2 mL cryopreserving tube contained 1 mL of cell suspension, and the cell suspension was frozen overnight in a gradient cooling box and transferred into liquid nitrogen for long-term preservation.

### Embodiment 8. Resuscitating primary cells of gastric cancer

Primary cells of gastric cancer preserved in liquid nitrogen were resuscitated:
1. 37°C sterile water was prepared five minutes in advance.
2. The cryopreserving tubes were removed from liquid nitrogen, and the cells were quickly melted in 37°C sterile water.
3. The cell solution was centrifugated at 800g at a room temperature for 10 minutes, and a supernatant was discarded.
4. The cell precipitation was resuspended with the primary cell culture medium for solid tumor tissues of gastric cancer (see Embodiment 1), the primary cells of gastric cancer were cultured using a low-attachment-surface, the cells in each tube were resuscitated into a 3.5 cm culture dish, and the cells were cultured in a cell incubator under the condition of 37 °C, 5% CO₂.

### Embodiment 9. HE staining identification of primary cells of gastric cancer

Description of reagent consumables to be used in the following embodiment:
HE Staining Kit (Beijing Solarbio Science & Technology Co., Ltd., #G1120);
Cationic anticreep slide (ZSGB-BIO);
Xylene, methanol, acetone (Beijing Beihua Zhongtuo Technology Co., Ltd. , analytical pure);
Neutral resin adhesive (Beijing Yili Fine Chemicals Co., Ltd.).

1. Primary cell masses from solid tumor tissues of gastric cancer obtained by culturing with the primary cell culture medium for solid tumor tissues of gastric cancer in Embodiment 1 (wherein the final concentration of human recombinant protein EGF was 20 ng/mL; the final concentration of human recombinant protein bFGF was 20 ng/mL; the final concentration of human recombinant protein HGF was 20 ng/mL; the final concentration of human recombinant protein FGF-10 was 20 ng/mL; the final concentration of human recombinant protein Wnt-3a was 200 ng/mL; the final concentration of human recombinant protein Noggin was 100 ng/mL; the final concentration of SB202190 was 5 µM; the final concentration of A83-01 was 1 µM; the final concentration of N-acetyl-L-cysteine was 1 mM; the final concentration of Nicotinamide was 10 mM; the final concentration of cortisol was 25 ng/mL; the final concentration of Y-27632 was 10 µM) were collected by centrifugation at 800g, and immobilized with 4% paraformaldehyde. Cell mass precipitation was embedded in paraffin and sectioned with a thickness of 5 µm_{∘}
2. Paraffin sections were immersed in dimethylbenzene solution and incubated at a room temperature for 5 minutes for dewaxing, and after this process was repeated for 3 times, the sections were flushed with deionized water twice.
3. The sections were immersed in absolute ethanol and incubated at a room temperature for 10 minutes, and this process was repeated twice.
4. The sections were immersed in 95% ethanol and incubated at a room temperature for 10 minutes, and after this process was repeated twice, the sections were flushed with deionized water twice.
5. 100 µL of hematoxylin staining solution was added for staining for 1 min when the moisture on the slide was slightly dried.
6. The hematoxylin staining solution was absorbed, and the slide was washed for 3 times with tap water.
7. 100 µL of differentiation solution was added dropwise for differentiation for 1min.
8. The differentiation solution was absorbed, the slide was washed twice with tap water and washed with distilled water once.
9. The moisture on the slide surface was absorbed, and 200 µL of eosin stain was added dropwise for staining for 40 s.
10. The eosin stain was absorbed, and rinsing and dehydration were performed successively with 75%, 80%, 90% and 100% ethanol for 20 s, 20 s, 40 s and 40 s.
11. After the ethanol was dried in the air, 50 µL of dimethylbenzene was added dropwise for cell permeability.
12. After dimethylbenzene was completely dried, a drop of neutral resin adhesive was added, and the slide was sealed with a cover glass, observed under the microscope and photographed.

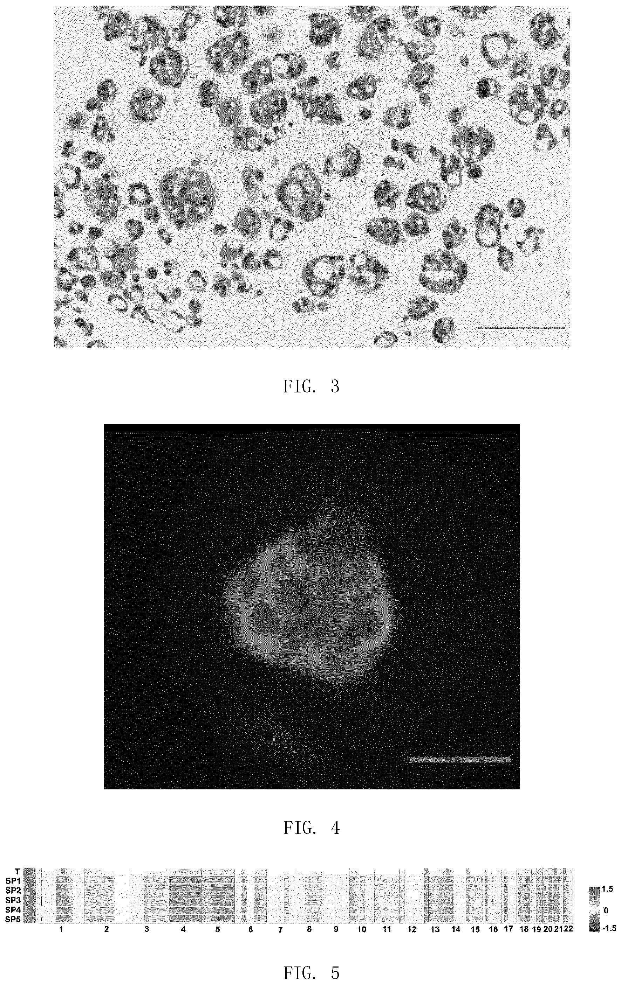

FIG. 3 shows an HE staining effect image of primary tumor cells of gastric cancer obtained by in vitro culture. It can be seen that these cells generally have the characteristics of cancer cells, such as high nuclear cytoplasmic ratio, hyperchromasia, chromatin condensation in nucleus, multinucleation and uneven cell size, and dozens to hundreds of tumor cells gather to form tumor cell masses with a certain three-dimensional structure.

### Embodiment 10. Immunofluorescence staining identification of primary cells of gastric cancer

Description of reagents to be used in the following embodiment:
Paraformaldehyde (Beijing Beihua Zhongtuo Technology Co., Ltd. analytical pure), paraformaldehyde powder was dissolved with ultrapure water to form 4% (4g/100mL paraformaldehyde solution;
Methanol and dimethyl sulfoxide (Beijing Beihua Zhongtuo Technology Co., Ltd., analytical pure);
Hydrogen peroxide (Beijing Beihua Zhongtuo Technology Co., Ltd., 35%);
Methanol, dimethyl sulfoxide and 35% hydrogen peroxide were mixed in the ratio of 4:4:1 (volume ratio) to form Dan's rinsing solution;
Bovine serum albumin (Sigma, #A1933), bovine serum albumin was dissolved with PBS solution to form 3% (3g/100mL) BSA solution;
Immunofluorescence primary antibody (Abcam, #ab17139);
Immunofluorescence secondary antibody (CST, #4408);
Hoechst staining solution (Beijing Solarbio Science & Technology Co., Ltd., #C0021);

The cell masses of gastric cancer obtained by culturing with the primary cell culture medium for solid tumor tissues of gastric cancer in Embodiment 1 (wherein the final concentration of human recombinant protein EGF was 50 ng/mL; the final concentration of human recombinant protein bFGF was 25 ng/mL; the final concentration of human recombinant protein HGF was 25 ng/mL; the final concentration of human recombinant protein FGF-10 was 25 ng/mL; the final concentration of human recombinant protein Wnt-3a was 300 ng/mL; the final concentration of human recombinant protein Noggin was 200 ng/mL; the final concentration of SB202190 was 10 µM; the final concentration of A83-01 was 0.5 µM; the final concentration of N-acetyl-L-cysteine was 1 mM; the final concentration of Nicotinamide was 10 mM; the final concentration of cortisol was 25 ng/mL; the final concentration of Y-27632 was 10 µM) were subject to immunofluorescence staining according to the following steps, the primary antibody was CK8+CK18, and epithelial-derived cells were characterized.
1. The cell masses in the culture dish were collected and washed once with PBS, the cell precipitation was resuspended with 4% paraformaldehyde, and immobilized at 4 °C overnight.
2. The cell solution was centrifugated at 800g, the supernatant was discarded, and the cell precipitation was resuspended with precooled methanol solution and placed on ice for 1 hour.
3. The cell solution was centrifugated at 800g, the supernatant was discarded, and the cell precipitation was resuspended with Dan's rinsing solution and placed at a room temperature for 2 hours.
4. The cell solution was centrifugated at 800g, the supernatant was discarded, and the cells were washed with 75%, 50% and 25% (volume percentage) methanol solution diluted with PBS for 10 minutes each time.
5. The cell solution was centrifugated at 800g, the supernatant was discarded, and the cell precipitation was suspended with 3% BSA solution and sealed at a room temperature for 2 hours.
6. The primary antibody was diluted with 3% BSA solution in a ratio of 1:500, the cell precipitation was resuspended with antibody diluent (3% BSA solution), and the primary antibody was placed at 4°C overnight.
7. The cell solution was centrifugated at 800g, the supernatant was discarded, and the cell precipitation was washed for 5 times with PBS solution, 20 minutes each time.
8. The secondary antibody was diluted with 3% BSA solution in a ratio of 1:2,000, the cell precipitation was resuspended with antibody diluent (3% BSA solution), and the secondary antibody was placed at a room temperature for 2 hours.
9. The cell solution was centrifugated at 800g, the supernatant was discarded, and the cell precipitation was washed for 5 times with PBS solution, 20 minutes each time.
10. 100×Hoechst staining solution was added in a volume ratio of 1/100 for staining at a room temperature for 20 minutes.
11. The cell precipitation was washed twice with PBS solution, 10 minutes each time. The staining of cell masses was observed using a laser confocal microscope.

FIG. 4 shows an immunofluorescence staining effect image of primary tumor cell masses of gastric cancer cultured in vitro. It can be seen that all the cells constituting the cell masses are CK8/CK18 positive and epithelial-derived, which confirms that the tumor cells obtained by the present method are those with a high purity. Immunofluorescence staining identification was performed on 20 primary cultures of gastric cancer samples, and the statistical results showed that the proportion of tumor cells in the primary cells of gastric cancer obtained in the present method reached 70%-93% (Table 27).

**Table 27 Immunofluorescence staining identification of primary cultures of gastric cancer samples**

| S/N | Sample No. | Positive Rate of CK8/CK18 |
|---|---|---|
| 1 | 20180305DHJ0296 | 74.9% |
| 2 | 20180305SYL9928 | 73.8% |
| 3 | 20180306TSR7222 | 71.5% |
| 4 | 20180306ZY5276 | 81.4% |
| 5 | 20180306ZXY5195 | 70.5% |
| 6 | 20180306WDH5845 | 83.2% |
| 7 | 20180306MX7450 | 87.3% |
| 8 | 20180307YXH1978 | 93.2% |
| 9 | 20180307CBP3705 | 86.0% |
| 10 | 20180308MDX7573 | 79.6% |
| 11 | 20180309ZBQ8443 | 89.8% |
| 12 | 20180309RDJ7864 | 93.0% |
| 13 | 20180309DYF0245 | 91.5% |
| 14 | 20180312CZZ0983 | 92.2% |
| 15 | 20180312SSL1528 | 92.5% |
| 16 | 20180314LMY0097 | 72.6% |
| 17 | 20180314WYC8956 | 76.8% |
| 18 | 20180315HZY9207 | 92.3% |
| 19 | 20180315ZSF3973 | 75.4% |
| 20 | 20180315YZM0341 | 70.4% |

### Embodiment 11. Primary cell cultures and primary tumor tissues of gastric cancer

The DNA extraction process mentioned in the following embodiment was performed with TIANGEN blood/tissue/cell genome extraction kit (DP304).

The library building process mentioned in the following embodiments was performed with NEB DNA sequencing and library building kit (E7645).

The high-throughput sequencing mentioned in the following embodiments refers to the Illumina HiSeq X-ten sequencing platform.
1. A solid tumor tissue sample of gastric cancer was obtained, 10 mg of solid tumor tissue sample of gastric cancer was firstly taken for DNA extraction, library building and whole-genome high-throughput sequencing (WGS), with a sequencing depth of 300× before in vitro culture operations were performed, and the remaining solid tumor tissue samples were used for culturing in vitro primary cells of gastric cancer.
2. Gastric cancer tissues were treated and cultured with the primary cell culture medium for solid tumor tissues of gastric cancer in Embodiment 1 (wherein the final concentration of human recombinant protein EGF was 50 ng/mL; the final concentration of human recombinant protein bFGF was 20 ng/mL; the final concentration of human recombinant protein HGF was 20 ng/mL; the final concentration of human recombinant protein FGF-10 was 20 ng/mL; the final concentration of human recombinant protein Wnt-3a was 250 ng/mL; the final concentration of human recombinant protein Noggin was 100 ng/mL; the final concentration of SB202190 was 10 µM; the final concentration of A83-01 was 1 µM; the final concentration of N-acetyl-L-cysteine was 1 mM; the final concentration of Nicotinamide was 8 mM; the final concentration of cortisol was 25 ng/mL; the final concentration of Y-27632 was 8 µM) for a period of time to form cell masses with a diameter of more than 100 µm, which were recorded as P0-generation cells, followed by P1, P2, ..., Pn successively according to the number of passages. 10⁶ cells were taken respectively from P1, P2, P3, P4 and P5-generation primary tumor cell cultures of gastric cancer for DNA extraction, library building and whole-genome high-throughput sequencing (WGS), with a sequencing depth of 300×.
3. Copy number variation (CNV) analysis was performed on the sequencing results of each group, and the copy number variations between tumor tissues of primary gastric cancer and all generations of primary cell cultures of gastric cancer were compared. As shown in FIG. 5, the copy number variation of all generations of primary cell cultures of gastric cancer (P1, P2, P3, P4 and P5) was highly consistent with that of the tumor tissue (Tumor) of primary gastric cancer, so the primary cells of gastric cancer obtained by the present method could represent the real condition of the patient's primary tumor.

### Embodiment 12. Comparing success rates of different primary cell culture media for culturing primary cells of gastric cancer

The operation methods and processes for culturing primary cells in all samples in this embodiment were identical (as mentioned above), and the only difference was the culture medium formula. Various tested primary cell culture media are shown in Table 28. Wherein Scheme D is the formula used in the present invention, and the details are shown in Table 9 (wherein the final concentration of human recombinant protein EGF was 50 ng/mL; the final concentration of human recombinant protein bFGF was 20 ng/mL; the final concentration of human recombinant protein HGF was 20 ng/mL; the final concentration of human recombinant protein FGF-10 was 20 ng/mL; the final concentration of human recombinant protein Wnt-3a was 250 ng/mL; the final concentration of human recombinant protein Noggin was 100 ng/mL; the final concentration of SB202190 was 10 µM; the final concentration of A83-01 was 1 µM; the final concentration of N-acetyl-L-cysteine was 1 mM; the final concentration of Nicotinamide was 8 mM; the final concentration of cortisol was 25 ng/mL; the final concentration of Y-27632 was 8 µM).

**Table 28 Primary cell culture medium formula for test (100 mL)**

| Culture Medium | Reagent | Brand Article N o. | Dosage | Final Concentration |
|---|---|---|---|---|
| Scheme A | mTeSR^{™}1 medium | Stemcell#0585 0 | 100 mL | |
| Scheme B | Alveolar Epithelial Cell Medium | ScienCell#3201 | 100 mL | |
| Scheme C | P/S | Gibco#1514012 2 | 1 mL | 1% |
| | HEPES | Gibco#1563008 0 | 1 mL | 10 mM |
| | 1000× human recombinant protein EGF | 1000× stock solution | 250 µL | 50 ng/mL |
| | 1000× human recombinant protein bFGF | 1000× stock solution | 100 µL | 20 ng/mL |
| | DMEM/F12 culture medium | Gibco# 1417016 1 | Replenish to 100 mL | |
| Scheme D | | | See Table 9 | |

After being prepared, the primary cell culture media were filtered and sterilized with a 0.22 µM syringe-driven filter (Millipore SLGP033RS). The primary cell culture media can be preserved at 4°C for two weeks.

20 samples were treated in each of the four primary cell culture medium schemes, the sample treatment and culture operations were performed according to the methods described in Embodiments 3, 4 and 5, and the success rates of those primary cell culture media for culturing primary cells in solid tumor tissues of gastric cancer were counted after 10 days of culture, as shown in Table 29:

**Table 29 Culture in different media**

| Primary cell culture medium scheme | Success rate |
|---|---|
| Scheme A | 0% (0/20) |
| Scheme B | 0% (0/20) |
| Scheme C | 10% (2/20) |
| Scheme D | 75% (15/20) |

It can be seen that different primary cell culture media have an extremely great impact on the success rate for culturing primary cells of gastric cancer, the primary cell culture medium for solid tumor tissues of gastric cancer used in the present invention (Table 9) can stimulate the proliferation of cancer cells in the solid tumor tissue sample of gastric cancer to the greatest extent and improve the success rate for culturing primary cells in solid tumor tissues of gastric cancer.

### Embodiment 13. Comparing success rates of different sample preservation solutions for culturing primary cells of gastric cancer

The operation methods and processes for culturing primary cells in all samples in this embodiment were identical (as mentioned above), and the only difference was the sample preservation solution formula. Various tested sample preservation solutions are shown in Table 30. Wherein Scheme E is the formula used in the present invention, and the details are shown in Table 1.

**Table 30 Sample preservation solution formula for test (100 mL)**

| Sample preservation solution scheme | Reagent | Brand Article No. | Dosage | Final Concentration |
|---|---|---|---|---|
| Scheme A | HBSS | Gibco# 14170161 | 100 mL | |
| | P/S | Gibco#15140122 | 1 mL | 1% |
| Scheme B | HBSS | Gibco# 14170161 | Replenish to 100 mL | |
| | P/S | Gibco#15140122 | 1 mL | 1% |
| Scheme C | DMEM culture medium | Gibco#11965-092 | Replenish to 100 mL | |
| | Antibiotic-antimycotic | Gibco#15240062 | 1 mL | 1% |
| Scheme D | Fetal bovine serum | Gibco#16000-044 | 10 mL | 10% |
| | DMEM culture medium | Gibco#11965-092 | Replenish to 100 mL | |
| Scheme E | | See Tab le 1 | | |

After being prepared, those sample preservation solutions in the above table were divided in 15 mL centrifuge tubes, and each tube was filled with 5 mL of sample preservation solutions. The sample preservation solution divided can be preserved at 4°C for 1 month.

20 samples were treated in each of the five sample preservation solution schemes, the samples were detached and temporarily preserved at 4 °C in the sample preservation solution; the sample treatment and culture operations were performed according to the methods described in Embodiments 3, 4 and 5 two hours after detachment, and the success rates of those sample preservation solutions for culturing primary cells in solid tumor tissues of gastric cancer were counted after 10 days of culture, as shown in Table 31:

**Table 31 Culture in different sample preservation solutions**

| Sample preservation solution scheme | Culture success rate | Bacteria/Fungus contamination rate |
|---|---|---|
| Scheme A | 45% (9/20) | 40% (15/20) |
| Scheme B | 55% (11/20) | 10% (2/20) |
| Scheme C | 60% (12/20) | 10% (2/20) |
| Scheme D | 70% (14/20) | 0% (0/20) |
| Scheme E | 75% (15/20) | 0% (0/20) |

It can be seen that different sample preservation solutions have a great impact on the culture success rate for culturing primary cells in solid tumor tissues of gastric cancer, the sample preservation solution used in the present invention (Table 1) can protect the activity of cancer cells in the solid tumor tissue sample of gastric cancer to the greatest extent and improve the success rate.

### Embodiment 14. Comparing success rates of different sample dissociation solutions for culturing primary cells of gastric cancer

The operation methods and processes of primary culture of all samples in this embodiment were identical (as mentioned above), and the only difference was the sample dissociation solution formula. Various tested sample dissociation solutions are shown in Table 32. Wherein Scheme D is the formula used in the present invention, and the details are shown in Table 3.

**Table 32 Sample dissociation solution formula for test (10 mL)**

| Sample dissociation solution | Reagent | Brand Article No. | Dosage | Final Concentration |
|---|---|---|---|---|
| Scheme A | Trypsin | Gibco# 25200056 | 10 mL | |
| Scheme B | 0.5M EDTA | Invitrogen#AM9261 | 10 µL | 5 mM |
| | DNAse | Thermo# EN0521 | 62.5 µL | 62.5 U/mL |
| | TrypLE^{™} Express | Gibco#12604013 | Replenish to 10 mL | |
| Scheme C | 20× collagenase IV | 20× stock solution | 1 mL | 200 U/mL |
| | DNAse | Thermo# EN0521 | 62.5 µL | 62.5 U/mL |
| | PBS | Gibco#21-040-CVR | Replenish to 10 mL | |
| Scheme D | See Table 3 | | | |

The sample dissociation solution is to be prepared just before use.

20 samples of solid tumor tissue masses of gastric cancer weighing more than 100 mg were selected and divided into four parts equally, and sample treatment and culture operations were performed with the above four sample dissociation solutions respectively according to the methods described in Embodiments 3, 4 and 5. The success rates of sample dissociation solutions for culturing primary cells in solid tumor tissues of gastric cancer were counted after 10 days of culture, as shown in Table 33 below:

**Table 33 Culture in different sample dissociation solutions**

| S/N | Sample No. | Scheme A | Scheme B | Scheme C | Scheme D |
|---|---|---|---|---|---|
| 1 | 20180316WDB9885 | Failed | Failed | Failed | Failed |
| 2 | 20180316WSX0073 | Failed | Successful | Successful | Successful |
| 3 | 20180319PSL9161 | Failed | Successful | Successful | Successful |
| 4 | 20180320YC8122 | Failed | Failed | Failed | Successful |
| 5 | 20180322XJH2568 | Failed | Failed | Successful | Successful |
| 6 | 20180323LAH6704 | Failed | Failed | Failed | Failed |
| 7 | 20180323WYL1663 | Failed | Failed | Failed | Successful |
| 8 | 20180327ZY3082 | Failed | Failed | Successful | Successful |
| 9 | 20180327WBS1138 | Failed | Failed | Failed | Successful |
| 10 | 20189327YXH5008 | Failed | Successful | Successful | Successful |
| 11 | 20180327WSQ2382 | Failed | Failed | Failed | Failed |
| 12 | 20180328WFM4371 | Failed | Failed | Failed | Failed |
| 13 | 20180328ZY2569 | Failed | Failed | Successful | Successful |
| 14 | 20180329MRL9449 | Failed | Failed | Failed | Successful |
| 15 | 20180329LJX5553 | Failed | Failed | Failed | Successful |
| 16 | 20180330WLJ3822 | Failed | Successful | Successful | Successful |
| 17 | 20180402LRY7640 | Failed | Failed | Failed | Successful |
| 18 | 20180402LY1226 | Failed | Failed | Failed | Successful |
| 19 | 20180403LRZ5217 | Failed | Failed | Successful | Successful |
| 20 | 20180404LGH0188 | Failed | Failed | Successful | Successful |
| | Summary | 0% (1/20) | 20% (4/20) | 45% (9/20) | 80% (16/20) |

It can be seen that different sample dissociation solutions have a very great impact on the success rate for culturing primary cells in solid tumor tissues of gastric cancer, the sample dissociation solution used in the present invention (Table 3) can isolate the cancer cells in solid tumor tissues of gastric cancer to the greatest extent and improve the success rate for culturing primary cells in solid tumor tissues of gastric cancer.

### Embodiment 15. Comparing success rates of different cell digestion solutions for passaging primary cells of gastric cancer

The operation methods and processes for passaging primary cells of all samples in this embodiment were identical (as mentioned above), and the only difference was the cell digestion solution formula. Various tested sample dissociation solutions are shown in Table 34. Wherein Scheme D is the formula used in the present invention, and the details are shown in Table 7.

**Table 34 Cell digestion solution formula for test (10 mL)**

| Cell digestion solution | Reagent | Brand Article No. | Dosage | Final Concentration |
|---|---|---|---|---|
| Scheme A | Trypsin | Gibco# 25200056 | 10 mL | |
| Scheme B | 0.5M EDTA | Invitrogen#AM9261 | 10 µL | 5 mM |
| | TrypLE^{™} Express | Gibco#12604013 | Replenish to 10 mL | |
| Scheme C | 10× collagenase I | 10× stock solution | 1 mL | 200 U/mL |
| | 10× collagenase II | 10× stock solution | 1 mL | 200 U/mL |
| | 20× collagenase IV | 20× stock solution | 0.5mL | 100 U/mL |
| | PBS | Gibco#21-040-CVR | Replenish to 10 mL | |
| Scheme D | See Table 7 | | | |

The cell digestion solution is to be prepared just before use.

20 successfully cultured samples of gastric cancer were selected, and the primary cells in solid tumor tissues of gastric cancer obtained by culture were continuously passaged with the above four cell digestion solutions respectively according to the method described in Embodiment 6. Passage (for no more than 10 times) was performed every time cancer cells amplified and formed cell masses with a diameter of 100 µm, and the maximum passage number was recorded. The statistical results are as shown in Table 35:

**Table 35 Culturing in different cell digestion solutions**

| S/N | Sample No. | Scheme A | Scheme B | Scheme C | Scheme D |
|---|---|---|---|---|---|
| 1 | 20180404DYZ6158 | 1 | 2 | 5 | 9 |
| 2 | 20180409XY0363 | 0 | 3 | 5 | 9 |
| 3 | 20180404LRJ6431 | 1 | 2 | 4 | 7 |
| 4 | 20180409LXS7372 | 1 | 3 | 7 | 10 |
| 5 | 20180410LCZ4904 | 0 | 1 | 4 | 7 |
| 6 | 20180410WXX8514 | 1 | 4 | 7 | 10 |
| 7 | 20180411LYC9049 | 1 | 4 | 6 | 9 |
| 8 | 20180411ZSJ2828 | 1 | 4 | 8 | 10 |
| 9 | 20180411GB9371 | 0 | 2 | 4 | 9 |
| 10 | 20180411ZFZ3083 | 1 | 3 | 6 | 10 |
| 11 | 20180413LJS1311 | 1 | 4 | 7 | 10 |
| 12 | 20180417XWY4460 | 1 | 3 | 8 | 10 |
| 13 | 20180419GFL5374 | 0 | 2 | 5 | 8 |
| 14 | 20180424ZLY5383 | 1 | 4 | 6 | 9 |
| 15 | 20180424YYC5496 | 0 | 1 | 5 | 7 |
| 16 | 20180425LSZ5296 | 1 | 4 | 6 | 9 |
| 17 | 20180426YCX7878 | 0 | 3 | 8 | 10 |
| 18 | 20180427LYQ2939 | 0 | 2 | 4 | 8 |
| 19 | 20180427GX6461 | 0 | 1 | 4 | 7 |
| 20 | 20180428CQF8086 | 1 | 4 | 7 | 10 |
| Summa ry of passage number | | 0-1 time | 2-4 times | 4-8 times | More than 7 times |

It can be seen that different cell digestion solutions have a very great impact on the success rate for passaging primary cells in solid tumor tissues of gastric cancer, the cell digestion solution used in the present invention (Table 7) can mildly dissociate the cancer cells in the cell masses to continuously passage the samples and maintain the activity of primary cells in solid tumor tissues of gastric cancer.

### Embodiment 16. Culturing primary tumor cells of gastric cancer with culture consumables of different materials

The operation methods and processes of primary culture of all samples in this embodiment were identical (as mentioned above), and the only difference was the cell culture consumable material (unmodified) (Table 36).

**Table 36 Effects of unmodified culture consumables of different materials on culturing primary tumor cells of gastric cancer**

| S/N | Sample No. | PS | PC | PMMA | COC | COP | LAS |
|---|---|---|---|---|---|---|---|
| 1 | 20180428JLS0901 | Successful | Successful | Successful | Successful | Failed | Successful |
| 2 | 20180428LSL4619 | Failed | Failed | Failed | Successful | Failed | Successful |
| 3 | 20180503WZP4591 | Successful | Successful | Successful | Successful | Successful | Successful |
| 4 | 20180504ZTL7916 | Successful | Failed | Successful | Successful | Successful | Successful |
| 5 | 20189597NAY7068 | Successful | Successful | Successful | Successful | Successful | Successful |
| 6 | 20180508CYM9714 | Successful | Failed | Successful | Failed | Failed | Successful |
| 7 | 20180508ZQY8343 | Failed | Failed | Failed | Failed | Failed | Failed |
| 8 | 20189510WSM9509 | Successful | Failed | Successful | Successful | Successful | Successful |
| 9 | 20180511ZYJ2145 | Successful | Successful | Successful | Successful | Successful | Successful |
| 10 | 20180511SYR5862 | Failed | Failed | Failed | Failed | Failed | Failed |
| Successful | | 7/10 | 4/10 | 7/10 | 7/10 | 5/10 | 8/10 |

Note: Polystyrene (abbreviated as PS), Polycarbonate (abbreviated as PC), poly-methyl methacrylate (abbreviated as PMMA), COC resin, Cyclo Olefin Polymer (abbreviated as COP), low-attachment-surface (abbreviated as LAS).

It can be seen from Table 36 that culture consumables of different materials have a certain impact on the success rates for culturing primary cells in solid tumor tissues of gastric cancer, wherein the success rate of low-attachment-surface (LAS) is the highest.

### Embodiment 17. Culturing primary tumor cells of gastric cancer with CYTOP-modified culture consumables

The operation methods and processes of primary culture of all samples in this embodiment were identical (as mentioned above), the CYTOP modification methods were identical, and the only difference was the culture consumable material (Table 37).

Wherein the CYTOP modification method was that: firstly, pure oxygen etching was performed on the cell culture vessel at a power of 20W power for 3 minutes. Then the surface of a culture dish or culture plate was covered with an appropriate amount (taking a 96-well plate as an example, with 20µL per well, an appropriate amount refers to complete coverage of the bottom of the culture dish) of 1*%* CYTOP solution, and the vessel could be used after the CYTOP solution was completely dried in the air.

**Table 37 Effects of CYTOP-modified culture consumables of different materials on culturing primary tumor cells of gastric cancer**

| S/ N | Sample No. | PS | PC | PMMA | COC | COP | LAS |
|---|---|---|---|---|---|---|---|
| 1 | 20180517GZX35 91 | Successf ul | Successf ul | Successf ul | Successf ul | Successf ul | Successf ul |
| 2 | 20180517GQL63 34 | Successf ul | Successf ul | Successf ul | Successf ul | Successf ul | Successf ul |
| 3 | 20180518ZXF24 20 | Successf ul | Successf ul | Successf ul | Successf ul | Successf ul | Successf ul |
| 4 | 20180518EJF585 1 | Failed | Failed | Failed | Failed | Failed | Failed |
| 5 | 20180518WBY1 609 | Successf ul | Successf ul | Successf ul | Successf ul | Successf ul | Successf ul |
| 6 | 20180521LXL35 51 | Successf ul | Successf ul | Successf ul | Successf ul | Successf ul | Successf ul |
| 7 | 20180522SJD627 5 | Successf ul | Successf ul | Successf ul | Successf ul | Successf ul | Successf ul |
| 8 | 20180523GSH20 64 | Successf ul | Successf ul | Successf ul | Successf ul | Successf ul | Successf ul |
| 9 | 20180524ZCS28 72 | Successf ul | Successf ul | Successf ul | Successf ul | Successf ul | Successf ul |
| 10 | 20180524XGY38 33 | Successf ul | Successf ul | Successf ul | Successf ul | Successf ul | Successf ul |
| Success rate | | 9/10 | 9/10 | 9/10 | 9/10 | 9/10 | 9/10 |

It can be seen from Table 37 that CYTOP modification can effectively improve the success rates of various materials.

### Example 18. Performing drug sensitivity test with primary tumor cells of gastric cancer

All the chemotherapeutic agents used in this embodiment, i.e. Irinotecan, 5-Fluorouracil and Oxaliplatin, are Selleck products.

The Celltiter-Glo cell viability test kit mentioned in this embodiment is a Promega product.

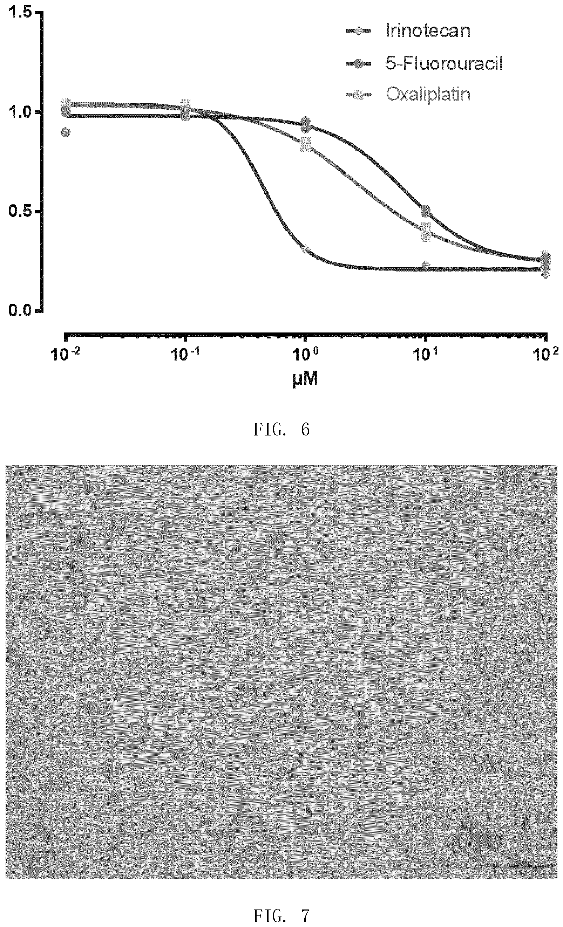

An in vitro drug sensitivity test was performed for the primary cells of gastric cancer cultured in the present invention: primary cells were inoculated at a density of 10⁵/well in a 96-well low-attachment cell culture plate of standard size, and 5 drug concentration gradients were set for each drug, n=3. After dosing, the cells were incubated under the condition of 37°C, 5% CO₂ for 7 days. Following the end of drug action, the cell viability in each well was detected by the Celltiter-Glo cell viability assay kit. The experimental results are as shown in FIG. 6. The results indicate that the primary cells of gastric cancer obtained by the present method can be used for in vitro drug sensitivity test.

### Embodiment 19. Preparing a reagent for culturing primary cells in solid tumor tissues of gallbladder cancer and cholangiocarcinoma

### 1. Sample preservation solution (100 mL)

For the specific formula (Table 1) and preparation method of sample preservation solution (100 mL), see step 1 in Embodiment 1.

### 2. Sample washing solution (100 mL)

For the specific formula (Table 2) and preparation method of sample washing solution (100 mL), see step 2 in Embodiment 1.

### 3. Sample dissociation solution (10 mL)

For the specific formula (Table 3) and preparation method of sample dissociation solution (10 mL), see step 3 in Embodiment 1.

### 4. Cell digestion solution (10 mL)

For the specific formula (Table 7) and preparation method of cell digestion solution (10 mL), see step 4 in Embodiment 1.

### 5. Digestion termination solution (100 mL)

For the specific formula (Table 8) and preparation method of digestion termination solution (100 mL), see step 5 in Embodiment 1.

### 6. Primary cell culture medium for solid tumor tissues of gallbladder cancer and cholangiocarcinoma (100 mL)

For the specific formula (Table 9) and preparation method of primary cell culture medium for solid tumor tissues of gallbladder cancer and cholangiocarcinoma (100 mL), see step 6 in Embodiment 1.

### 7. Cell cryopreserving solution

For the specific formula (Table 24) and preparation method of cell cryopreserving solution, see step 7 in Embodiment 1.

### 8. 1% CYTOP solution

For the specific formula (Table 26) and preparation method of 1*%* CYTOP solution, see step 8 in Embodiment 1.

### Embodiment 20. Obtaining postoperative specimens/biopsy puncture specimens of gallbladder cancer and cholangiocarcinoma

1. The inventor cooperated with national triple A, first-class hospitals in China, and the cooperation passed formal medical ethical review.
2. The attending physician selected the enrolled patients according to the clinical indications specified in the medical guidelines, and selected suitable samples for in vitro culture according to the intraoperative clinical indications. The selection criteria of samples during surgery were as follows: primary gallbladder cancer or cholangiocarcinoma, with pathological staging of stage II, III or IV, metastatic lesions of gallbladder cancer and cholangiocarcinoma of various pathological types, and samples with a surgical specimen weighing more than 20 mg. The selection criteria of biopsy puncture samples were as follows: primary gallbladder cancer or cholangiocarcinoma, with pathological staging of stage II, III or IV, metastatic lesions of gallbladder cancer and cholangiocarcinoma of various pathological types, and samples with more than 4 puncture specimens.
3. The attending physician provided the patient's basic clinical information, such as gender, age, medical history, family history, smoking history, pathological stages and types and clinical diagnosis. The information related to patient privacy, such as patient's name and ID number, was concealed and replaced by a unified experimental number, and the naming principle of the experimental number was the 8-digit date of sample collection + last four digits of the patient's admission number. For example, for a sample provided on January 1, 2018, when the patient's admission number was T001512765, then the sample experiment number was 201801012765.
4. During the operation, a surgeon collected a fresh surgical specimen in a sterile environment of an operating room and placed it in a sample preservation solution prepared in advance (see Embodiment 19). After being detached, the sample was temporarily stored on ice and transported to a laboratory within two hours for the next operation.
5. A puncture physician collected a fresh puncture specimen in a sterile environment of a puncture operating room and placed it in a sample preservation solution prepared in advance (see Embodiment 19). After being detached, the sample was temporarily stored on ice and transported to a laboratory within two hours for the next operation.

### Embodiment 21. Performing predissociation treatment for samples of gallbladder cancer and cholangiocarcinoma

The following steps were operated on ice, and the whole operation process was completed within 10 minutes.

All the surgical apparatuses used in the following actions were used only after high-temperature and high-pressure sterilization and oven drying in advance.
1. The sample was weighed.
2. The sample surface was washed with 75*%* (volume percentage) ethanol for 10 to 30 seconds.
3. The sample was washed with sample washing solution for 10 times and with sterile PBS solution for 5 times.
4. Fatty tissues, connective tissues and necrotic tissues in the sample were carefully peeled off with ophthalmic scissors, ophthalmic forceps, scalpel and other apparatuses.

### Embodiment 22. Dissociating tissue samples of gallbladder cancer and cholangiocarcinoma

All the surgical apparatuses used in the following actions were used only after high-temperature and high-pressure sterilization and oven drying in advance.
1. The tissues were cut into small pieces of about 1mm³ with ophthalmic scissors.
2. The tissue sample cut into pieces was treated with sample dissociation solution preheated to 37°C in advance according to the dosage of 0.1 mL of sample dissociation solution (see Embodiment 19) per mg of tissue, and the sample was dissociated at 37°C for 15 minutes to 3 hours. Observing the dissociation of the sample under a microscope every 15 minutes until a large number of individual cells are observed.
3. The dissociation reaction was terminated with a 10 × volume of digestion termination solution (see Embodiment 19), and the cell suspension was collected.
4. The cell suspension was filtered with a 40 µm sterile cell strainer to remove tissue fragments and adherent cells.
5. The cell suspension was centrifuged at 800g at a room temperature for 10 minutes, and a supernatant was discarded.
6. The cells were resuspended with 5mL of sterile PBS and centrifugated at 800g at a room temperature for 10 minutes, and a supernatant was discarded.
7. The cell precipitation was resuspended with the primary cell culture medium for solid tumor tissues of gallbladder cancer and cholangiocarcinoma (see Embodiment 19), the cell state was observed under a microscope, and the cells were counted.

In addition to tumor cells, there were also a large number of various types of other cells, such as red blood cells, lymphocytes and fiber cells, mixed in the single cell suspension obtained by dissociation, as shown in FIG. 7. One of the advantages of the present method is that only the cancer cells could be amplified in the subsequent culture process, the proportion of other cells gradually decreases or even disappears, and primary tumor cells of gallbladder cancer and cholangiocarcinoma with a high purity are finally obtained.

### Embodiment 23. Culturing primary cells of gallbladder cancer and cholangiocarcinoma

1. A low-attachment-surface was used for suspension-culturing primary cells in solid tumor tissues of gallbladder cancer and cholangiocarcinoma, and the culture medium in use was the primary cell culture medium for gallbladder cancer and cholangiocarcinoma in Embodiment 19 (wherein the final concentration of human recombinant protein EGF was 50 ng/mL; the final concentration of human recombinant protein bFGF was 20 ng/mL; the final concentration of human recombinant protein HGF was 20 ng/mL; the final concentration of human recombinant protein FGF-10 was 20 ng/mL; the final concentration of human recombinant protein Wnt-3a was 250 ng/mL; the final concentration of human recombinant protein noggin was 100 ng/mL; the final concentration of SB202190 was 10 µM; the final concentration of A83-01 was 0.5 µM; the final concentration of N-acetyl-L-cysteine was 1 mM; the final concentration of Nicotinamide was 10 mM; the final concentration of cortisol was 25 ng/mL; the final concentration of Y-27632 was 10 µM); a 6-well plate was taken as an example, and cells were planked at the density of 10⁶ cells per well in a cell incubator under the condition of 37°C, 5*%* CO₂.
2. The cell state was observed every day, and the culture medium was replaced every 3 days until the cells formed masses with a diameter of about 80µm.

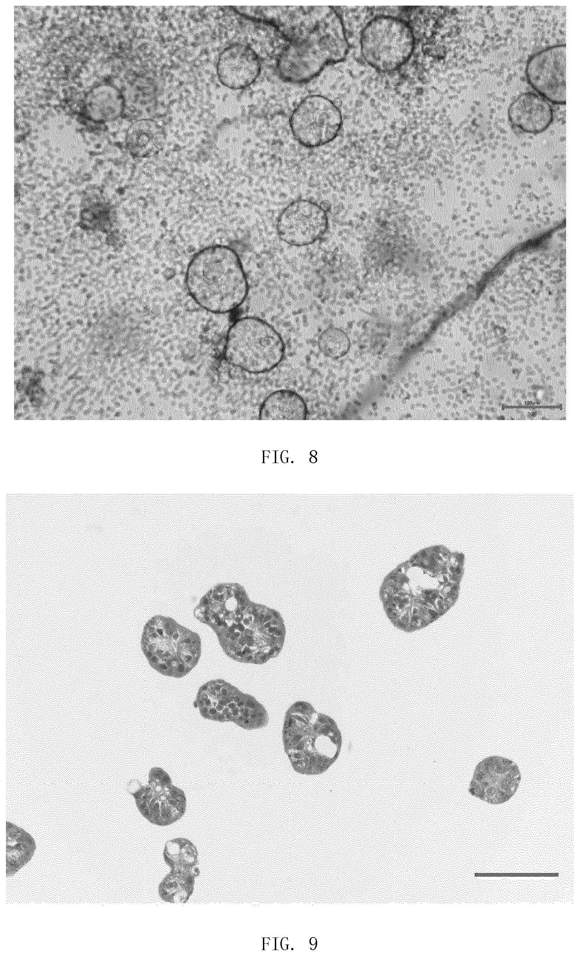

As shown in FIG. 8, after 3-10 days of culture, cancer cells were amplified, forming cell masses with a diameter of 80µm, the total number of tumor cells exceeded 10⁷, and the number of other types of cells was significantly reduced or even disappeared. After a large number of sample tests, the success rate of the present method for culturing in vitro primary tumor cells of gallbladder cancer and cholangiocarcinoma could reach 80*%*.

### Embodiment 24. Passaging primary cells in solid tumor tissues of gallbladder cancer and cholangiocarcinoma

1. The cell masses in a culture dish were collected and centrifugated at 800g at a room temperature for 10 minutes, and a supernatant was discarded.
2. The cell masses were washed with sterile PBS solution and centrifugated at 800g at a room temperature for 10 minutes, and a supernatant was discarded.
3. The cell masses were resuspended with cell digestion solution (see Embodiment 19) and digested at 37°C. The digestion of cell masses was observed under the microscope every 5 minutes until all the cell masses were digested into individual cells.
4. The dissociation reaction was terminated with a 10 × volume of digestion termination solution (see Embodiment 19), and the cell suspension was collected.
5. The cell suspension was centrifuged at 800g at a room temperature for 10 minutes, and a supernatant was discarded.
6. The cell precipitation was resuspended with the primary cell culture medium of gallbladder cancer and cholangiocarcinoma, and the cells were counted.
7. A low-attachment-surface was used for culturing primary cells of gallbladder cancer and cholangiocarcinoma, and the culture medium in use was the primary cell culture medium for solid tumor tissues of gallbladder cancer and cholangiocarcinoma in Embodiment 19; a 6-well plate was taken as an example, and cells were planked at the density of 10⁶ cells per well in a cell incubator under the condition of 37°C, 5*%* CO₂.

### Embodiment 25. Cryopreserving primary cells in solid tumor tissues of gallbladder cancer and cholangiocarcinoma

The primary cells in solid tumor tissues of gallbladder cancer and cholangiocarcinoma suspension-cultured could be frozen after 2-3 passages and amplifications:
1. The cell masses in a culture dish were collected and centrifugated at 800g at a room temperature for 10 minutes, and a supernatant was discarded.
2. The cell masses were washed with sterile PBS solution and centrifugated at 800g at a room temperature for 10 minutes, and a supernatant was discarded.
3. The cell masses were resuspended with cell digestion solution (see Embodiment 19) and digested at 37°C. The digestion of cell masses was observed under the microscope every 15 minutes until all the cell masses were digested into individual cells.
4. The dissociation reaction was terminated with a 10 × volume of digestion termination solution (see Embodiment 19), the cell suspension was collected, and the cells were counted.
5. The cell suspension was centrifuged at 800g at a room temperature for 10 minutes, and a supernatant was discarded.
6. The cell precipitation was resuspended at a density of 10⁶/mL with cell cryopreserving solution (see Embodiment 19), each 2 mL cryopreserving tube contained 1 mL of cell suspension, and the cell suspension was frozen overnight in a gradient cooling box and transferred into liquid nitrogen for long-term preservation.

### Embodiment 26. Resuscitating primary cells in solid tumor tissues of gallbladder cancer and cholangiocarcinoma

Primary cells in solid tumor tissues of gallbladder cancer and cholangiocarcinoma preserved in liquid nitrogen were resuscitated:
1. 37°C sterile water was prepared five minutes in advance.
2. The cryopreserving tubes were removed from liquid nitrogen, and the cells were quickly melted in 37°C sterile water.
3. The cell solution was centrifuged at 800g at a room temperature for 10 minutes, and a supernatant was discarded.
4. The cell precipitation was resuspended with the primary cell culture medium for solid tumor tissues of gallbladder cancer and cholangiocarcinoma (see Embodiment 19), the primary cells of gallbladder cancer and cholangiocarcinoma were cultured using a low-attachment-surface, the cells in each tube were resuscitated into a 3.5 cm culture dish, and the cells were cultured in a cell incubator under the condition of 37°C, 5*%* CO₂.

### Embodiment 27. HE staining identification of primary cells in solid tumor tissues of gallbladder cancer and cholangiocarcinoma

Description of reagent consumables to be used in the following embodiment:
HE Staining Kit (Beijing Solarbio Science & Technology Co., Ltd., #G1120);
Cationic anticreep slide (ZSGB-BIO);
Xylene, methanol, acetone (Beijing Beihua Zhongtuo Technology Co., Ltd., analytical pure);
Neutral resin adhesive (Beijing Yili Fine Chemicals Co., Ltd.).

1. Primary cell masses from solid tumor tissues of gallbladder cancer and cholangiocarcinoma obtained by culturing with the primary cell culture medium for solid tumor tissues of gallbladder cancer and cholangiocarcinoma in Embodiment 19 (wherein the final concentration of human recombinant protein EGF was 20 ng/mL; the final concentration of human recombinant protein bFGF was 20 ng/mL; the final concentration of human recombinant protein HGF was 20 ng/mL; the final concentration of human recombinant protein FGF-10 was 20 ng/mL; the final concentration of human recombinant protein Wnt-3a was 200 ng/mL; the final concentration of human recombinant protein Noggin was 100 ng/mL; the final concentration of SB202190 was 5 µM; the final concentration of A83-01 was 1 µM; the final concentration of N-acetyl-L-cysteine was 1 mM; the final concentration of Nicotinamide was 10 mM; the final concentration of cortisol was 25 ng/mL; the final concentration of Y-27632 was 10 µM) were collected by centrifugation at 800g, and immobilized with 4*%* paraformaldehyde. Cell mass precipitation was imbedded in paraffin and sectioned with a thickness of 5 µm_{∘}
2. Paraffin sections were immersed in dimethylbenzene solution and incubated at a room temperature for 5 minutes for dewaxing, and after this process was repeated for 3 times, the sections were flushed with deionized water twice.
3. The sections were immersed in absolute ethanol and incubated at a room temperature for 10 minutes, and this process was repeated twice.
4. The sections were immersed in 95*%* ethanol and incubated at a room temperature for 10 minutes, and after this process was repeated twice, the sections were flushed with deionized water twice.
5. 100 µL of hematoxylin staining solution was added for staining for 1 min when the moisture on the slide was slightly dried.
6. The hematoxylin staining solution was absorbed, and the slide was washed for 3 times with tap water.
7. 100 µL of differentiation solution was added dropwise for differentiation for 1min.
8. The differentiation solution was absorbed, the slide was washed twice with tap water and washed with distilled water once.
9. The moisture on the slide surface was absorbed, and 200 µL of eosin stain was added dropwise for staining for 40 s.
10. The eosin stain was absorbed, and rinsing and dehydration were performed successively with 75*%*, 80*%*, 90*%* and 100*%* ethanol for 20 s, 20 s, 40 s and 40 s.
11. After the ethanol was dried in the air, 50 µL of dimethylbenzene was added dropwise for cell permeability.
12. After dimethylbenzene was completely dried, a drop of neutral resin adhesive was added, and the slide was sealed with a cover glass, observed under the microscope and photographed.

FIG. 9 shows an HE staining effect image of primary tumor cells of cholangiocarcinoma obtained by in vitro culture. It can be seen that these cells generally have the characteristics of cancer cells, such as high nuclear cytoplasmic ratio, hyperchromasia, chromatin condensation in nucleus, multinucleation and uneven cell size, and dozens to hundreds of tumor cells gather to form tumor cell masses with a certain three-dimensional structure.

### Embodiment 28. Immunohistochemical staining identification of primary cells in solid tumor tissues of gallbladder cancer and cholangiocarcinoma

Description of reagents to be used in the following embodiment:
Paraformaldehyde (Beijing Beihua Zhongtuo Technology Co., Ltd., analytical pure), paraformaldehyde powder was dissolved with ultrapure water to form 4*%* (4g/100mL paraformaldehyde solution;
Hydrogen peroxide (Beijing Beihua Zhongtuo Technology Co., Ltd., 35%);
Normal goat serum for blocking (Solarbio, SL038);
Immunofluorescence primary antibody (Abcam, ab215838);
Immunofluorescence secondary antibody (Abcam, ab205719);
EDTA repair solution (Abcam, ab93684);
DAB chromogen (SignalStain^{®} DAB Substrate Kit, 8059S)

The cell masses of gallbladder cancer and cholangiocarcinoma obtained by culturing with the primary cell culture medium for solid tumor tissues of gallbladder cancer and cholangiocarcinoma in Embodiment 19 (wherein the final concentration of human recombinant protein EGF was 50 ng/mL; the final concentration of human recombinant protein bFGF was 25 ng/mL; the final concentration of human recombinant protein HGF was 25 ng/mL; the final concentration of human recombinant protein FGF-10 was 25 ng/mL; the final concentration of human recombinant protein Wnt-3a was 300 ng/mL; the final concentration of human recombinant protein Noggin was 200 ng/mL; the final concentration of SB202190 was 10 µM; the final concentration of A83-01 was 0.5 µM; the final concentration of N-acetyl-L-cysteine was 1 mM; the final concentration of Nicotinamide was 10 mM; the final concentration of cortisol was 25 ng/mL; the final concentration of Y-27632 was 10 µM) were collected for paraffin section, and epithelial-derived cells were characterized with pan-CK antibody according to the following steps.
1. The section was successively immersed in dimethylbenzene I for 10 min and dimethylbenzene II (10 min).
2. The section was immersed in absolute ethyl alcohol I (5 min) - absolute ethyl alcohol II (5 min) - 95*%* alcohol (5 min) - 80*%* alcohol (5 min) - 70*%* alcohol (5 min), and then flushed with deionized water twice, 2 min each time.
3. The tissue section was put into a repair box, then an appropriate amount of diluted EDTA repair solution (pH 9.0) was added, with the level higher than the tissue section.
4. The tissue section was repaired with microwave mid-range for 10 min (time started when the liquid boiled), and a dry tissue should be avoided in this process.
5. The repair box was taken out of the microwave oven and cooled down in air, and after the repair solution dropped to the room temperature, the glass slide was taken out and flushed with PBS (pH 7.4) for 3 times, 3 min each time (do not flush toward the tissue in the flushing process to avoid breaking the tissue).
6. Prepared 3*%* hydrogen peroxide (30*%* hydrogen peroxide diluted with deionized water) was added to the section tissue dropwise to block endogenous peroxidase, incubated at a room temperature for 15 min, and flushed with PBS for 3 times, 3 min each time.
7. PBS was dried out with absorbent paper, 10*%* goat serum (consistent with or similar to the species source of the secondary antibody) was added dropwise on the glass slide, and the glass slide was blocked at 37°C for 60 min.
8. The liquid around the tissue was wiped dry with absorbent paper, a circle was drawn around the tissue by mark pen, then the diluted primary antibody was added dropwise, and the slide was incubated in a wet box at 4°C overnight.
9. The section was flushed with PBS for 3 times, 3 min each time, a horseradish peroxidase labeled secondary antibody was added dropwise after the section was wiped dry with absorbent paper, and the section was incubated at a room temperature for 60 min.
10. The section was flushed with PBS for 3 times, 3 min each time, the section was wiped dry with absorbent paper after PBS liquid was thrown backward, freshly-prepared DAB chromogen was added to each section dropwise, the sections were observed under a microscope, and the section was flushed with tap water after a positive signal was shown to stop the color development.
11. Hematoxylin counterstaining was performed for 1 min, and the section was differentiated with acid ethanol differentiation solution after being washed with water, and flushed with tap water until cell nucleus turned blue.
12. After being put into water and flushed, the section was successively put into: 70*%* alcohol - 80*%* alcohol - 90*%* alcohol - 95*%* alcohol - absolute ethyl alcohol I - absolute ethyl alcohol II - dimethylbenzene I - dimethylbenzene II for dehydration and clearing, 2 min in each reagent, and finally the section was dried in a fume cupboard.
13. The section was sealed with neutral balsam and covered with cover glass. The section was placed in a fume cupboard and dried.
14. The dried section was observed or photographed under a microscope.

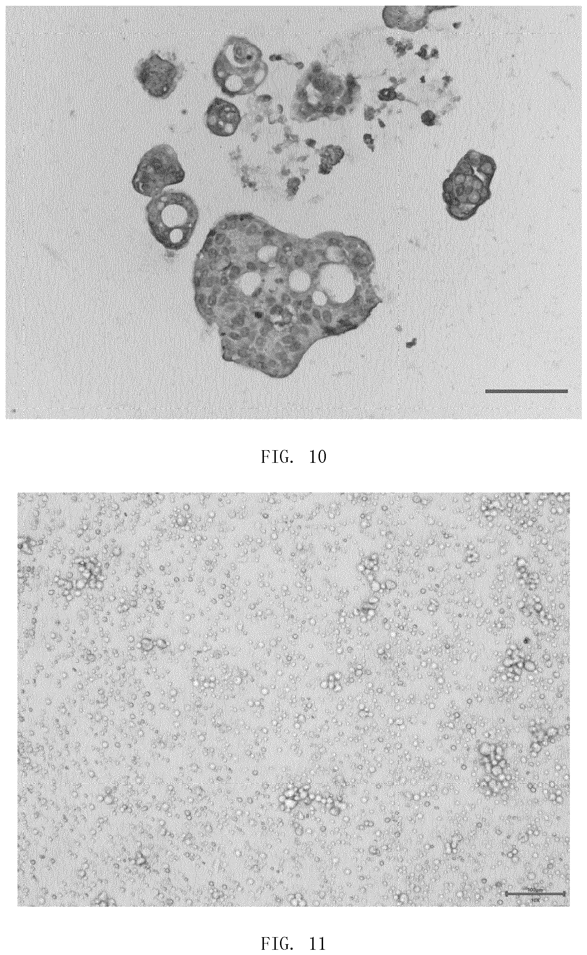

FIG. 10 shows an immunofluorescence staining effect image of primary tumor cell masses of cholangiocarcinoma cultured in vitro. It can be seen that all the cells constituting the cell masses are pan-CK positive and epithelial-derived, which confirms that the tumor cells obtained by the present method are those with a high purity. Immunofluorescence staining identification was performed on 5 primary cultures of gallbladder cancer and cholangiocarcinoma samples, and the statistical results showed that the proportion of tumor cells in the primary cells of gallbladder cancer and cholangiocarcinoma obtained in the present method reached 84*%*-95*%* (Table 38).

### Embodiment 38 Immunohistochemical staining identification of primary cultures of gallbladder cancer and cholangiocarcinoma

| S/N | Sample No. | Type | Positive Rate of Pan-CK |
|---|---|---|---|
| 1 | 20181127LXG2000 | cholangiocarcinoma | 95*%* |
| 2 | 20190225LZL1104 | Cholangiocarcinoma | 84*%* |
| 3 | 20190225SHM1105 | Cholangiocarcinoma | 89*%* |
| 4 | 20190514JJK1147 | Gallbladder cancer | 91*%* |
| 5 | 20190712HHL0332 | Cholangiocarcinoma | 87*%* |

### Embodiment 29. Culturing primary tumor cells in solid tumor tissues of gallbladder cancer and cholangiocarcinoma with CYTOP-modified culture consumables

The operation methods and processes of primary culture of all samples in this embodiment were identical (as mentioned above), the CYTOP modification methods were identical, and the only difference was the cell culture consumable material (Table 39).

Wherein the CYTOP modification method was that: firstly, pure oxygen etching was performed on the cell culture vessel at a power of 20W for 3 minutes. Then the surface of a culture dish or culture plate was covered with an appropriate amount (taking a 96-well plate as an example, with 20µL per well, an appropriate amount refers to complete coverage of the bottom of the culture dish) of 1*%* CYTOP solution, and the vessel could be used after the CYTOP solution was completely dried in the air.

**Table 39. Effects of CYTOP-modified consumables on culturing primary tumor cells in solid tumor tissues of gallbladder cancer and cholangiocarcinoma**

| S/N | Sample No. | Type | PS | PS CYTOP modification |
|---|---|---|---|---|
| 1 | 20190321CZ1114 | Cholangiocarcinoma | Failed | Successful |
| 2 | 20190823WJW0401 | Cholangiocarcinoma | Failed | Successful |
| 3 | 20191009LCH0776 | Gallbladder cancer | Failed | Successful |
| 4 | 20190923HSE0482 | Gallbladder cancer | Failed | Failed |
| 10 | 20190924WMY0335 | Cholangiocarcinoma | Failed | Successful |
| | Success rate | | 0/5 | 4/5 |

Note: Polystyrene (abbreviated as PS).

It can be seen from Table 39 that CYTOP modification can improve the success rate for culturing samples greatly.

### Embodiment 30. Preparing a reagent for culturing primary cells in the bile samples of gallbladder cancer and cholangiocarcinoma

### 1. Cell isolation buffer (100 mL)

The specific formula of cell isolation buffer (100 mL) is shown in Table 40:

**Table 40 Cell isolation buffer (100 mL)**

| Reagent | Brand Article No. | Dosage | Final Concentration |
|---|---|---|---|
| P/S | Gibco#15140122 | 1 mL | 1% |
| Heparin sodium solution | 1000× stock solution | 1 mL | 10 IU/mL |
| PBS | Gibco#21-040-CVR | Replenish to 100 mL | |

The prepared cell isolation buffer can be preserved at 4°C for 1 month.

In Table 40, the preparation of heparin sodium solution is shown in Table 41.

**Table 41 1000× heparin sodium (1 mL)**

| Reagent | Brand Article No. | Dosage | Final Concentration |
|---|---|---|---|
| Heparin sodium | Solarbio#H8270 | 10 kIU | 10 IU/L |
| Ultrapure water | | | Replenish to 1 mL |

1000× heparin sodium solution is to be prepared just before use.

### 2. Cell digestion solution (10 mL)

For the specific formula (Table 7) and preparation method of cell digestion solution (10 mL), see step 4 in Embodiment 1.

### 3. Digestion termination solution (100 mL)

For the specific formula (Table 8) and preparation method of digestion termination solution (100 mL), see step 5 in Embodiment 1.

### 4. Primary cell culture medium for bile sample of gallbladder cancer and cholangiocarcinoma (100 mL)

For the specific formula (Table 9) and preparation method of primary cell culture medium for the bile sample of gallbladder cancer and cholangiocarcinoma (100 mL), see step 6 in Embodiment 1.

### 5. Cell cryopreserving solution

For the specific formula (Table 24) and preparation method of cell cryopreserving solution, see step 7 in Embodiment 1.

### Embodiment 31. Obtaining bile samples of gallbladder cancer and cholangiocarcinoma

1. The inventor cooperated with national triple A, first-class hospitals in China, and the cooperation passed formal medical ethical review.
2. The attending physician selected the enrolled patients according to the clinical indications specified in the medical guidelines, and selected suitable samples for in vitro culture according to the intraoperative clinical indications. The selection criteria of samples during surgery were as follows: sample of primary gallbladder cancer or cholangiocarcinoma, with pathological staging of stage II, III or IV, and bile sample volume of more than 20 mL.
3. The attending physician provided the patient's basic clinical information, such as gender, age, medical history, family history, smoking history, pathological stages and types and clinical diagnosis. The information related to patient privacy, such as patient's name and ID number, was concealed and replaced by a unified experimental number, and the naming principle of the experimental number was the 8-digit date of sample collection + last four digits of the patient's admission number. For example, for a sample provided on January 1, 2018, when the patient's admission number was T001512765, then the sample experiment number was 201801012765.
4. The doctor in charge of the patient collected more than 10 mL of fresh bile sample with sterile apparatus. The sample was temporarily stored on ice and transported to a laboratory for the next operation within 48 hours.

### Embodiment 32. Pretreating bile samples of gallbladder cancer and cholangiocarcinoma

The following steps were operated on ice, and the whole operation process was completed within 10 minutes.
1. The bile sample was kept still on ice for about 30 minutes, so that blood clots and large insoluble solids in the sample were settled to the bottom of the sample tube;
2. The supernatant was carefully transferred into a 50 mL sterile centrifuge tube, and a 1× volume of precooled PBS was added and blended;
3. The cell suspension was centrifuged at 2,000g at 4 °C for 5 minutes, and the supernatant was discarded;
4. The cell precipitation was resuspended with cell separation buffer (see Embodiment 30) and centrifuged at 2,000g at 4°C for 5 minutes, and the supernatant was discarded;
5. The cell precipitation was resuspended with cell separation buffer (see Embodiment 30), and the cell concentration was adjusted to 10⁷/mL.

### Embodiment 33. Density gradient centrifugation of bile samples of gallbladder cancer and cholangiocarcinoma

1. Ficoll cell separation solution (MP#50494) of the same volume as the cell suspension was taken by a 50 mL sterile centrifuge tube.
2. The cell suspension was carefully added to the upper layer of the cell separation solution, so that a clear interface was formed between them.
3. The cell suspension was horizontally centrifuged at 2,000 at a room temperature for 20 minutes.
4. A white membrane in an intermediate layer was sucked into a new tube.
5. The cell precipitation was resuspended with 20 mL sterile PBS and centrifuged at 1,500g at a room temperature for 10 minutes, and the supernatant was discarded.
6. The cell precipitation was resuspended with the primary cell culture medium for the bile samples of gallbladder cancer and cholangiocarcinoma (see Embodiment 30), the cell state was observed under a microscope, and the cells were counted.

In addition to tumor cells, there were also a large number of various types of other cells, such as red blood cells, lymphocytes and fiber cells, mixed in the single cell suspension obtained by separation, as shown in FIG. 11. One of the advantages of the present method is that only the cancer cells could be amplified in the subsequent culture process, the proportion of other cells gradually decreases or even disappears, and primary tumor cells of gallbladder cancer and cholangiocarcinoma with a high purity are finally obtained.

### Embodiment 34. Culturing primary cells in the bile samples of gallbladder cancer and cholangiocarcinoma

1. A low-attachment-surface was used for suspension-culturing primary cells **in** the bile sample of gallbladder cancer and cholangiocarcinoma, and the culture medium in use was the primary cell culture medium for the bile samples of gallbladder cancer and cholangiocarcinoma in Embodiment 30 (wherein the final concentration of human recombinant protein EGF was 50 ng/mL; the final concentration of human recombinant protein bFGF was 20 ng/mL; the final concentration of human recombinant protein HGF was 20 ng/mL; the final concentration of human recombinant protein FGF-10 was 20 ng/mL; the final concentration of human recombinant protein Wnt-3a was 250 ng/mL; the final concentration of human recombinant protein noggin was 100 ng/mL; the final concentration of SB202190 was 10 µM; the final concentration of A83-01 was 0.5 µM; the final concentration of N-acetyl-L-cysteine was 1 mM; the final concentration of Nicotinamide was 10 mM; the final concentration of cortisol was 25 ng/mL; the final concentration of Y-27632 was 10 µM); a 6-well plate was taken as an example, and cells were planked at the density of 10⁶ cells per well in a cell incubator under the condition of 37 °C, 5*%* CO₂.
2. The cell state was observed every day, and the culture medium was replaced every 3 days until the cells formed masses with a diameter of about 80µm.

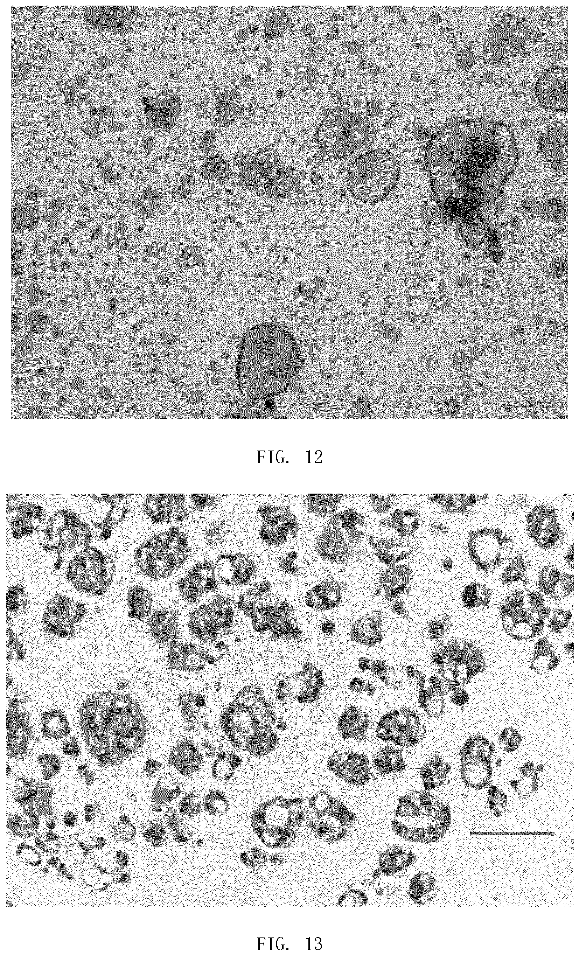

As shown in FIG. 12, after 3-10 days of culture, cancer cells were amplified, forming cell masses with a diameter of 80µm, the total number of tumor cells exceeded 10⁷, and the number of other types of cells was significantly reduced or even disappeared. After a large number of sample tests, the success rate of the present method for culturing in vitro primary tumor cells in the bile sample of gallbladder cancer and cholangiocarcinoma could reach 70*%*.

### Embodiment 35. Passaging primary cells in the bile samples of gallbladder cancer and cholangiocarcinoma

1. The cell masses in a culture dish were collected and centrifugated at 800g at a room temperature for 10 minutes, and a supernatant was discarded.
2. The cell masses were washed with sterile PBS solution and centrifugated at 800g at a room temperature for 10 minutes, and a supernatant was discarded.
3. The cell masses were resuspended with cell digestion solution (see Embodiment 30) and digested at 37°C. The digestion of cell masses was observed under the microscope every 5 minutes until all the cell masses were digested into individual cells.
4. The dissociation reaction was terminated with a 10 × volume of digestion termination solution (see Embodiment 30), and the cell suspension was collected.
5. The cell suspension was centrifuged at 800g at a room temperature for 10 minutes, and a supernatant was discarded.
6. The cell precipitation was resuspended with the primary cell culture medium of gallbladder cancer and cholangiocarcinoma, and the cells were counted.
7. A low-attachment-surface was used for culturing primary cells in the bile sample of gallbladder cancer and cholangiocarcinoma, and the culture medium in use was the primary cell culture medium for bile samples of gallbladder cancer and cholangiocarcinoma in Embodiment 30; a 6-well plate was taken as an example, and cells were planked at the density of 10⁶ cells per well in a cell incubator under the condition of 37°C, 5*%* CO₂.

### Embodiment 36. Cryopreserving primary cells in the bile samples of gallbladder cancer and cholangiocarcinoma

The primary cells in the bile sample of gallbladder cancer and cholangiocarcinoma suspension-cultured could be frozen after 2-3 passages and amplifications:
1. The cell masses in a culture dish were collected and centrifugated at 800g at a room temperature for 10 minutes, and a supernatant was discarded.
2. The cell masses were washed with sterile PBS solution and centrifugated at 800g at a room temperature for 10 minutes, and a supernatant was discarded.
3. The cell masses were resuspended with cell digestion solution (see Embodiment 30) and digested at 37°C. The digestion of cell masses was observed under the microscope every 15 minutes until all the cell masses were digested into individual cells.
4. The dissociation reaction was terminated with a 10 × volume of digestion termination solution (see Embodiment 30), the cell suspension was collected, and the cells were counted.
5. The cell suspension were centrifuged at 800g at a room temperature for 10 minutes, and a supernatant was discarded.
6. The cell precipitation was resuspended at a density of 10⁶/mL with cell cryopreserving solution (see Embodiment 30), each 2 mL cryopreserving tube contained 1 mL of cell suspension, and the cell suspension was frozen overnight in a gradient cooling box and transferred into liquid nitrogen for long-term preservation.

### Embodiment 37. Resuscitating primary cells in the bile samples of gallbladder cancer and cholangiocarcinoma

Primary cells in the bile sample of gallbladder cancer and cholangiocarcinoma preserved in liquid nitrogen were resuscitated:
1. 37°C sterile water was prepared five minutes in advance.
2. The cryopreserving tubes were removed from liquid nitrogen, and the cells were quickly melted in 37°C sterile water.
3. The cell solution were centrifuged at 800g at a room temperature for 10 minutes, and a supernatant was discarded.
4. The cell precipitation was resuspended with the primary cell culture medium for the bile sample of gallbladder cancer and cholangiocarcinoma (see Embodiment 30), the primary cells in the bile sample of gallbladder cancer and cholangiocarcinoma were cultured using a low-attachment-surface, the cells in each tube were resuscitated into a 3.5 cm culture dish, and the cells were cultured in a cell incubator under the condition of 37°C, 5*%* CO₂.

### Embodiment 38. HE staining identification of primary cells in the bile samples of gallbladder cancer and cholangiocarcinoma

Description of reagent consumables to be used in the following embodiment:
HE Staining Kit (Beijing Solarbio Science & Technology Co., Ltd., #G1120);
Cationic anticreep slide (ZSGB-BIO);
Xylene, methanol, acetone (Beijing Beihua Zhongtuo Technology Co., Ltd., analytical pure);
Neutral resin adhesive (Beijing Yili Fine Chemicals Co., Ltd.).

1. Primary cell masses from solid tumor tissues of gallbladder cancer and cholangiocarcinoma obtained by culturing with the primary cell culture medium for bile samples of gallbladder cancer and cholangiocarcinoma in Embodiment 30 (wherein the final concentration of human recombinant protein EGF was 20 ng/mL; the final concentration of human recombinant protein bFGF was 20 ng/mL; the final concentration of human recombinant protein HGF was 20 ng/mL; the final concentration of human recombinant protein FGF-10 was 20 ng/mL; the final concentration of human recombinant protein Wnt-3a was 200 ng/mL; the final concentration of human recombinant protein Noggin was 100 ng/mL; the final concentration of SB202190 was 5 µM; the final concentration of A83-01 was 1 µM; the final concentration of N-acetyl-L-cysteine was 1 mM; the final concentration of Nicotinamide was 10 mM; the final concentration of cortisol was 25 ng/mL; the final concentration of Y-27632 was 10 µM) were collected by centrifugation at 800g, and immobilized with 4*%* paraformaldehyde. Cell mass precipitation was imbedded in paraffin and sectioned with the thickness of 5 µm_{∘}
2. Paraffin sections were immersed in dimethylbenzene solution and incubated at a room temperature for 5 minutes for dewaxing, and after this process was repeated for 3 times, the sections were flushed with deionized water twice.
3. The sections were immersed in absolute ethanol and incubated at a room temperature for 10 minutes, and this process was repeated twice.
4. The sections were immersed in 95*%* ethanol and incubated at a room temperature for 10 minutes, and after this process was repeated twice, the sections were flushed with deionized water twice.
5. 100 µL of hematoxylin staining solution was added for staining for 1 min when the moisture on the slide was slightly dried.
6. The hematoxylin staining solution was absorbed, and the slide was washed for 3 times with tap water.
7. 100 µL of differentiation solution was added dropwise for differentiation for 1min.
8. The differentiation solution was absorbed, the slide was washed twice with tap water and washed with distilled water once.
9. The moisture on the slide surface was absorbed, and 200 µL of eosin stain was added dropwise for staining for 40 s.
10. The eosin stain was absorbed, and rinsing and dehydration were performed successively with 75*%*, 80*%*, 90*%* and 100*%* ethanol for 20 s, 20 s, 40 s and 40 s.
11. After the ethanol was dried in the air, 50 µL of dimethylbenzene was added dropwise for cell permeability.
12. After dimethylbenzene was completely dried, a drop of neutral resin adhesive was added, and the slide was sealed with a cover glass, observed under the microscope and photographed.

FIG. 13 shows an HE staining effect image of primary tumor cells in the bile sample of cholangiocarcinoma obtained by in vitro culture. It can be seen that these cells generally have the characteristics of cancer cells, such as high nuclear cytoplasmic ratio, hyperchromasia, chromatin condensation in nucleus, multinucleation and uneven cell size, and dozens to hundreds of tumor cells gather to form tumor cell masses with a certain three-dimensional structure.

### Embodiment 39. Immunohistochemical staining identification of primary cells in the bile samples of gallbladder cancer and cholangiocarcinoma

Description of reagents to be used in the following embodiment:
Paraformaldehyde (Beijing Beihua Zhongtuo Technology Co., Ltd., analytical pure), paraformaldehyde powder was dissolved with ultrapure water to form 4*%* (4g/100mL paraformaldehyde solution;
Hydrogen peroxide (Beijing Beihua Zhongtuo Technology Co., Ltd., 35*%*);
Normal goat serum for blocking (Solarbio, SL038);
Immunofluorescence primary antibody (Abcam, ab215838);
Immunofluorescence secondary antibody (Abcam, ab205719);
EDTA repair solution (Abcam, ab93684);
DAB chromogen (SignalStain^{®} DAB Substrate Kit, 8059S)

The primary cell masses from the bile samples of gallbladder cancer and cholangiocarcinoma obtained by culturing with the primary cell culture medium for bile sample of gallbladder cancer and cholangiocarcinoma in Embodiment 30 (wherein the final concentration of human recombinant protein EGF was 50 ng/mL; the final concentration of human recombinant protein bFGF was 25 ng/mL; the final concentration of human recombinant protein HGF was 25 ng/mL; the final concentration of human recombinant protein FGF-10 was 25 ng/mL; the final concentration of human recombinant protein Wnt-3a was 300 ng/mL; the final concentration of human recombinant protein Noggin was 200 ng/mL; the final concentration of SB202190 was 10 µM; the final concentration of A83-01 was 0.5 µM; the final concentration of N-acetyl-L-cysteine was 1 mM; the final concentration of Nicotinamide was 10 mM; the final concentration of cortisol was 25 ng/mL; the final concentration of Y-27632 was 10 µM) were collected for paraffin section, and epithelial-derived cells were characterized with pan-CK antibody according to the following steps.
1. The section was successively immersed in dimethylbenzene I for 10 min and dimethylbenzene II (10 min).
2. The section was immersed in absolute ethyl alcohol I (5 min) - absolute ethyl alcohol II (5 min) - 95*%* alcohol (5 min) - 80*%* alcohol (5 min) - 70*%* alcohol (5 min), and then flushed with deionized water twice, 2 min each time.
3. The tissue section was put into a repair box, then an appropriate amount of diluted EDTA repair solution (pH 9.0) was added, with the level higher than the tissue section.
4. The tissue section was repaired with microwave mid-range for 10 min (time started when the liquid boiled), and a dry tissue should be avoided in this process.
5. The repair box was taken out of the microwave oven and cooled down in air, and after the repair solution dropped to the room temperature, the glass slide was taken out and flushed with PBS (pH 7.4) for 3 times, 3 min each time (do not flush toward the tissue in the flushing process to avoid breaking the tissue).
6. Prepared 3*%* hydrogen peroxide (30*%* hydrogen peroxide diluted with deionized water) was added to the section tissue dropwise to block endogenous peroxidase, incubated at a room temperature for 15 min, and flushed with PBS for 3 times, 3 min each time.
7. PBS was dried out with absorbent paper, 10*%* goat serum (consistent with or similar to the species source of the secondary antibody) was added dropwise on the glass slide, and the glass slide was blocked at 37°C for 60 min.
8. The liquid around the tissue slide was wiped dry with absorbent paper, a circle was drawn around the tissue by mark pen, then the diluted primary antibody was added dropwise, and the slide was incubated in a wet box at 4°C overnight.
9. The section was flushed with PBS for 3 times for 3 min each time, a horseradish peroxidase labeled secondary antibody was added dropwise after the section was wiped dry with absorbent paper, and the section was incubated at a room temperature for 60 min.
10. The section was flushed with PBS for 3 times for 3 min each time, the section was wiped dry with absorbent paper after PBS liquid was thrown backward, freshly-prepared DAB chromogen was added to each section dropwise, the sections were observed under the microscope, and the section was flushed with tap water after a positive signal was shown to stop the color development.
11. Hematoxylin counterstaining was performed for 1 min, and the section was differentiated with acid ethanol differentiation solution after being washed with water, and flushed with tap water until cell nucleus turned blue.
12. After being put into water and flushed, the section was successively put into: 70*%* alcohol - 80*%* alcohol - 90*%* alcohol - 95*%* alcohol - absolute ethyl alcohol I - absolute ethyl alcohol II - dimethylbenzene I - dimethylbenzene II for dehydration and clearing, 2 min in each reagent, and finally the section was dried in a fume cupboard.
13. The section was sealed with neutral balsam and covered with cover glass. The section was placed in a fume cupboard and dried.
14. The dried section was observed or photographed under a microscope.

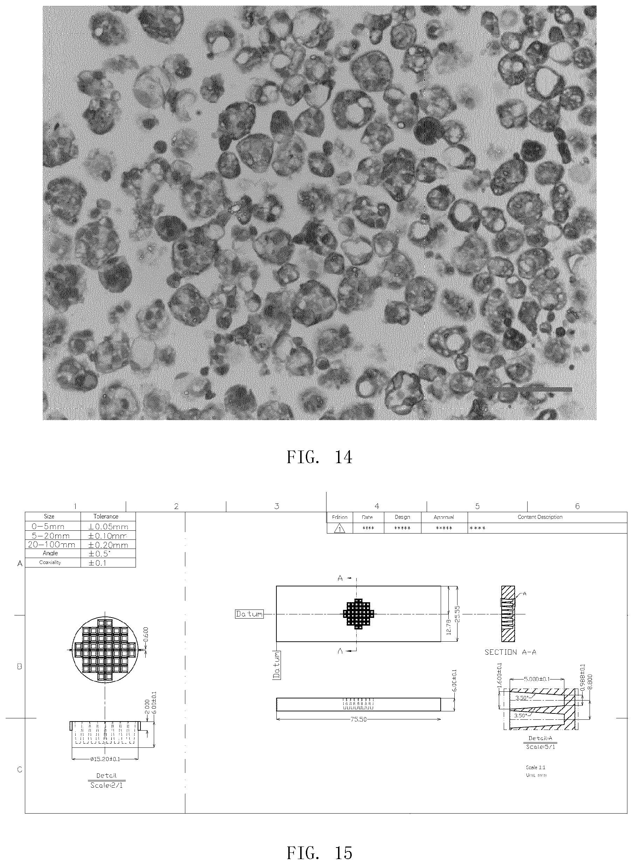

FIG. 14 shows an immunofluorescence staining effect drawing of primary tumor cell masses of cholangiocarcinoma cultured in vitro. It can be seen that all the cells constituting the cell masses are pan-CK positive and epithelial-derived, which confirms that the tumor cells obtained by culture in the present method are those with a high purity. Immunofluorescence staining identification was performed on 5 primary cultures of gallbladder cancer and cholangiocarcinoma samples, and the statistical results showed that the proportion of tumor cells in the primary cells in the bile sample of gallbladder cancer and cholangiocarcinoma obtained in the present method reached 64*%*-80*%* (Table 42). Embodiment 42 Immunohistochemical staining identification of primary cultures from the bile samples of gallbladder cancer and cholangiocarcinoma

| S/N | Sample No. | Type | Positive Rate of Pan-CK |
|---|---|---|---|
| 1 | 20190913LHY0460 | Gallbladder cancer | 77*%* |
| 2 | 20190913WGH0461 | Cholangiocarcinoma | 64*%* |
| 3 | 20190902YHQ0423 | Cholangiocarcinoma | 80*%* |
| 4 | 20190805NZZ0126 | Gallbladder cancer | 69*%* |
| 5 | 20190719WR1473 | Cholangiocarcinoma | 72*%* |

### Embodiment 40. Processing a microplate chip

In the embodiment, a microplate chip used for culturing the primary cells of gastric cancer, gallbladder cancer and cholangiocarcinoma in the present invention was made from PMMA material (or PS, PC, COC, COP, LAS and other materials) by injection molding. The chip can be used for culturing primary cells of gastric cancer, gallbladder cancer and cholangiocarcinoma and conducting in vitro drug sensitivity test. The design drawing of the microplate chip is as shown in FIG. 15.

Specifically, in the process of practical application, the microplate chip structure (design drawing as shown in FIG. 15) was prepared from PMMA material (or PS, PC, COC, COP, LAS and other materials), then the surface of the microplate chip was CYTOP-modified by the above CYTOP modification method (see Embodiment 29), so that the microplate chip available for culturing the primary cells of gastric cancer, gallbladder cancer and cholangiocarcinoma as stated herein was obtained.

### Industrial Application

The present invention provides a method for extracting and culturing primary tumor cells of gastric cancer, gallbladder cancer and cholangiocarcinoma from a fresh surgical sample of gastric cancer, a surgical sample or a biopsy puncture tissue sample of gallbladder cancer and cholangiocarcinoma, or a bile sample of gallbladder cancer and cholangiocarcinoma, and auxiliary reagents. The method has the following advantages: the tissue sample size is less, and only about 20 mg of surgical sample or about 10-20mL of bile sample is required; the method can be used for either culturing primary tumor cells in primary tumors of gastric cancer, gallbladder cancer and cholangiocarcinoma, or culturing primary tumor cells in metastatic lesions of gastric cancer, gallbladder cancer and cholangiocarcinoma; the culture cycle is short, and the primary tumor cells with the order of magnitude of 10⁶-10⁷ can be obtained in only 3-10 days; the culture stability is high, and the success rate of the method for culturing in vitro qualified samples is as high as 70*%*; the cell purity is high, the proportion of cancer cells in the primary cell cultures of gastric cancer, gallbladder cancer and cholangiocarcinoma obtained by the method can be up to 60*%*-95*%*, with less interference from impurity cells. The primary cell cultures of gastric cancer, gallbladder cancer and cholangiocarcinoma obtained by the method of the present invention can be used in in vitro tests, next generation sequencing, construction of animal models, construction of cell lines and the like at various cell levels. It is predictable that such culture method will have a wide application prospect in the fields of research, clinical diagnosis and treatment of gastric cancer, gallbladder cancer and cholangiocarcinoma.

## Claims

1. A primary cell culture medium for gastric cancer and/or gallbladder cancer and cholangiocarcinoma, is composed of antibiotic-antimycotic, HEPES, GlutaMax, non-essential amino acids, human recombinant protein EGF, human recombinant protein bFGF, human recombinant protein HGF, human recombinant protein FGF-10, human recombinant protein Wnt-3a, human recombinant protein Noggin, SB202190, A83-01, Primocin, N-acetyl-L-cysteine, Nicotinamide, N-2 Supplement, cortisol, B27, ITS-X, Gastrin 1, Y-27632 and Advanced DMEM/F12 culture medium; wherein, the final concentration of penicillin in the antibiotic-antimycotic is 100-200U/mL; the final concentration of streptomycin in the antibiotic-antimycotic is 100-200pg/mL; the final concentration of amphotericin B in the antibiotic-antimycotic is 250-250ng/mL; the final concentration of the HEPES is 8-12mM; the final concentration of the GlutaMax is 0.8-1.2*%* (volume percentage); the concentration of glycine in the non-essential amino acids is 80-120µM; the concentration of L-alanine in the non-essential amino acids is 80-120µM; the concentration of L-asparagine in the non-essential amino acids is 80-120µM; the concentration of L-aspartic acid in the non-essential amino acids is 80-120µM; the concentration of L-glutamic acid in the non-essential amino acids is 80-120µM; the concentration of L-proline in the non-essential amino acids is 80-120µM; the concentration of L-serine in the non-essential amino acids is 80-120µM; the final concentration of the human recombinant protein EGF is 10-100 ng/mL; the final concentration of the human recombinant protein bFGF is 10-50 ng/mL; the final concentration of the human recombinant protein HGF is 5-25 ng/mL; the final concentration of the human recombinant protein FGF-10 is 5-25 ng/mL; the final concentration of the human recombinant protein Wnt-3a is 200-300 ng/mL; the final concentration of the human recombinant protein Noggin is 100-200 ng/mL; the final concentration of the SB202190 is 5-10µM; the final concentration of the A83-01 is 0.25-1.25µM; the final concentration of Primocin is 1*%* (volume percentage); the final concentration of the N-acetyl-L-cysteine is 0.5-2mM; the final concentration of the Nicotinamide is 5-10mM; the final concentration of the N-2 Supplement is 1*%* (volume percentage); the final concentration of the cortisol is 20-50 ng/mL; the final concentration of the B27 is 1.5-2.5*%* (volume percentage); the final concentration of the ITS-X is 0.8-1.2*%* (volume percentage); the final concentration of the Gastrin 1 is 8-12nM; the final concentration of the Y-27632 is 5-20 µM; and the rest is the Advanced DMEM/F12 medium,
wherein,:
the gastric cancer is primary gastric cancer; and the gallbladder cancer and cholangiocarcinoma is primary gallbladder cancer and cholangiocarcinoma;
or, the gastric cancer is a metastatic lesion of gastric cancer; and the gallbladder cancer and cholangiocarcinoma is a metastatic lesion of gallbladder cancer and cholangiocarcinoma.

2. A kit of reagents for culturing primary cells of gastric cancer and/or gallbladder cancer and cholangiocarcinoma, which is any of the following:
(A1) Consisting of the culture medium described in claim 1 and all or part of the following: sample dissociation solution, sample preservation solution and sample washing solution;
The sample dissociation solution is composed of collagenase I, collagenase II, collagenase IV and PBS; wherein the final concentration of the collagenase I is 150-250 U/mL; the final concentration of the collagenase II is 150-250 U/mL; the final concentration of the collagenase IV is 50-150 U/mL; and the rest is PBS;
The sample preservation solution is composed of fetal bovine serum, antibiotic-antimycotic, HEPES and HBSS; wherein the final concentration of the fetal bovine serum is 1-5*%* (volume percentage); the final concentration of penicillin in the antibiotic-antimycotic is 100-200U/mL; the final concentration of streptomycin in the antibiotic-antimycotic is 100-200µg/mL; the final concentration of amphotericin B in the antibiotic-antimycotic is 250-500 ng/mL; the final concentration of the HEPES is 8-12mM; and the rest is HBSS.
The sample washing solution is composed of antibiotic-antimycotic and PBS; wherein the final concentration of penicillin in the antibiotic-antimycotic
is 100-200 U/mL; the final concentration of streptomycin in the antibiotic-antimycotic is 100-200 µg /mL; the final concentration of amphotericin B in the antibiotic-antimycotic is 250-500 ng /mL; and the rest is PBS.
(A2) Consisting of the culture medium described in claim 1 and the cell isolation buffer;
The cell isolation buffer is composed of P/S, heparin sodium and PBS; wherein the final concentration of penicillin in the P/S is 100-200 U/mL; the final concentration of streptomycin in the P/S is 100-200 µg /mL; the final concentration of the heparin sodium is 10IU/mL; and the rest is PBS;
(A3) Consisting of (A1) and all or part of the following reagents: cell digestion solution, digestion termination solution and cell cryopreserving solution;
(A4) Consisting of (A2) and all or part of the following reagents: cell digestion solution, digestion termination solution and cell cryopreserving solution;
The composition of the cell digestion solution is as follows: each 10mL of the cell digestion solution contains 4-6mL of Accutase, EDTA with a final concentration of 5mM and 1.5-2.5mL of TrypLE Express, and the rest is PBS;
The digestion termination solution is composed of fetal bovine serum, antibiotic-antimycotic and DMEM culture medium; wherein the final concentration of the fetal bovine serum is 8-12% (volume percentage); the final concentration of penicillin in the antibiotic-antimycotic is 100-200 U/mL; the final concentration of streptomycin in the antibiotic-antimycotic is 100-200 µg /mL; the final concentration of amphotericin B in the antibiotic-antimycotic is 250-500 ng /mL; and the rest is the DMEM culture medium;
The cell cryopreserving solution is composed of Advanced DMEM/F12 medium, DMSO and 1% methylcellulose solution; wherein the volume ratio of the Advanced DMEM/F12 medium, the DMSO and the 1*%* methylcellulose solution is 20:2: (0.8-1.2); the 1*%* methylcellulose solution is an aqueous solution of methylcellulose with a concentration of 1g/100ml.

3. An *in vitro* method for culturing primary cells of gastric cancer and/or gallbladder cancer and cholangiocarcinoma is either method A or method B:
Method A: An *in vitro* method for culturing primary cells in obtained solid tumor tissues of gastric cancer and/or gallbladder cancer and cholangiocarcinoma, comprising the following steps:
(a1) dissociating obtained solid tumor tissues of gastric cancer and/or gallbladder cancer and cholangiocarcinoma with the sample dissociation solution described in claim 2, to obtain primary cells in solid tumor tissues of gastric cancer and/or gallbladder cancer and cholangiocarcinoma;
(a2) suspension-culturing the dissociated primary cells in solid tumor tissues of gastric cancer and/or gallbladder cancer and cholangiocarcinoma in step (a1) with the medium described in claim 1;
Method B: An *in vitro* method for culturing primary tumor cells in a bile sample of gallbladder cancer and cholangiocarcinoma, comprising the following steps:
(b1) separating primary tumor cells in the bile sample of gallbladder cancer and cholangiocarcinoma, to obtain primary tumor cells in the bile sample of gallbladder cancer and cholangiocarcinoma;
(b2) suspension-culturing the primary tumor cells in the bile sample of gallbladder cancer and cholangiocarcinoma separated in step (b1) with the medium described in claim 1.

4. The *in vitro* method according to claim 3, wherein, in step (a1), the obtained solid tumor tissues of gastric cancer and/or gallbladder cancer and cholangiocarcinoma are dissociated with the sample dissociation solution according to the method comprising the following steps: according to the dosage of 0.1-0.3 mL of sample dissociation solution per mg of tissue, treating the trimmed solid tumor tissues of gastric cancer and/or gallbladder cancer and cholangiocarcinoma with the sample dissociation solution preheated at 37°C, dissociating the sample at 37°C for 15 minutes to 3 hours;
In step (b1), the primary tumor cells in the bile sample of gallbladder cancer and cholangiocarcinoma are separated from the bile sample of gallbladder cancer and cholangiocarcinoma according to a method comprising the following steps: suspending the cells in the bile sample of gallbladder cancer and cholangiocarcinoma with the cell isolation buffer described in claim 2, and then obtaining the primary tumor cells in the bile sample of gallbladder cancer and cholangiocarcinoma by density gradient centrifugation.

5. The *in vitro* method according to claim 3 or 4, wherein, in step (a2), the primary cells in solid tumor tissues of gastric cancer and/or gallbladder cancer and cholangiocarcinoma are suspension-cultured with the medium according to a method comprising the following steps: suspension-culturing the primary cells in solid tumor tissues of gastric cancer and/or gallbladder cancer and cholangiocarcinoma with the medium using a cell culture vessel M under the condition of 37°C, 5*%* CO₂, and replacing the medium every 2-4 days;
In step (b2), the primary tumor cells in the bile sample of gallbladder cancer and cholangiocarcinoma are suspension-cultured with the medium according to a method comprising the following steps: suspension-culturing the primary tumor cells in the bile sample of gallbladder cancer and cholangiocarcinoma with the medium using a cell culture vessel M under the condition of 37°C, 5*%* CO₂, and replacing the medium every 2-4 days;
The cell culture vessel M is any of the following: (I) cell culture vessels made of polystyrene, cell culture vessels made of polycarbonate, cell culture vessels made of polymethylmethacrylate, cell culture vessels made of COC resin, cell culture vessels made of cycloolefin polymer or cell culture vessels with a low attachment surface; (II) cell culture vessels after CYTOP modification on the cell culture vessels in (I).

6. The *in vitro* method according to claim 5, wherein, In (II), the cell culture vessels in (I) are CYTOP-modified according to a method comprising the following steps: performing pure oxygen etching on the cell culture vessels in (I) at an etching power of 20W for 3 minutes; then covering the surface of the cell culture vessels with 1*%* CYTOP solution, and drying the 1*%* CYTOP solution in the air to complete CYTOP modification;
The composition of the 1*%* CYTOP solution is as follows: each 100mL of the 1*%* CYTOP solution contains 1mL of CYTOP, and the rest is fluorocarbon oil.

7. The *in vitro* method according to any one of claims 3-6, wherein,before step (a1), the following steps of predissociation treatment the obtained solid tumor tissues of gastric cancer and/or gallbladder cancer and cholangiocarcinoma are also included: washing the surface of a solid tumor tissue sample of gastric cancer and/or gallbladder cancer and cholangiocarcinoma with 70-75*%* ethanol (volume percentage); washing the solid tumor tissue sample of gastric cancer and/or gallbladder cancer and cholangiocarcinoma successively with the sample washing solution described in claim 2 and the sterile PBS solution

8. The *in vitro* method according to claim 7, wherein, the in vitro isolation time of the solid tumor tissue sample of gastric cancer and/or gallbladder cancer and cholangiocarcinoma for the predissociation treatment is within 2 hours, and the solid tumor tissue sample of gastric cancer and/or gallbladder cancer and cholangiocarcinoma is preserved in the sample preservation solution described in claim 2 before the predissociation treatment.

9. The *in vitro* method according to any one of claims 3-8,wherein, in step (a1), the following steps are also included after dissociation treatment of the obtained solid tumor tissue of gastric cancer and/or gallbladder cancer and cholangiocarcinoma with the sample dissociation solution: terminating the dissociation reaction with the digestion termination solution described in claim 2, and collecting the cell suspension; filtering the cell suspension and removing tissue fragments and adherent cells; centrifuging and then resuspending cells with sterile PBS; centrifuging again, then resuspending the cell precipitation with the medium described in claim 1.

10. The *in vitro* method according to any one of claims 3-9, wherein, before step (a2), the following step are also included: passaging the primary cells in solid tumor of gastric cancer and/or gallbladder cancer and cholangiocarcinoma when masses with a diameter of 50-80 µm are formed by the primary cells in solid tumor of gastric cancer and/or gallbladder cancer and cholangiocarcinoma.
In step (b2), the following steps are also included: passaging the primary tumor cells in the bile sample of gallbladder cancer and cholangiocarcinoma when masses with a diameter of 50-80 µm are formed by the primary tumor cells in the bile sample of gallbladder cancer and cholangiocarcinoma.

11. The *in vitro* method according to claims 10,wherein, the cell digestion solution used for the passage is the cell digestion solution described in claim 2.

12. The *in vitro* method according to claim 10 or 11, wherein, the digestion termination solution used for the passage is the digestion termination solution described in claim 2.

13. The *in vitro* method according to any of claims 3-12, wherein,
the method also comprise steps of cryopreserving and/or resuscitating the primary cells in solid tumor of gastric cancer and/or gallbladder cancer and cholangiocarcinoma or the primary tumor cells in the bile sample of gallbladder cancer and cholangiocarcinoma after 2-3 passages and amplifications;
The cell cryopreserving solution for the cryopreserving is the cell cryopreserving solution described in claim 2.

14. The kit of reagents or *in vitro* method according to any one of claims 2-13, wherein,
the gastric cancer is primary gastric cancer; and the gallbladder cancer and cholangiocarcinoma is primary gallbladder cancer and cholangiocarcinoma;
or, the gastric cancer is a metastatic lesion of gastric cancer; and the gallbladder cancer and cholangiocarcinoma is a metastatic lesion of gallbladder cancer and cholangiocarcinoma.

15. The culture medium or kit of reagents or *in vitro* method according to any one of claims 1-14, wherein,
the primary cells of gastric cancer are primary cells in solid tumor of gastric cancer; and the primary cells of gallbladder cancer and cholangiocarcinom are primary cells in solid tumor of gallbladder cancer and cholangiocarcinoma or primary tumor cells in the bile sample of gallbladder cancer and cholangiocarcinoma;
or, the primary cells of gastric cancer are isolated from surgical samples of patients with gastric cancer; and the primary cells of gallbladder cancer and cholangiocarcinoma are isolated from a surgical sample, a puncture sample or a bile sample.

## Patentansprüche

1. Primärzellkulturmedium für Magenkrebs und/oder Gallenblasenkrebs und Cholangiokarzinom, bestehend aus Antibiotikum-Antimykotikum, HEPES, GlutaMax, nicht-essentiellen Aminosäuren, menschlichem rekombinantem Protein EGF, menschlichem rekombinantem Protein bFGF, menschlichem rekombinantem Protein HGF, menschlichem rekombinantem Protein FGF-10, menschlichem rekombinantem Protein Wnt-3a, menschlichem rekombinantem Protein Noggin, SB202190, A83-01, Primocin, N-Acetyl-L-Cystein, Nicotinamid, N-2-Supplement, Cortisol, B27, ITS-X, Gastrin 1, Y-27632 und Advanced DMEM/F12-Kulturmedium; wobei die Endkonzentration von Penicillin in dem Antibiotikum/Antimykotikum 100-200 U/ml beträgt; die Endkonzentration von Streptomycin im Antibiotikum/Antimykotikum 100-200 µg/ml beträgt; die Endkonzentration von Amphotericin B im Antibiotikum/Antimykotikum 250-250 ng/ml beträgt; die Endkonzentration von HEPES 8-12 mM beträgt; die Endkonzentration von GlutaMax 0,8-1,2 % (Volumenprozent); die Konzentration von Glycin in den nichtessenziellen Aminosäuren 80-120 µM beträgt; die Konzentration von L-Alanin in den nichtessenziellen Aminosäuren 80-120 µM beträgt; die Konzentration von L-Asparagin in den nichtessenziellen Aminosäuren 80-120 µM beträgt; die Konzentration von L-Asparaginsäure in den nicht nichtessentiellen Aminosäuren 80-120 µM beträgt; die Konzentration von L-Glutaminsäure in den nichtessentiellen Aminosäuren 80-120 µM beträgt; die Konzentration von L-Prolin in den nichtessentiellen Aminosäuren 80-120 µM beträgt; die Konzentration von L-Serin in den nichtessentiellen Aminosäuren 80-120 µM beträgt; die Endkonzentration des rekombinanten menschlichen Proteins EGF 10-100 ng/ml beträgt; die Endkonzentration des rekombinanten menschlichen Proteins bFGF 10-50 ng/ml beträgt; die Endkonzentration des rekombinanten menschlichen Proteins HGF 5-25 ng/ml beträgt; die Endkonzentration des rekombinanten menschlichen Proteins FGF-10 5-25 ng/ml beträgt; die Endkonzentration des humanen rekombinanten Proteins Wnt-3a 200-300 ng/ml; die Endkonzentration des humanen rekombinanten Proteins Noggin beträgt 100-200 ng/ml beträgt; die Endkonzentration von SB202190 5-10 µM beträgt; die Endkonzentration von A83-01 0,25-1,25 µM beträgt; die Endkonzentration von Primocin 1 % (Volumenprozent) beträgt; die Endkonzentration von N-Acetyl-L-Cystein 0,5-2 mM beträgt; die Endkonzentration von Nicotinamid 5-10 mM beträgt; die Endkonzentration des N-2-Supplements 1 % (Volumenprozent) beträgt; die Endkonzentration von Cortisol 20-50 ng/ml beträgt; die Endkonzentration von B27 1,5-2,5 % (Volumenprozent); die Endkonzentration des ITS-X 0,8-1,2 % (Volumenprozent) beträgt; die Endkonzentration des Gastrin 1 8-12 nM beträgt; die Endkonzentration des Y-27632 5-20 µM beträgt; und der Rest das Advanced DMEM/F12-Medium ist,
wobei:
der Magenkrebs primärer Magenkrebs ist; und der Gallenblasenkrebs und das Cholangiokarzinom primärer Gallenblasenkrebs und Cholangiokarzinom sind;
oder der Magenkrebs eine metastatische Läsion von Magenkrebs ist; und der Gallenblasenkrebs und das Cholangiokarzinom eine metastatische Läsion von Gallenblasenkrebs und Cholangiokarzinom sind.

2. Reagenzien-Kit zur Kultivierung von Primärzellen von Magenkrebs und/oder Gallenblasenkrebs und Cholangiokarzinom, der eines des folgenden ist:
(A1) bestehend aus dem in Anspruch 1 beschriebenen Kulturmedium und der Gesamtheit oder einem Teil der folgenden: Proben-Dissoziationslösung, ProbenKonservierungslösung und Proben-Waschlösung; wobei
die Proben-Dissoziationslösung aus Kollagenase I, Kollagenase II, Kollagenase IV und PBS beträgt; wobei die Endkonzentration der Kollagenase I 150-250 U/ml beträgt; die Endkonzentration der Kollagenase II 150-250 U/ml beträgt; die Endkonzentration der Kollagenase IV 50-150 U/ml beträgt; und der Rest PBS ist;
die Probenkonservierungslösung aus fötalem Kälberserum, Antibiotikum-Antimykotikum, HEPES und HBSS besteht; wobei die Endkonzentration des fötalen Kälberserums 1-5 % (Volumenprozent) beträgt; die Endkonzentration von Penicillin im Antibiotikum-Antimykotikum 100-200 U/ml beträgt; die Endkonzentration von Streptomycin im Antibiotikum-Antimykotikum 100-200 µg/ml beträgt; die Endkonzentration von Amphotericin B im Antibiotikum-Antimykotikum 250-500 ng/ml beträgt; die Endkonzentration von HEPES 8-12 mM beträgt; und der Rest HBSS ist;
die Waschlösung für die Proben aus Antibiotikum-Antimykotikum und PBS besteht, wobei die Endkonzentration von Penicillin im Antibiotikum-Antimykotikum100-200 U/ml beträgt; die Endkonzentration von Streptomycin im Antibiotikum-Antimykotikum 100-200 µg/ml beträgt; die Endkonzentration von Amphotericin B im Antibiotikum-Antimykotikum 250-500 ng/ml beträgt; und der Rest PBS ist;
(A2) bestehend aus dem in Anspruch 1 beschriebenen Kulturmedium und dem Zellisolierungspuffer; wobei
der Zellisolierungspuffer aus P/S, Heparin-Natrium und PBS besteht; wobei die Endkonzentration von Penicillin in P/S 100-200 U/ml beträgt; die Endkonzentration von Streptomycin in P/S 100-200 µg/ml beträgt; die Endkonzentration von Heparin-Natrium 10IE/ml beträgt; und der Rest PBS ist;
(A3) bestehend aus (A1) und allen oder einem Teil der folgenden Reagenzien: Zellverdauungslösung, Verdauungsbeendigungslösung und Kryokonservierungslösung für Zellen;
(A4) bestehend aus (A2) und allen oder einem Teil der folgenden Reagenzien: Zellverdauungslösung, Verdauungsbeendigungslösung und Kryokonservierungslösung für Zellen; wobei
die Zusammensetzung der Zellverdauungslösung wie folgt ist: 10 ml der Zellverdauungslösung enthalten 4-6 ml Accutase, EDTA mit einer Endkonzentration von 5 mM und 1,5-2,5 ml TrypLE Express, der Rest PBS ist; wobei
die Lösung zum Beenden der Verdauung aus fötalem Kälberserum, Antibiotikum/Antimykotikum und DMEM-Kulturmedium besteht; wobei die Endkonzentration des fötalen Kälberserums 8-12 % (Volumenprozent) beträgt; die Endkonzentration von Penicillin im Antibiotikum/Antimykotikum 100-200 U/ml , die Endkonzentration von Streptomycin im Antibiotikum/Antimykotikum 100-200 µg/ml, die Endkonzentration von Amphotericin B im Antibiotikum/Antimykotikum 250-500 ng/ml besteht und der Rest das DMEM-Kulturmedium ist; wobei
die Kryokonservierungslösung für Zellen aus Advanced DMEM/F12-Medium, DMSO und 1 % Methylcelluloselösung besteht, wobei das Volumenverhältnis von Advanced DMEM/F12-Medium, DMSO und 1 % Methylcelluloselösung 20:2:(0,8-1,2) beträgt; die 1 % Methylcelluloselösung eine wässrige Lösung von Methylcellulose mit einer Konzentration von 1 g/100 ml ist.

3. *In vitro* Verfahren zum Kultivieren von Primärzellen von Magenkrebs und/oder Gallenblasenkrebs und Cholangiokarzinom, das entweder Verfahren A oder Verfahren B ist:
Verfahren A: *in vitro* Verfahren zum Kultivieren von Primärzellen in erhaltenen festen Tumorgeweben von Magenkrebs und/oder Gallenblasenkrebs und Cholangiokarzinom, umfassend die folgenden Schritte:
(a1) Dissoziieren der erhaltenen soliden Tumorgewebe von Magenkrebs und/oder Gallenblasenkrebs und Cholangiokarzinom mit der in Anspruch 2 beschriebenen Proben-Dissoziationslösung, um Primärzellen in soliden Tumorgeweben von Magenkrebs und/oder Gallenblasenkrebs und Cholangiokarzinom zu erhalten;
(a2) Suspensionskultivieren der dissoziierten primären Zellen in festen Tumorgeweben von Magenkrebs und/oder Gallenblasenkrebs und Cholangiokarzinom in Schritt (a1) mit dem in Anspruch 1 beschriebenen Medium;
Verfahren B: *in vitro* Verfahren zur Kultivierung primärer Tumorzellen in einer Gallenprobe von Gallenblasenkrebs und Cholangiokarzinom, umfassend die folgenden Schritte:
(b1) Trennen von primären Tumorzellen in der Gallenprobe von Gallenblasenkrebs und Cholangiokarzinom, um primäre Tumorzellen in der Gallenprobe von Gallenblasenkrebs und Cholangiokarzinom zu erhalten;
(b2) Suspensionskultivieren der primären Tumorzellen in der in Schritt (b1) getrennten Gallenprobe von Gallenblasenkrebs und Cholangiokarzinom mit dem in Anspruch 1 beschriebenen Medium.

4. *In-vitro*-Verfahren nach Anspruch 3, wobei in Schritt (a1) die erhaltenen festen Tumorgewebe von Magenkrebs und/oder Gallenblasenkrebs und Cholangiokarzinom mit der Proben-Dissoziationslösung gemäß dem Verfahren dissoziiert werden, das die folgenden Schritte umfasst: gemäß der Dosierung von 0,1 bis 0,3 ml Proben-Dissoziationslösung pro mg Gewebe, Behandlung des zugeschnittenen festen Tumorgewebes von Magenkrebs und/oder Gallenblasenkrebs und Cholangiokarzinom mit der auf 37 °C vorerhitzten Proben-Dissoziationslösung, Dissoziieren der Probe bei 37 °C für 15 Minuten bis 3 Stunden;
in Schritt (b1) werden die primären Tumorzellen in der Gallenprobe von Gallenblasenkrebs und Cholangiokarzinom von der Gallenprobe von Gallenblasenkrebs und Cholangiokarzinom gemäß einem Verfahren getrennt, das die folgenden Schritte umfasst: Suspendieren der Zellen in der Gallenprobe von Gallenblasenkrebs und Cholangiokarzinom mit dem in Anspruch 2 beschriebenen Zellisolierungspuffer und anschließendes Gewinnen der primären Tumorzellen in der Gallenprobe von Gallenblasenkrebs und Cholangiokarzinom durch Dichtegradientenzentrifugation.

5. *In-vitro*-Verfahren nach Anspruch 3 oder 4, wobei in Schritt (a2) die Primärzellen in festen Tumorgeweben von Magenkrebs und/oder Gallenblasenkrebs und Cholangiokarzinom mit dem Medium in Suspension kultiviert werden, gemäß einem Verfahren, das die folgenden Schritte umfasst: Suspensionskultivieren der Primärzellen in festem Tumorgewebe von Magenkrebs und/oder Gallenblasenkrebs und Cholangiokarzinom mit dem Medium unter Verwendung eines Zellkulturgefäßes M unter den Bedingungen von 37 °C, 5 % CO₂ und Ersetzen des Mediums alle 2 bis 4 Tage; wobei
in Schritt (b2) die primären Tumorzellen in der Gallenprobe von Gallenblasenkrebs und Cholangiokarzinom mit dem Medium in Suspensionskultur gezüchtet werden, und zwar nach einem Verfahren, das die folgenden Schritte umfasst: Suspensionskultur der primären Tumorzellen in der Gallenprobe von Gallenblasenkrebs und Cholangiokarzinom mit dem Medium unter Verwendung eines Zellkulturgefäßes M unter den Bedingungen von 37 °C, 5 % CO₂ und Ersetzen des Mediums alle 2-4 Tage;
das Zellkulturgefäß M eines der folgenden ist: (I) Zellkulturgefäße aus Polystyrol, Zellkulturgefäße aus Polycarbonat, Zellkulturgefäße aus Polymethylmethacrylat,
Zellkulturgefäße aus COC-Harz, Zellkulturgefäße aus Cycloolefinpolymer oder Zellkulturgefäße mit einer Oberfläche mit geringer Anhaftung; (II) Zellkulturgefäße nach CYTOP-Modifizierung der Zellkulturgefäße in (I).

6. *In-vitro*-Verfahren nach Anspruch 5, wobei in (II) die Zellkulturgefäße in (I) gemäß einem Verfahren CYTOP-modifiziert werden, das die folgenden Schritte umfasst:
durchführen einer reinen Sauerstoffätzung an den Zellkulturgefäßen in (I) ) mit einer Ätzleistung von 20 W für 3 Minuten; dann Bedecken der Oberfläche der Zellkulturgefäße mit 1 % CYTOP-Lösung und Trocknen der 1 % CYTOP-Lösung an der Luft, um die CYTOP-Modifikation abzuschließen; wobei
die Zusammensetzung der 1 % CYTOP-Lösung wie folgt ist: 100 ml der 1 % CYTOP-Lösung enthalten 1 ml CYTOP und der Rest ist Fluorkohlenstofföl.

7. *In vitro* Verfahren gemäß einem der Ansprüche 3-6, wobei vor Schritt (a1) auch die folgenden Schritte der Vordissoziationsbehandlung der erhaltenen festen Tumorgewebe von Magenkrebs und/oder Gallenblasenkrebs und Cholangiokarzinom eingeschlossen sind: Waschen der Oberfläche einer festen Tumorgewebeprobe von Magenkrebs und/oder Gallenblasenkrebs und Cholangiokarzinom mit 70-75 % Ethanol (Volumenprozent); Waschen der festen Tumorgewebeprobe von Magenkrebs und/oder Gallenblasenkrebs und Cholangiokarzinom nacheinander mit der in Anspruch 2 beschriebenen Probenwaschlösung und der sterilen PBS-Lösung.

8. *In*-*vitro*-Verfahren nach Anspruch 7, wobei die In-vitro-Isolationszeit der festen Tumorgewebeprobe von Magenkrebs und/oder Gallenblasenkrebs und Cholangiokarzinom für die Vordissoziationsbehandlung innerhalb von 2 Stunden liegt und die feste Tumorgewebeprobe von Magenkrebs und/oder Gallenblasenkrebs und Cholangiokarzinom vor der Vordissoziationsbehandlung in der in Anspruch 2 beschriebenen Probenkonservierungslösung konserviert wird.

9. *In vitro* Verfahren gemäß einem der Ansprüche 3-8, wobei in Schritt (a1) nach der Dissoziationsbehandlung des erhaltenen festen Tumorgewebes von Magenkrebs und/oder Gallenblasenkrebs und Cholangiokarzinom mit der Proben-Dissoziationslösung auch die folgenden Schritte enthalten sind: Beenden der Dissoziationsreaktion mit der in Anspruch 2 beschriebenen Verdauungsbeendigungslösung und Sammeln der Zellsuspension; Filtrieren der Zellsuspension und Entfernen von Gewebefragmenten und anhaftenden Zellen; Zentrifugieren und anschließendes Resuspendieren der Zellen mit sterilem PBS; erneutes Zentrifugieren und anschließendes Resuspendieren der Zellpräzipitation mit dem in Anspruch 1 beschriebenen Medium.

10. *In-vitro*-Verfahren gemäß einem der Ansprüche 3 bis 9, wobei vor Schritt (a2) auch der folgende Schritt eingeschlossen ist: Passagieren der Primärzellen in einem soliden Tumor von Magenkrebs und/oder Gallenblasenkrebs und Cholangiokarzinom, wenn Massen mit einem Durchmesser von 50 bis 80 µm durch die Primärzellen in einem soliden Tumor von Magenkrebs und/oder Gallenblasenkrebs und Cholangiokarzinom gebildet werden, wobei
in Schritt (b2) auch die folgenden Schritte enthalten sind: Passagieren der primären Tumorzellen in der Gallenprobe von Gallenblasenkrebs und Cholangiokarzinom, wenn Massen mit einem Durchmesser von 50-80 µm von den primären Tumorzellen in der Gallenprobe von Gallenblasenkrebs und Cholangiokarzinom gebildet werden.

11. *In-vitro-Verfahren* gemäß Anspruch 10, wobei die für die Passage verwendete Zellverdauungslösung die in Anspruch 2 beschriebene Zellverdauungslösung ist.

12. *In-vitro-Verfahren* gemäß Anspruch 10 oder 11, wobei die für die Passage verwendete Verdauungsbeendigungslösung die in Anspruch 2 beschriebene Verdauungsbeendigungslösung ist.

13. *In-vitro-Verfahren* gemäß einem der Ansprüche 3 bis 12, wobei das Verfahren auch die Schritte der Kryokonservierung und/oder Reanimation der Primärzellen in einem soliden Tumor von Magenkrebs und/oder Gallenblasenkrebs und Cholangiokarzinom oder der Primärtumorzellen in der Gallenprobe von Gallenblasenkrebs und Cholangiokarzinom nach 2-3 Passagen und Amplifikationen umfasst; wobei
die Kryokonservierungslösung für die Kryokonservierung die in Anspruch 2 beschriebene Kryokonservierungslösung für Zellen ist.

14. Reagenzien-Kit oder *in vitro* Verfahren gemäß einem der Ansprüche 2-13, wobei der Magenkrebs primärer Magenkrebs ist; und der Gallenblasenkrebs und das Cholangiokarzinom primärer Gallenblasenkrebs und Cholangiokarzinom sind;
oder der Magenkrebs eine metastatische Läsion von Magenkrebs ist; und der Gallenblasenkrebs und das Cholangiokarzinom eine metastatische Läsion von Gallenblasenkrebs und Cholangiokarzinom sind.

15. Kulturmedium oder Reagenzien-Kit oder *in vitro*-Verfahren gemäß einem der Ansprüche 1-14, wobei
die primären Zellen von Magenkrebs primäre Zellen in einem soliden Tumor von Magenkrebs sind; und die primären Zellen von Gallenblasenkrebs und Cholangiokarzinom primäre Zellen in einem soliden Tumor von Gallenblasenkrebs und Cholangiokarzinom oder primäre Tumorzellen in der Gallenprobe von Gallenblasenkrebs und Cholangiokarzinom sind;
oder die primären Zellen von Magenkrebs aus chirurgischen Proben von Patienten mit Magenkrebs isoliert werden; und die primären Zellen von Gallenblasenkrebs und Cholangiokarzinom aus einer chirurgischen Probe, einer Punktionsprobe oder einer Gallenprobe isoliert werden.

## Revendications

1. Un milieu de culture cellulaire primaire pour le cancer gastrique et/ou le cancer de la vésicule biliaire et le cholangiocarcinome, est composé d'antibiotique-antimycosique, HEPES, GlutaMax, acides aminés non essentiels, protéine recombinante humaine EGF, protéine recombinante humaine bFGF, protéine recombinante humaine HGF, protéine recombinante humaine FGF-10, protéine recombinante humaine Wnt-3a, protéine recombinante humaine Noggin, SB202190, A83-01, Primocin, N-acétyl-L-cystéine, Nicotinamide, supplément N-2, cortisol, B27, ITS-X, Gastrine 1, Y-27632 et milieu de culture DMEM/F12 avancé ; dans lequel la concentration finale de pénicilline dans l'antibiotique-antimycosique est de 100-200U/mL ; la concentration finale de streptomycine dans l'antibiotique-antimycosique est de 100-200µg/mL ; la concentration finale d'amphotéricine B dans l'antibiotique-antimycosique est de 250-250ng/mL ; la concentration finale de l'HEPES est de 8-12mM ; la concentration finale de GlutaMax est de 0. 8-1. 2% (pourcentage du volume) ; la concentration de glycine dans les acides aminés non essentiels est de 80-120µM ; la concentration de L-alanine dans les acides aminés non essentiels est de 80-120µM ; la concentration de L-asparagine dans les acides aminés non essentiels est de 80-120µM ; la concentration d'acide L-aspartique dans les acides aminés non essentiels est de 80-120µM ; la concentration d'acide L-glutamique dans les acides aminés non essentiels est de 80-120µM ; la concentration de L-proline dans les acides aminés non essentiels est de 80-120µM ; la concentration de L-sérine dans les acides aminés non essentiels est de 80-120µM ; la concentration finale de la protéine recombinante humaine EGF est de 10-100 ng/mL; la concentration finale de la protéine recombinante humaine bFGF est de 10-50 ng/mL ; la concentration finale de la protéine recombinante humaine HGF est de 5-25 ng/mL; la concentration finale de la protéine recombinante humaine FGF-10 est de 5-25 ng/mL; la concentration finale de la protéine recombinante humaine Wnt-3a est de 200-300 ng/mL; la concentration finale de la protéine recombinante humaine Noggin est de 100-200 ng/mL ; la concentration finale du SB202190 est de 5-10µM ; la concentration finale de l'A83-01 est de 0. 25-1,25µM ; la concentration finale de Primocin est de 1 % (pourcentage du volume) ; la concentration finale de N-acétyl-L-cystéine est de 0,5-2mM ; la concentration finale de Nicotinamide est de 5-10mM ; la concentration finale du supplément N-2 est de 1 % (pourcentage du volume) ; la concentration finale de cortisol est de 20-50 ng/mL; la concentration finale de B27 est de 1. 5-2,5 % (pourcentage de volume) ; la concentration finale de l'ITS-X est de 0,8-1,2 % (pourcentage de volume) ; la concentration finale de la Gastrine 1 est de 8-12nM ; la concentration finale de l'Y-27632 est de 5-20 µM ; et le reste est le milieu DMEM/F12 avancé,
dans lequel, :
le cancer gastrique est un cancer gastrique primaire ; et le cancer de la vésicule biliaire et le cholangiocarcinome sont un cancer de la vésicule biliaire primaire et un cholangiocarcinome ;
ou, le cancer gastrique est une lésion métastatique d'un cancer gastrique ; et le cancer de la vésicule biliaire et le cholangiocarcinome est une lésion métastatique d'un cancer de la vésicule biliaire et d'un cholangiocarcinome.

2. Kit de réactifs pour la culture de cellules primaires de cancer gastrique et/ou de cancer de la vésicule biliaire et de cholangiocarcinome, présentant l'une des caractéristiques suivantes :
(A1) Composé du milieu de culture décrit dans la revendication 1 et de tout ou en partie des éléments suivants : solution de dissociation de l'échantillon, solution de conservation de l'échantillon et solution de lavage de l'échantillon ;
La solution de dissociation de l'échantillon est composée de collagénase I, de collagénase II, de collagénase IV et de PBS ; dans laquelle la concentration finale de la collagénase I est de 150-250 U/mL ; la concentration finale de la collagénase II est de 150-250 U/mL ; la concentration finale de la collagénase IV est de 50-150 U/mL ; et le reste est du PBS ;
La solution de conservation des échantillons est composée de sérum bovin foetal, d'antibiotique-antimycosique, d'HEPES et d'HBSS ; la concentration finale de sérum bovin foetal est de 1 à 5 % (pourcentage de volume) ; la concentration finale de pénicilline dans l'antibiotique-antimycosique est de 100 à 200 U/mL ; la concentration finale de streptomycine dans l'antibiotique-antimycosique est de 100-200µg/mL ; la concentration finale d'amphotéricine B dans l'antibiotique-antimycosique est de 250-500 ng/mL; la concentration finale de l'HEPES est de 8-12mM ; et le reste est du HBSS.
La solution de lavage de l'échantillon est composée d'antibiotique-antimycosique et de PBS ; la concentration finale de pénicilline dans l'antibiotique-antimycosique est de 100-200 U/mL; la concentration finale de streptomycine dans l'antibiotique-antimycosique est de 100-200 µg /mL; la concentration finale d'amphotéricine B dans l'antibiotique-antimycosique est de 250-500 ng /mL ; et le reste est du PBS.
(A2) Composé du milieu de culture décrit dans la revendication 1 et du tampon d'isolement cellulaire ;
Le tampon d'isolement cellulaire est composé de P/S, d'héparine sodique et de PBS ; dans lequel la concentration finale de pénicilline dans le P/S est de 100-200 U/mL ; la concentration finale de streptomycine dans le P/S est de 100-200 µg /mL ; la concentration finale de l'héparine sodique est de 10IU/mL ; et le reste est du PBS ;
(A3) Composé en (A1) et tout ou en partie des réactifs suivants : solution de digestion cellulaire, solution de fin de digestion et solution de cryoconservation cellulaire ;
(A4) Composé en (A2) et tout ou en partie des réactifs suivants : solution de digestion cellulaire, solution de fin de digestion et solution de cryoconservation cellulaire ;
La composition de la solution de digestion cellulaire est la suivante : chaque 10 ml de solution de digestion cellulaire contient 4 à 6 ml d'Accutase, de l'EDTA à une concentration finale de 5 mM et 1,5 à 2,5 ml de TrypLE Express, le reste étant du PBS ;
La solution de fin de digestion est composée de sérum bovin foetal, d'antibiotique-antimycosique et de milieu de culture DMEM ; la concentration finale de sérum bovin foetal est de 8-12% (pourcentage de volume) ; la concentration finale de pénicilline dans l'antibiotique-antimycosique est de 100-200 U/mL ; la concentration finale de streptomycine dans l'antibiotique-antimycosique est de 100-200 µg /mL ; la concentration finale d'amphotéricine B dans l'antibiotique-antimycosique est de 250-500 ng /mL ; et le reste est le milieu de culture DMEM ;
La solution de cryoconservation cellulaire est composée de milieu DMEM/F12 avancé, de DMSO et d'une solution de méthylcellulose à 1 % ; le rapport de volume entre le milieu DMEM/F12 avancé, le DMSO et la solution de méthylcellulose à 1 % est de 20:2 : (0,8-1,2) ; la solution de méthylcellulose à 1 % est une solution aqueuse de méthylcellulose d'une concentration de 1g/100ml.

3. Une méthode *in vitro* de culture de cellules primaires de cancer gastrique et/ou de cancer de la vésicule biliaire et de cholangiocarcinome est soit la méthode A, soit la méthode B :
Méthode A : Méthode *in vitro* de culture de cellules primaires dans des tissus tumoraux solides obtenus de cancer gastrique et/ou de cancer de la vésicule biliaire et de cholangiocarcinome, comprenant les étapes suivantes :
(a1) dissociation des tissus tumoraux solides obtenus de cancer gastrique et/ou de cancer de la vésicule biliaire et de cholangiocarcinome avec la solution de dissociation d'échantillon décrite dans la revendication 2, pour obtenir des cellules primaires dans les tissus tumoraux solides de cancer gastrique et/ou de cancer de la vésicule biliaire et de cholangiocarcinome ;
(a2) mise en culture en suspension des cellules primaires dissociées dans les tissus tumoraux solides du cancer gastrique et/ou du cancer de la vésicule biliaire et du cholangiocarcinome à l'étape (a1) avec le milieu décrit dans la revendication 1 ;
Méthode B : Méthode *in vitro* de culture de cellules tumorales primaires dans un échantillon de bile de cancer de la vésicule biliaire et de cholangiocarcinome, comprenant les étapes suivantes :
(b1) séparation des cellules tumorales primaires dans l'échantillon de bile du cancer de la vésicule biliaire et du cholangiocarcinome, pour obtenir des cellules tumorales primaires dans l'échantillon de bile du cancer de la vésicule biliaire et du cholangiocarcinome ;
(b2) mise en culture en suspension des cellules tumorales primaires dans l'échantillon de bile du cancer de la vésicule biliaire et du cholangiocarcinome séparées à l'étape (b1) avec le milieu décrit dans la revendication 1.

4. Méthode *in vitro* selon la revendication 3, dans laquelle, à l'étape (a1), les tissus tumoraux solides obtenus de cancer gastrique et/ou de cancer de la vésicule biliaire et de cholangiocarcinome sont dissociés avec la solution de dissociation d'échantillon selon la méthode comprenant les étapes suivantes : selon le dosage de 0. 1-0,3 mL de solution de dissociation d'échantillon par mg de tissu, traiter les tissus tumoraux solides parés de cancer gastrique et/ou de cancer de la vésicule biliaire et de cholangiocarcinome avec la solution de dissociation d'échantillon préchauffée à 37°C, dissocier l'échantillon à 37°C pendant 15 minutes à 3 heures ;
À l'étape (b1), les cellules tumorales primaires dans l'échantillon de bile du cancer de la vésicule biliaire et du cholangiocarcinome sont séparées de l'échantillon de bile du cancer de la vésicule biliaire et du cholangiocarcinome selon une méthode comprenant les étapes suivantes : suspension des cellules dans l'échantillon de bile du cancer de la vésicule biliaire et du cholangiocarcinome avec le tampon d'isolement cellulaire décrit dans la revendication 2, puis obtention des cellules tumorales primaires dans l'échantillon de bile du cancer de la vésicule biliaire et du cholangiocarcinome par centrifugation par gradient de densité.

5. Procédé *in vitro* selon la revendication 3 ou 4, dans lequel, à l'étape (a2), les cellules primaires des tissus tumoraux solides du cancer gastrique et/ou du cancer de la vésicule biliaire et du cholangiocarcinome sont cultivées en suspension avec le milieu selon un procédé comprenant les étapes suivantes : culture en suspension des cellules primaires des tissus tumoraux solides du cancer gastrique et/ou du cancer de la vésicule biliaire et du cholangiocarcinome avec le milieu à l'aide d'un récipient de culture cellulaire M dans les conditions de 37°C, 5%_{de CO2}, et remplacement du milieu tous les 2 à 4jours ;
À l'étape (b2), les cellules tumorales primaires dans l'échantillon de bile du cancer de la vésicule biliaire et du cholangiocarcinome sont cultivées en suspension avec le milieu selon une méthode comprenant les étapes suivantes : culture en suspension des cellules tumorales primaires dans l'échantillon de bile du cancer de la vésicule biliaire et du cholangiocarcinome avec le milieu en utilisant un récipient de culture cellulaire M dans les conditions de 37°C, 5 % de_{CO2}, et remplacement du milieu tous les 2 à 4 jours ;
Le récipient de culture cellulaire M est l'un des suivants : (I) récipients de culture cellulaire en polystyrène, récipients de culture cellulaire en polycarbonate, récipients de culture cellulaire en polyméthacrylate de méthyle, récipients de culture cellulaire en résine COC, récipients de culture cellulaire en polymère de cyclooléfine ou récipients de culture cellulaire avec une faible surface de fixation ; (II) récipients de culture cellulaire après modification CYTOP sur les récipients de culture cellulaire en (I).

6. Méthode *in vitro* selon la revendication 5, dans laquelle, en (II), les récipients de culture cellulaire en (I) sont modifiés par CYTOP selon une méthode comprenant les étapes suivantes : effectuer une gravure à l'oxygène pur sur les récipients de culture cellulaire en (I) à une puissance de gravure de 20 W pendant 3 minutes ; couvrir ensuite la surface des récipients de culture cellulaire avec une solution de CYTOP à 1 %, et sécher la solution de CYTOP à 1 % à l'air pour compléter la modification par CYTOP ;
La composition de la solution de CYTOP à 1 % est la suivante : chaque 100 ml de solution de CYTOP à 1 % contient 1 ml de CYTOP, le reste étant de l'huile fluorocarbonée.

7. Méthode *in vitro* selon l'une des revendications 3 à 6, dans laquelle, avant l'étape (a1), les étapes suivantes du traitement de prédissociation des tissus tumoraux solides obtenus du cancer gastrique et/ou du cancer de la vésicule biliaire et du cholangiocarcinome sont également incluses : lavage de la surface d'un échantillon de tissu tumoral solide de cancer gastrique et/ou de cancer de la vésicule biliaire et de cholangiocarcinome avec de l'éthanol à 70-75% (pourcentage en volume) ; lavage de l'échantillon de tissu tumoral solide de cancer gastrique et/ou de cancer de la vésicule biliaire et de cholangiocarcinome successivement avec la solution de lavage de l'échantillon décrite dans la revendication 2 et la solution PBS stérile.

8. Méthode *in vitro* selon la revendication 7, dans laquelle, le temps d'isolement in vitro de l'échantillon de tissu tumoral solide de cancer gastrique et/ou de cancer de la vésicule biliaire et de cholangiocarcinome pour le traitement de prédissociation est inférieur à 2 heures, et l'échantillon de tissu tumoral solide de cancer gastrique et/ou de cancer de la vésicule biliaire et de cholangiocarcinome est conservé dans la solution de conservation de l'échantillon décrite dans la revendication 2 avant le traitement de prédissociation.

9. Méthode *in vitro* selon l'une quelconque des revendications 3 à 8, dans laquelle, à l'étape (a1), les étapes suivantes sont également incluses après le traitement de dissociation du tissu tumoral solide obtenu du cancer gastrique et/ou du cancer de la vésicule biliaire et du cholangiocarcinome avec la solution de dissociation de l'échantillon : terminer la réaction de dissociation avec la solution de fin de digestion décrite dans la revendication 2, et recueillir la suspension cellulaire ; filtrer la suspension cellulaire et éliminer les fragments de tissus et les cellules adhérentes ; centrifuger puis remettre en suspension les cellules avec du PBS stérile ; centrifuger à nouveau, puis remettre en suspension la précipitation cellulaire avec le milieu décrit dans la revendication 1.

10. Procédé *in vitro* selon l'une quelconque des revendications 3 à 9, dans lequel, avant l'étape (a2), les étapes suivantes sont également incluses : passage des cellules primaires dans la tumeur solide du cancer gastrique et/ou du cancer de la vésicule biliaire et du cholangiocarcinome lorsque des masses d'un diamètre de 50 à 80 µm sont formées par les cellules primaires dans la tumeur solide du cancer gastrique et/ou du cancer de la vésicule biliaire et du cholangiocarcinome.
À l'étape (b2), les étapes suivantes sont également incluses : faire passer les cellules tumorales primaires dans l'échantillon de bile du cancer de la vésicule biliaire et du cholangiocarcinome lorsque des masses d'un diamètre de 50-80 µm sont formées par les cellules tumorales primaires dans l'échantillon de bile du cancer de la vésicule biliaire et du cholangiocarcinome.

11. Méthode *in vitro* selon les revendications 10,dans laquelle, la solution de digestion cellulaire utilisée pour le passage est la solution de digestion cellulaire décrite dans la revendication 2.

12. Méthode *in vitro* selon la revendication 10 ou 11, dans laquelle la solution de terminaison de digestion utilisée pour le passage est la solution de terminaison de digestion décrite dans la revendication 2.

13. Méthode *in vitro* selon l'une des revendications 3 à 12, dans laquelle,
la méthode comprend également des étapes de cryoconservation et/ou de réanimation des cellules primaires dans la tumeur solide du cancer gastrique et/ou du cancer de la vésicule biliaire et du cholangiocarcinome ou des cellules tumorales primaires dans l'échantillon de bile du cancer de la vésicule biliaire et du cholangiocarcinome après 2 ou 3 passages et amplifications ;
La solution de cryoconservation des cellules pour la cryoconservation est la solution de cryoconservation des cellules décrite dans la revendication 2.

14. Kit de réactifs ou méthode *in vitro* selon l'une des revendications 2 à 13, dans lequel,
le cancer gastrique est un cancer gastrique primaire ; et le cancer de la vésicule biliaire et le cholangiocarcinome est un cancer de la vésicule biliaire primaire et un cholangiocarcinome ;
ou, le cancer gastrique est une lésion métastatique d'un cancer gastrique ; et le cancer de la vésicule biliaire et le cholangiocarcinome est une lésion métastatique d'un cancer de la vésicule biliaire et d'un cholangiocarcinome.

15. Milieu de culture, kit de réactifs ou méthode *in vitro* selon l'une des revendications 1 à 14, dans lequel,
les cellules primaires du cancer gastrique sont des cellules primaires de la tumeur solide du cancer gastrique ; et les cellules primaires du cancer de la vésicule biliaire et du cholangiocarcinome sont des cellules primaires de la tumeur solide du cancer de la vésicule biliaire et du cholangiocarcinome ou des cellules tumorales primaires dans l'échantillon de bile du cancer de la vésicule biliaire et du cholangiocarcinome ;
ou, les cellules primaires du cancer gastrique sont isolées à partir d'échantillons chirurgicaux de patients atteints de cancer gastrique ; et les cellules primaires du cancer de la vésicule biliaire et du cholangiocarcinome sont isolées à partir d'un échantillon chirurgical, d'un échantillon de ponction ou d'un échantillon de bile.
